(19) Europäisches Patentamt
European Patent Office
Office européen des brevets

(11) EP 1 584 925 B1

(12) **EUROPEAN PATENT SPECIFICATION**

(45) Date of publication and mention of the grant of the patent:
**01.06.2011 Bulletin 2011/22**

(21) Application number: **04702440.1**

(22) Date of filing: **15.01.2004**

(51) Int Cl.:
***G01N 33/50*** *(2006.01)*

(86) International application number:
**PCT/JP2004/000248**

(87) International publication number:
**WO 2004/065960 (05.08.2004 Gazette 2004/32)**

# (54) NOVEL SCREENING METHOD

NEUES SCREENING-VERFAHREN

NOUVEAU PROCEDE DE CRIBLAGE

(84) Designated Contracting States:
**AT BE BG CH CY CZ DE DK EE ES FI FR GB GR HU IE IT LI LU MC NL PT RO SE SI SK TR**

(30) Priority:
**17.01.2003 JP 2003010001**
**08.04.2003 JP 2003104540**
**09.07.2003 JP 2003194497**
**19.09.2003 JP 2003329080**

(43) Date of publication of application:
**12.10.2005 Bulletin 2005/41**

(73) Proprietor: **Takeda Pharmaceutical Company Limited**
**Osaka 541-0045 (JP)**

(72) Inventors:
- **ITO, Yasuaki**
**Takeda Pharmaceutical Company Limited**
**Ibaraki 300-4293 (JP)**
- **FUJII, Ryo**
**Takeda Pharmaceutical Company Limited**
**Ibaraki 300-4293 (JP)**
- **HINUMA, Shuji**
**Takeda Pharmaceutical Company Limited**
**Ibaraki 300-4293 (JP)**
- **FUKUSUMI, Shoji**
**Takeda Pharmaceutical Company Limited**
**Ibaraki 300-4293 (JP)**
- **MARUYAMA, Minoru**
**Takeda Pharmaceutical Company Limited**
**Ibaraki 300-4293 (JP)**

(74) Representative: **Weiss, Wolfgang et al**
**Weickmann & Weickmann**
**Patentanwälte**
**Postfach 86 08 20**
**81635 München (DE)**

(56) References cited:
**WO-A-02/057783**
**WO-A1-02/088182**
**WO-A1-03/068959**
**WO-A2-02/067868**
**JP-A- 2001 211 885**
**JP-A- 2002 522 011**
**JP-A- 2002 536 997**
**US-B1- 6 448 005**

- **HOWARD A D ET AL: "Orphan G-protein-coupled receptors and natural ligand discovery" TRENDS IN PHARMACOLOGICAL SCIENCES, ELSEVIER, HAYWARTH, GB, vol. 22, no. 3, 1 March 2001 (2001-03-01), pages 132-140, XP004317091 ISSN: 0165-6147**
- **DATABASE JPO PROTEINS [Online] 30 July 2008 'Agonist.' Retrieved from EBI, accession no. JPOP:DD650648 Database accession no. DD650648**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 584 925 B1

SEQUENCE LISTING

<110> Takeda Pharmaceutical Company Limited

<120> Novel Screening Method

<130> G05-0036

<150> JP 2003-010001
<151> 2003-01-17

<150> JP 2003-104540
<151> 2003-04-08

<150> JP 2003-194497
<151> 2003-07-09

<150> JP 2003-329080
<151> 2003-09-19

<150> PCT/JP2004/000248
<151> 2004-01-15

<160> 22

<210> 1
<211> 361
<212> PRT
<213> Homo sapiens

<400> 1

```
Met Ser Pro Glu Cys Ala Arg Ala Ala Gly Asp Ala Pro Leu Arg Ser
                5                   10                  15
Leu Glu Gln Ala Asn Arg Thr Arg Phe Pro Phe Phe Ser Asp Val Lys
            20                  25                  30
Gly Asp His Arg Leu Val Leu Ala Ala Val Glu Thr Thr Val Leu Val
```

35 40 45
Leu Ile Phe Ala Val Ser Leu Leu Gly Asn Val Cys Ala Leu Val Leu
50 55 60
Val Ala Arg Arg Arg Arg Arg Gly Ala Thr Ala Cys Leu Val Leu Asn
65 70 75 80
Leu Phe Cys Ala Asp Leu Leu Phe Ile Ser Ala Ile Pro Leu Val Leu
85 90 95
Ala Val Arg Trp Thr Glu Ala Trp Leu Leu Gly Pro Val Ala Cys His
100 105 110
Leu Leu Phe Tyr Val Met Thr Leu Ser Gly Ser Val Thr Ile Leu Thr
115 120 125
Leu Ala Ala Val Ser Leu Glu Arg Met Val Cys Ile Val His Leu Gln
130 135 140
Arg Gly Val Arg Gly Pro Gly Arg Arg Ala Arg Ala Val Leu Leu Ala
145 150 155 160
Leu Ile Trp Gly Tyr Ser Ala Val Ala Ala Leu Pro Leu Cys Val Phe
165 170 175
Phe Arg Val Val Pro Gln Arg Leu Pro Gly Ala Asp Gln Glu Ile Ser
180 185 190
Ile Cys Thr Leu Ile Trp Pro Thr Ile Pro Gly Glu Ile Ser Trp Asp
195 200 205
Val Ser Phe Val Thr Leu Asn Phe Leu Val Pro Gly Leu Val Ile Val
210 215 220
Ile Ser Tyr Ser Lys Ile Leu Gln Ile Thr Lys Ala Ser Arg Lys Arg
225 230 235 240
Leu Thr Val Ser Leu Ala Tyr Ser Glu Ser His Gln Ile Arg Val Ser
245 250 255
Gln Gln Asp Phe Arg Leu Phe Arg Thr Leu Phe Leu Leu Met Val Ser
260 265 270
Phe Phe Ile Met Trp Ser Pro Ile Ile Ile Thr Ile Leu Leu Ile Leu
275 280 285
Ile Gln Asn Phe Lys Gln Asp Leu Val Ile Trp Pro Ser Leu Phe Phe
290 295 300
Trp Val Val Ala Phe Thr Phe Ala Asn Ser Ala Leu Asn Pro Ile Leu
305 310 315 320
Tyr Asn Met Thr Leu Cys Arg Asn Glu Trp Lys Lys Ile Phe Cys Cys

```
                    325                 330                 335
        Phe Trp Phe Pro Glu Lys Gly Ala Ile Leu Thr Asp Thr Ser Val Lys
                340                 345                 350
        Arg Asn Asp Leu Ser Ile Ile Ser Gly
            355                 360
```

<210> 2
<211> 1083
<212> DNA
<213> Homo sapiens

<400> 2

```
atgtcccctg aatgcgcgcg ggcagcgggc gacgcgccct tgcgcagcct ggagcaagcc    60
aaccgcaccc gctttccctt cttctccgac gtcaagggcg accaccggct ggtgctggcc   120
gcggtggaga caaccgtgct ggtgctcatc tttgcagtgt cgctgctggg caacgtgtgc   180
gccctggtgc tggtggcgcg ccgacgacgc cgcggcgcga ctgcctgcct ggtactcaac   240
ctcttctgcg cggacctgct cttcatcagc gctatccctc tggtgctggc cgtgcgctgg   300
actgaggcct ggctgctggg ccccgttgcc tgccacctgc tcttctacgt gatgaccctg   360
agcggcagcg tcaccatcct cacgctggcc gcggtcagcc tggagcgcat ggtgtgcatc   420
gtgcacctgc agcgcggcgt gcggggtcct gggcggcggg cgcgggcagt gctgctggcg   480
ctcatctggg gctattcggc ggtcgccgct ctgcctctct gcgtcttctt ccgagtcgtc   540
ccgcaacggc tccccggcgc cgaccaggaa atttcgattt gcacactgat ttggcccacc   600
attcctggag agatctcgtg ggatgtctct tttgttactt tgaacttctt ggtgccagga   660
ctggtcattg tgatcagtta ctccaaaatt ttacagatca caaaggcatc aaggaagagg   720
ctcacggtaa gcctggccta ctcggagagc caccagatcc gcgtgtccca gcaggacttc   780
cggctcttcc gcaccctctt cctcctcatg gtctccttct tcatcatgtg gagccccatc   840
atcatcacca tcctcctcat cctgatccag aacttcaagc aagacctggt catctggccg   900
tccctcttct tctgggtggt ggccttcaca tttgctaatt cagccctaaa ccccatcctc   960
tacaacatga cactgtgcag gaatgagtgg aagaaaattt tttgctgctt ctggttccca  1020
gaaaagggag ccattttaac agacacatct gtcaaaagaa atgacttgtc gattatttct  1080
ggc                                                                1083
```

<210> 3
<211> 361
<212> PRT
<213> Mus musculus

<400> 3

Met Ser Pro Glu Cys Ala Gln Thr Thr Gly Pro Gly Pro Ser His Thr
5 10 15
Leu Asp Gln Val Asn Arg Thr His Phe Pro Phe Phe Ser Asp Val Lys
20 25 30
Gly Asp His Arg Leu Val Leu Ser Val Val Glu Thr Thr Val Leu Gly
35 40 45
Leu Ile Phe Val Val Ser Leu Leu Gly Asn Val Cys Ala Leu Val Leu
50 55 60
Val Ala Arg Arg Arg Arg Arg Gly Ala Thr Ala Ser Leu Val Leu Asn
65 70 75 80
Leu Phe Cys Ala Asp Leu Leu Phe Thr Ser Ala Ile Pro Leu Val Leu
85 90 95
Val Val Arg Trp Thr Glu Ala Trp Leu Leu Gly Pro Val Val Cys His
100 105 110
Leu Leu Phe Tyr Val Met Thr Met Ser Gly Ser Val Thr Ile Leu Thr
115 120 125
Leu Ala Ala Val Ser Leu Glu Arg Met Val Cys Ile Val Arg Leu Arg
130 135 140
Arg Gly Leu Ser Gly Pro Gly Arg Arg Thr Gln Ala Ala Leu Leu Ala
145 150 155 160
Phe Ile Trp Gly Tyr Ser Ala Leu Ala Ala Leu Pro Leu Cys Ile Leu
165 170 175
Phe Arg Val Val Pro Gln Arg Leu Pro Gly Gly Asp Gln Glu Ile Pro
180 185 190
Ile Cys Thr Leu Asp Trp Pro Asn Arg Ile Gly Glu Ile Ser Trp Asp
195 200 205
Val Phe Phe Val Thr Leu Asn Phe Leu Val Pro Gly Leu Val Ile Val
210 215 220
Ile Ser Tyr Ser Lys Ile Leu Gln Ile Thr Lys Ala Ser Arg Lys Arg
225 230 235 240
Leu Thr Leu Ser Leu Ala Tyr Ser Glu Ser His Gln Ile Arg Val Ser
245 250 255
Gln Gln Asp Tyr Arg Leu Phe Arg Thr Leu Phe Leu Leu Met Val Ser
260 265 270

Phe Phe Ile Met Trp Ser Pro Ile Ile Ile Thr Ile Leu Leu Ile Leu
275 280 285

Ile Gln Asn Phe Arg Gln Asp Leu Val Ile Trp Pro Ser Leu Phe Phe
290 295 300

Trp Val Val Ala Phe Thr Phe Ala Asn Ser Ala Leu Asn Pro Ile Leu
305 310 315 320

Tyr Asn Met Ser Leu Phe Arg Asn Glu Trp Arg Lys Ile Phe Cys Cys
325 330 335

Phe Phe Phe Pro Glu Lys Gly Ala Ile Phe Thr Asp Thr Ser Val Arg
340 345 350

Arg Asn Asp Leu Ser Val Ile Ser Ser
355 360

<210> 4
<211> 1083
<212> DNA
<213> Mus musculus

<400> 4

atgtcccctg agtgtgcaca gacgacgggc cctggcccct cgcacaccct ggaccaagtc 60
aatcgcaccc acttcccttt cttctcggat gtcaagggcg accaccggtt ggtgttgagc 120
gtcgtggaga ccaccgttct ggggctcatc tttgtcgtct cactgctggg caacgtgtgt 180
gctctagtgc tggtggcgcg ccgtcggcgc cgtggggcga cagccagcct ggtgctcaac 240
ctcttctgcg cggatttgct cttcaccagc gccatccctc tagtgctcgt cgtgcgctgg 300
actgaggcct ggctgttggg gcccgtcgtc tgccacctgc tcttctacgt gatgacaatg 360
agcggcagcg tcacgatcct cacactggcc gcggtcagcc tggagcgcat ggtgtgcatc 420
gtgcgcctcc ggcgcggctt gagcggcccg gggcggcgga ctcaggcggc actgctggct 480
ttcatatggg gttactcggc gctcgccgcg ctgcccctct gcatcttgtt ccgcgtggtc 540
ccgcagcgcc ttcccggcgg ggaccaggaa attccgattt gcacattgga ttggcccaac 600
cgcataggag aaatctcatg ggatgtgttt tttgtgactt tgaacttcct ggtgccggga 660
ctggtcattg tgatcagtta ctccaaaatt ttacagatca cgaaagcatc gcggaagagg 720
cttacgctga gcttggcata ctctgagagc caccagatcc gagtgtccca acaagactac 780
cgactcttcc gcacgctctt cctgctcatg gtttccttct tcatcatgtg gagtcccatc 840
atcatcacca tcctcctcat cttgatccaa aacttccggc aggacctggt catctggcca 900
tcccttttct tctgggtggt ggccttcacg tttgccaact ctgccctaaa ccccatactg 960
tacaacatgt cgctgttcag gaacgaatgg aggaagattt tttgctgctt ctttttttcca 1020

gagaagggag ccatttttac agacacgtct gtcaggcgaa atgacttgtc tgttatttcc 1080

agc 1083

<210> 5
<211> 20
<212> DNA
<213> Artificial Sequence

<220>
<223> primer

<400> 5
gctgtggcat gcttttaaac 20

<210> 6
<211> 20
<212> DNA
<213> Artificial Sequence

<220>
<223> primer

<400> 6
cgctgtggat gtctatttgc 20

<210> 7
<211> 30
<212> DNA
<213> Artificial Sequence

<220>
<223> primer

<400> 7
agttcatttc cagtaccctc catcagtggc 30

<210> 8
<211> 361
<212> PRT
<213> Rattus norvegicus

<400> 8

Met Ser Pro Glu Cys Ala Gln Thr Thr Gly Pro Gly Pro Ser Arg Thr
5 10 15
Pro Asp Gln Val Asn Arg Thr His Phe Pro Phe Phe Ser Asp Val Lys
20 25 30
Gly Asp His Arg Leu Val Leu Ser Val Leu Glu Thr Thr Val Leu Gly
35 40 45
Leu Ile Phe Val Val Ser Leu Leu Gly Asn Val Cys Ala Leu Val Leu
50 55 60
Val Val Arg Arg Arg Arg Arg Gly Ala Thr Val Ser Leu Val Leu Asn
65 70 75 80
Leu Phe Cys Ala Asp Leu Leu Phe Thr Ser Ala Ile Pro Leu Val Leu
85 90 95
Val Val Arg Trp Thr Glu Ala Trp Leu Leu Gly Pro Val Val Cys His
100 105 110
Leu Leu Phe Tyr Val Met Thr Met Ser Gly Ser Val Thr Ile Leu Thr
115 120 125
Leu Ala Ala Val Ser Leu Glu Arg Met Val Cys Ile Val Arg Leu Arg
130 135 140
Arg Gly Leu Ser Gly Pro Gly Arg Arg Thr Gln Ala Ala Leu Leu Ala
145 150 155 160
Phe Ile Trp Gly Tyr Ser Ala Leu Ala Ala Leu Pro Leu Cys Ile Leu
165 170 175
Phe Arg Val Val Pro Gln Arg Leu Pro Gly Gly Asp Gln Glu Ile Pro
180 185 190
Ile Cys Thr Leu Asp Trp Pro Asn Arg Ile Gly Glu Ile Ser Trp Asp
195 200 205
Val Phe Phe Val Thr Leu Asn Phe Leu Val Pro Gly Leu Val Ile Val
210 215 220
Ile Ser Tyr Ser Lys Ile Leu Gln Ile Thr Lys Ala Ser Arg Lys Arg
225 230 235 240

Leu Thr Leu Ser Leu Ala Tyr Ser Glu Ser His Gln Ile Arg Val Ser
245 250 255
Gln Gln Asp Tyr Arg Leu Phe Arg Thr Leu Phe Leu Leu Met Val Ser
260 265 270
Phe Phe Ile Met Trp Ser Pro Ile Ile Ile Thr Ile Leu Leu Ile Leu
275 280 285
Ile Gln Asn Phe Arg Gln Asp Leu Val Ile Trp Pro Ser Leu Phe Phe
290 295 300
Trp Val Val Ala Phe Thr Phe Ala Asn Ser Ala Leu Asn Pro Ile Leu
305 310 315 320
Tyr Asn Met Ser Leu Phe Arg Ser Glu Trp Arg Lys Ile Phe Cys Cys
325 330 335
Phe Phe Phe Pro Glu Lys Gly Ala Ile Phe Thr Glu Thr Ser Ile Arg
340 345 350
Arg Asn Asp Leu Ser Val Ile Ser Thr
355 360

<210> 9
<211> 1083
<212> DNA
<213> Rattus norvegicus

<400> 9

atgtcccctg agtgtgcgca gacgacgggc cctggcccct cgcgcacccc ggaccaagtc 60
aatcgcaccc acttcccttt cttctcggat gtcaagggcg accaccggct ggtgctgagc 120
gtcctggaga ccaccgttct gggactcatc tttgtggtct cactgctggg caacgtgtgt 180
gccctggtgc tggtggtgcg ccgtcggcgc cgtggggcga cagtcagctt ggtgctcaac 240
ctcttctgcg cggatttgct cttcaccagc gccatccctc tagtgctcgt ggtgcgctgg 300
actgaagcct ggctgctggg gcccgtcgtc tgccacctgc tcttctacgt gatgaccatg 360
agcggcagcg tcacgatcct cacgctggcc gcggtcagcc tggagcgcat ggtgtgcatc 420
gtgcgcctgc ggcgcggctt gagcggcccg ggcggcggga cgcaggcggc gctgctggct 480
ttcatatggg gttactcggc gctcgccgcg ctgcccctct gcatcttgtt ccgcgtggtc 540
ccgcagcgcc ttcccggcgg ggaccaggaa attccgattt gcacattgga ttggcccaac 600
cgcataggag aaatctcatg ggatgtgttt tttgtgactt tgaacttcct ggtaccagga 660
ctggtcattg tgatcagcta ctccaagatt ttacagatca cgaaagcctc gcggaagagg 720
cttacgctga gcttggcata ctccgagagc caccagatcc gagtgtccca gcaggactac 780

cggctcttcc gaacgctctt cctgctcatg gtttccttct tcatcatgtg gagtcccatc 840
atcatcacca tcctcctcat cttgatccag aacttccggc aggacctggt tatctggccg 900
tccctttct tctgggtggt ggccttcacg tttgccaact ccgccctaaa ccccattctg 960
tacaacatgt cgctgttcag gagcgagtgg aggaagattt tttgctgctt ctttttccca 1020
gagaagggag ccatttttac agaaacgtct atcaggcgaa atgacttgtc tgttatttcc 1080
acc 1083

<210> 10
<211> 19
<212> DNA
<213> Artificial Sequence

<220>
<223> primer

<400> 10
gtggtggcct tcacgtttg 19

<210> 11
<211> 19
<212> DNA
<213> Artificial Sequence

<220>
<223> primer

<400> 11
cgctcctgaa cagcgacat 19

<210> 12
<211> 26
<212> DNA
<213> Artificial Sequence

<220>
<223> probe

<400> 12
caactccgcc ctaaacccca ttctgt 26

<210> 13
<211> 33
<212> DNA
<213> Artificial Sequence

<220>
<223> primer

<400> 13
gtcgacatgt cccctgagtg tgcgcagacg acg 33

<210> 14
<211> 33

<212> DNA
<213> Artificial Sequence

<220>
<223> primer

<400> 14
gctagcttag gtggaaataa cagacaagtc att 33

<210> 15
<211> 23
<212> DNA
<213> Artificial Sequence

<220>
<223> primer

<400> 15
tccgagtgtc ccaacaagac tac 23

<210> 16
<211> 24
<212> DNA
<213> Artificial Sequence

<220>
<223> primer

<400> 16
gactccacat gatgaagaag gaaa 24

<210> 17
<211> 22
<212> DNA
<213> Artificial Sequence

<220>
<223> probe

<400> 17
ccgcacgctc ttcctgctca tg 22

<210> 18
<211> 19
<212> DNA
<213> Artificial Sequence

<220>
<223> primer

<400> 18
gtggtggcct tcacgtttg 19

<210> 19
<211> 19
<212> DNA
<213> Artificial Sequence

<220>
<223> primer

<400> 19
cgctcctgaa cagcgacat 19

<210> 20
<211> 26
<212> DNA
<213> Artificial Sequence

<220>
<223> probe

<400> 20
caactccgcc ctaaacccca ttctgt 26

<210> 21
<211> 21
<212> RNA
<213> Artificial Sequence

<220>
<221> misc_RNA
<222> (20).. (21)
<223> n stands for deoxy thymidine

<400> 21
ggaccaggaa auuccgauun n 21

<210> 22
<211> 21
<212> RNA
<213> Artificial Sequence

<220>
<221> misc_RNA
<222> (1) .. (2)
<223> n stands for deoxy thymidine

<400> 22
nnccuggucc uuuaaggcua a 21

## Description

TECHNICAL FIELD

The present invention relates to a method of screening a compound or its salt that changes the binding property of a G protein-coupled receptor protein as well as a corresponding kit.

BACKGROUND ART

physiological active substances such as various hormones, neurotransmitters, etc. regulate the biological function via specific receptor proteins present on cell membranes. Many of these receptor proteins are coupled with guanine nucleotide-binding protein (hereinafter sometimes simply referred to as G protein) and mediate the intracellular signal transduction via activation of G protein. These receptor proteins possess the common structure containing seven transmembrane domains and are thus collectively referred to as G protein-coupled receptor proteins (GPCR) or seven-transmembrane receptor proteins (7TMR).

G protein-coupled receptor proteins are present on the cell surface of each functional cell and organ in the body, and play important physiological roles as the target of the molecules that regulate the functions of the cells and organs, e.g., hormones, neurotransmitters, physiologically active substances and the like. Receptor proteins transmit signals to cells via binding with physiologically active substances, and the signals induce various reactions such as activation and inhibition of the cells.

To clarify the relationship between substances that regulate complex biological functions in various cells and organs in the body, and their specific receptor proteins, in particular, G protein-coupled receptor proteins will elucidate the functional mechanisms in various cells and organs in the body to provide a very important means for development of drugs closely associated with these functions.

For example, in various organs, their physiological functions are controlled in vivo through regulation by many hormones, hormone-like substances, neurotransmitters or physiologically active substances. In particular, physiologically active substances are found in numerous sites of the body and regulate the physiological functions through their corresponding receptor proteins. Many unknown hormones, neurotransmitters or many other physiologically active substances still exist in the body and, as to their receptor proteins, many of these proteins have not yet been reported. In addition, it is still unknown if there are subtypes of known receptor proteins.

It is very important for development of drugs to clarify the relationship between substances that regulate elaborated functions in vivo and their specific receptor proteins. Furthermore, for efficient screening of agonists and antagonists to receptor proteins in development of drugs, it is required to clarify functional mechanisms of receptor protein genes expressed in vivo and express the genes in an appropriate expression system.

In recent years, random analysis of cDNA sequences has been actively studied as a means for analyzing genes expressed in vivo. The sequences of cDNA fragments thus obtained have been registered on and published to databases as Expressed Sequence Tag (EST). Further, recently presence of many novel genes has been revealed from results of comprehensive analysis for genomic DNA sequence. However, since many ESTs contain sequence information only, it is difficult to predict their functions.

The amino acid sequence of human- and mouse-derived 14273 and the DNA encoding the same are reported (open sequence database: ACCESSION XP_061208, open sequence database: ACCESSION XP_129252, and WO 2000/00611). It is reported that the 14273 knock-out mice gain more weight (WO 2002/67868).

An object of the present invention is to find a ligand for the 14273 and provide a method of screening the 14273 agonist or antagonist using the same, and so on.

The present invention relates to a method of screening a compound or its salt that changes the binding property of a G protein-coupled receptor protein as well as a corresponding kit.

WO 02/067868 discloses a method of identifying a compound capable of treating a metabolic disorder related to 14273 nucleic acid expression or to 14273 polypeptide activity comprising assaying the ability of the compound to modulate the expression or activity.

US-B1-6 448 005 discloses human 14273. Further disclosed are methods using the receptor polypeptides and polynucleotides to identify agonists and antagonists for diagnosis and treatment in receptor mediated disorders.

WO 02/057783 discloses methods of screening for ligands of GPR40 including methods utilizing a fatty acid GPR40 ligand.

DISCLOSURE OF THE INVENTION

The present invention is defined by the appended set of claims.

The present inventors have made extensive investigations and as a result, found that an intrinsic ligand for human and mouse 14273 is a fatty acid. The inventors have further found that the 14273 is abundantly expressed in the hypophysis, adipose tissue, intestinal tissue, colorectal cancer cells, etc. and that the fatty acid acts on the 14273 to induce increase in intracellular $Ca^{2+}$ level, inhibition of intracellular cAMP production, activation of MAP kinase, suppression of adrenocorticotropic hormone (ACTH) secretion, suppression of glycerol production from adipose tissues, suppression of lipolysis, etc. Based on these findings, the inventors have further made investigations and come to accomplish the present invention.

That is, the present invention provides the following features.

[1] A method of screening a compound or its salt that changes the binding property of a G protein-coupled receptor protein comprising
the amino acid sequence represented by SEQ ID NO: 1, SEQ ID NO: 3 or SEQ ID NO: 8, or a salt thereof, to a fatty acid or a salt thereof, which comprises using (1) the receptor protein, or a salt thereof and (2) the fatty acid or a salt thereof.

[2] A kit for screening a compound or its salt that changes the binding property of a G protein-coupled receptor protein comprising
the amino acid sequence represented by SEQ ID NO: 1, SEQ ID NO: 3 or SEQ ID NO: 8, or a salt thereof, to a fatty acid or a salt thereof, comprising (1) the receptor protein or a salt thereof and (2) the fatty acid or a salt thereof.

[3] Described is also a compound or its salt that changes the binding property of a fatty acid or a salt thereof to a G protein-coupled receptor protein comprising
the amino acid sequence represented by SEQ ID NO: 1, SEQ ID NO: 3 or SEQ ID NO: 8, or a salt thereof, which is obtained by using the screening method according to [1] or the screening kit according to [2].

[4] A method of screening an agonist or an antagonist to a G protein-coupled receptor protein comprising the amino acid sequence represented by SEQ ID NO: 1, SEQ ID NO: 3 or SEQ ID NO: 8, or a salt thereof, which comprises using (1) the receptor protein, or a salt thereof and (2) a compound or its salt that changes the binding property of the receptor protein, or a salt thereof to a fatty acid or a salt thereof.

[5] A kit for screening an agonist or an antagonist to a G protein-coupled receptor protein comprising the amino acid sequence represented by SEQ ID NO: 1, SEQ ID NO: 3 or SEQ ID NO: 8, or a salt thereof, comprising (1) the receptor protein, or a salt thereof and (2) a compound or its salt that changes the binding property of the receptor protein, or a salt thereof to a fatty acid or a salt thereof.

[6] Described is also an agonist or an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof, which is obtained by using the screening method according to [4] or the screening kit according to [5].

[7] Described is also a pharmaceutical comprising a compound or its salt that changes the binding property of a fatty acid or a salt thereof to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[8] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction), comprising an agonist to a G protein-coupled receptor protein comprising the same or substantially the same amino acid sequence as the amino acid sequence represented by SEQ ID NO: 1. SEQ ID NO: 3 or SEQ ID NO: 8, or a salt thereof.

[9] Described is also an agent for regulating stress, an agent for suppressing glycerol production from adipocytes, an agent for lowering blood glycerol, an agent for suppressing lipolysis or an agent for suppressing insulin resistance, comprising an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[10] Described is also an agent for suppressing adrenocorticotropic hormone (ACTH) secretion, comprising an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[11] Described is also an agent for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, comprising an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[12] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation), comprising an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[13] Described is also an agent for regulating stress, an agent for promoting glycerol production from adipocytes, an agent for increasing blood glycerol, an agent for promoting lipolysis or an agent for promoting insulin resistance, comprising an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[14] Described is also an agent for promoting adrenocorticotropic hormone (ACTH) secretion, comprising an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[15] Described is also an agent for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, comprising an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[16] A method of screening an agonist to a G protein-coupled receptor protein comprising the amino acid sequence represented by SEQ ID NO: 1, SEQ ID NO: 3 or SEQ ID NO: 8, or a salt thereof, which comprises assaying the intracellular $Ca^{2+}$ level increasing activity, the intracellular cAMP production suppressing activity, MAP kinase phosphorylation or activation, or the adrenocorticotropic hormone (ACTH) secretion suppressing activity, in the case where a test compound is contacted with cells (e.g., CHO cells, adipocytes, AtT-20 cells or 3T3-L1 cells) containing a G protein-coupled receptor protein comprising the amino acid sequence represented by SEQ ID NO: 1, SEQ ID NO: 3 or SEQ ID NO: 8, or a salt thereof.

[17] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction), comprising a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[18] Described is also an agent for regulating stress, an agent for suppressing glycerol production from adipocytes, an agent for lowering blood glycerol, an agent for suppressing lipolysis or an agent for suppressing insulin resistance, comprising a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[19] Described is also an agent for suppressing adrenocorticotropic hormone (ACTH) secretion, comprising a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[20] Described is also an agent for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, comprising a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[21] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction), which comprises a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[22] Described is also an agent for regulating stress, an agent for suppressing glycerol production from adipocytes, an agent for lowering blood glycerol, an agent for suppressing lipolysis or an agent for suppressing insulin resistance, comprising a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[23] Described is also an gent for suppressing adrenocorticotropic hormone (ACTH) secretion, comprising a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[24] Described is also an agent for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, comprising a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[25] Described is also a diagnostic agent for diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory disease, comprising a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[26] Described is also a diagnostic agent for stress, comprising a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[27] Described is also a diagnostic agent for ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, comprising a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[28] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation), comprising an antibody to a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[29] Described is also an agent for regulating stress, an agent for promoting glycerol production from adipocytes, an agent for increasing blood glycerol, an agent for promoting lipolysis or an agent for promoting insulin resistance, comprising an antibody to a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[30] Described is also an agent for promoting adrenocorticotropic hormone (ACTH) secretion, comprising an antibody to a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof

[31] Described is also an agent for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, comprising an antibody to a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[32] Described is also a diagnostic agent for diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory disease, comprising an antibody to a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[33] Described is also a diagnostic agent for stress, comprising an antibody to a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[34] Described is also a diagnostic agent for ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, comprising an antibody to a G protein-coupled receptor protein comprising the same or substantially the same amino acid sequence as the amino acid sequence represented by SEQ ID NO: 1, SEQ ID NO: 3 or SEQ ID NO: 8, or its partial peptide, or a salt thereof.

[35] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation), comprising a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein.

[36] Described is also an agent for regulating stress, an agent for promoting glycerol production from adipocytes, an agent for increasing blood glycerol, an agent for promoting lipolysis or an agent for promoting insulin resistance, comprising a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein.

[37] Described is also an agent for promoting adrenocorticotropic hormone (ACTH) secretion, comprising a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein.

[38] Described is also an agent for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, comprising a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein.

[39] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction), comprising a compound or its salt that increases the expression level of a G protein-coupled receptor protein comprising the same or substantially the same amino acid sequence as the amino acid sequence represented by SEQ ID NO: I, SEQ ID NO: 3 or SEQ ID NO: 8, or a partial peptide thereof.

[40] Described is also an agent for regulating stress, an agent for suppressing glycerol production from adipocytes, an agent for lowering blood glycerol, an agent for suppressing lipolysis or an agent for suppressing insulin resistance, comprising a compound or its salt that increases the expression level of a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[41] Described is also an agent for suppressing adrenocorticotropic hormone (ACTH) secretion, comprising a compound or its salt that increases the expression level of a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[42] Described is also an agent for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, comprising a compound or its salt that increases the expression level of a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[43] Described is also an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic n, exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation), comprising a compound or its salt that decreases the expression level of a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[44] Described is also an agent for regulating stress, an agent for promoting glycerol production from adipocytes, an agent for increasing blood glycerol, an agent for promoting lipolysis or an agent for promoting insulin resistance, comprising a compound or its salt that decreases the expression level of a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[45] Described is also an agent for promoting adrenocorticotropic hormone (ACTH) secretion, comprising a compound or its salt that decreases the expression level of a G protein-coupled receptor protein comprising the same or substantially the same amino acid sequence as the amino acid sequence represented by SEQ ID NO: 1, SEQ JD NO: 3 or SEQ ID NO: 8, or a partial peptide thereof.

[46] Described is also an agent for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, comprising a compound or its salt that decreases the expression level of a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[47] Described is also an agent for potentiating the signal transducing activity of a G protein-coupled receptor protein comprising the same or substantially the same amino acid sequence as the amino acid sequence represented by SEQ ID NO: 1, SEQ ID NO: 3 or SEQ ID NO: 8, comprising a fatty acid or a salt thereof.

[48] The agent according to [47], which is an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction).

[49] The agent according to [47], which is an agent for regulating stress, an agent for suppressing glycerol production from adipocytes, an agent for lowering blood glycerol, an agent for suppressing lipolysis or an agent for suppressing insulin resistance.

[50] The agent according to [47], which is an agent for suppressing adrenocorticotropic hormone (ACTH) secretion.

[51] The agent according to [47], which is an agent for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy.

[52] The agent according to [47], which is an agent for suppressing adrenocorticotropic hormone (ACTH) secretion.

[53] Described is also a method of preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction), which comprises administering to a mammal an effective dose of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[54] Described is also a method of regulating stress, a method of suppressing glycerol production from adipocytes, a method of lowering blood glycerol, a method of suppressing lipolysis or a method of suppressing insulin resistance, which comprises administering to a mammal an effective dose of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[55] Described is also a method of suppressing adrenocorticotropic hormone (ACTH) secretion, which comprises administering to a mammal an effective dose of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[56] Described is also a method of preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, which comprises administering to a mammal an effective dose of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[57] Described is a method of preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation), which comprises administering to a mammal an effective dose of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[58] Described is also a method of regulating stress, a method of promoting glycerol production from adipocytes, a method of increasing blood glycerol, a method of promoting lipolysis or a method of promoting insulin resistance, which comprises administering to a mammal an effective dose of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[59] Described is also a method of promoting adrenocorticotropic hormone (ACTH) secretion, which comprises administering to a mammal an effective dose of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[60] Described is also a method of preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, which comprises administering to a mammal an effective dose of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof.

[61] Described is also the use of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1, SEQ ID NO: 3 or SEQ ID NO: 8, to produce an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction).

[62] Described is also the use of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1. SEQ ID NO: 3 or SEQ ID NO: 8, to produce an agent for regulating stress, an agent for suppressing glycerol production from adipocytes, an agent for lowering blood glycerol, an agent for suppressing lipolysis or an agent for suppressing insulin resistance.

[63] Described is also the use of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1, SEQ ID NO: 3 or SEQ ID NO: 8 to produce an agent for suppressing adrenocorticotropic hormone (ACTH) secretion..

[64] Described is also the use of (i) a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, or (iii) an agonist to a G protein-coupled receptor protein comprising the same or substantially the same amino acid sequence as the amino acid sequence represented by SEQ ID NO: 1, SEQ ID NO: 3 or SEQ ID NO: 8, to produce an agent for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy.

[65] Described is also the use of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, to produce an agent for preventing/treating mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation).

[66] Described is also the use of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, to produce an agent for regulating stress, an agent for promoting glycerol production from adipocytes, an agent for increasing blood glycerol, an agent for promoting lipolysis or an agent for promoting insulin resistance.

[67] Described is also the use of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, to produce an agent for promoting adrenocorticotropic hormone (ACTH) secretion.

[68] Described is the use of (i) an antibody to a G protein-coupled receptor protein comprising the same or substantially the same amino acid sequence as the amino acid sequence represented by SEQ ID NO: 1, SEQ ID NO: 3 or SEQ ID NO: 8, its partial peptide, or a salt thereof, (ii) a polynucleotide comprising the entire or part of a base sequence complementary to a polynucleotide comprising a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide of the receptor protein, or (iii) an antagonist to a G protein-coupled receptor protein comprising the same or substantially the same amino acid sequence as the amino acid sequence represented by SEQ ID NO: 1, SEQ ID NO: 3 or SEQ ID NO: 8, to produce an agent for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock.

[69] Described is a method for confirming that (i) a drug for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis; angina pectoris, myocardial infarction, anorexia or obesity), (ii) a drug for regulating stress, a drug for regulating glycerol production from adipocytes, a drug for regulating blood glycerol, a drug for regulating lipolysis or a drug for regulating insulin resistance, (iii) a drug for regulating adrenocorticotropic hormone (ACTH) secretion, (iv) a drug for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, or (v) chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, binds to a G protein-coupled receptor protein comprising the same or substantially the same amino acid sequence as the amino acid sequence represented by SEQ ID NO: 1, SEQ ID NO: 3 or SEQ ID NO: 8, or a salt thereof, which comprises using the receptor protein or a salt thereof.

[70] Described is a method for confirmation that a drug for preventing/treating (i) a drug for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris or myocardial infarction), (ii) a drug for regulating stress, a drug for suppressing glycerol production from adipocytes, a drug for lowering blood glycerol, a drug for suppressing lipolysis or a drug for suppressing insulin resistance, (iii) a drug for suppressing adrenocorticotropic hormone (ACTH) secretion, or (iv) a drug for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, is an agonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof, which comprises using the receptor protein or a salt thereof.

[71] Described is a method for confirmation that (i) a drug for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation), (ii) a drug for regulating stress, a drug for promoting glycerol production from adipocytes, a drug for increasing blood glycerol, a drug for promoting lipolysis or a drug for promoting insulin resistance, (iii) a drug for promoting adrenocorticotropic hormone (ACTH) secretion, or (iv) a drug for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, is an antagonist to a G protein-coupled receptor protein comprising the same or substantially the same amino acid sequence as the amino acid sequence represented by SEQ ID NO: 1, SEQ ID NO: 3 or SEQ ID NO: 8, or a salt thereof, which comprises using the receptor protein or a salt thereof.

[72] Described is the method for confirmation according to [69] through [71], wherein the binding amount of each drug to the receptor protein, its partial peptide or a salt thereof is determined when each drug is contacted with the receptor protein, its partial peptide or a salt thereof.

[73] Described is a pharmaceutical comprising the combination of (1) (i) an agonist or antagonist to a G protein-coupled receptor protein comprising the same or substantially the same amino acid sequence as the amino acid sequence represented by SEQ ID NO: 1, SEQ ID NO: 3 or SEQ ID NO: 8, or/and (ii) a compound or its salt that changes the expression level of a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof, and (2) (i) a drug for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris, myocardial infarction, anorexia or obesity), (ii) a drug for regulating stress, a drug for regulating glycerol production from adipocytes, a drug for regulating blood glycerol, a drug for regulating lipolysis or a drug for regulating insulin resistance, (iii) a drug for regulating adrenocorticotropic hormone (ACTH) secretion, (iv) a drug for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome or adrenocortical atrophy, or (v) a drug for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock.

[74] Described is a G protein-coupled receptor protein consisting of the amino acid sequence represented by SEQ is NO: 8, its partial peptide or a salt thereof.

[75] Described is a polynucleotide comprising a polynucleotide encoding the G protein-coupled receptor protein according to [74].

[76] Described is a DNA consisting of the base sequence represented by SEQ ID NO: 9.

[77] Described is a recombinant vector comprising the polynucleotide according to [75].

[78] Described is a transformant transfonned by the recombinant vector according to [77].

[79] Described is a method of producing the G protein-coupled receptor protein or a salt thereof according to [74], which comprises culturing the transformant according to [78] and producing the G protein-coupled receptor protein or a salt thereof according to [74].

The present invention further provides the following features and so on.

[80] The screening method according to [1], wherein comparison is made between (i) the case where a G protein-coupled receptor protein comprising

the amino acid sequence represented by SEQ ID NO: 1 or SEQ ID NO: 3 (hereinafter sometimes briefly referred to as the 14273) or a salt thereof is contacted with a fatty acid or a salt thereof, and (ii) the case where the 14273 or a salt thereof is contacted with a fatty acid or a salt thereof and a test compound.

[81] the screening method according to [1], characterized in that (i) when a labeled fatty acid or a salt thereof is contacted with the 14273 or a salt thereof and (ii) when a labeled fatty acid or a salt thereof and a test compound are contacted with the 14273, or a salt thereof, the binding amounts of the labeled fatty acid or a salt thereof to said 14273 or a salt thereof, are determined and compared.

[82] The screening method according to [1], characterized in that (i) when a labeled fatty acid or a salt thereof is contacted with cells containing the 14273 and (ii) when a labeled fatty acid or a salt thereof and a test compound are contacted with cells containing the 14273, the binding amounts of the labeled fatty acid or a salt thereof to said cells are determined and compared.

[83] The screening method according to [I], characterized in that (i) when a labeled fatty acid or a salt thereof is contacted with a membrane fraction of cells containing the 14273 and (ii) when a labeled fatty acid or a salt thereof and a test compound are contacted with a membrane fraction of cells containing the 14273, the binding amounts of the labeled fatty acid or a salt thereof to said membrane fraction are determined and compared.

[84] The screening method according to [1], characterized in that (i) when a labeled fatty acid or a salt thereof is contacted with the 14273 expressed on a cell membrane with a transformants by culturing the transformant bearing a DNA comprising a DNA encoding the 14273 and (ii) when a labeled fatty acid or a salt thereof and a test compound are contacted with the 14273 expressed on a cell membrane of said transformant, the binding amounts of the labeled fatty acid or a salt thereof to the 14273 are determined and compared.

[85] The screening method according to [1], characterized in that (i) when a compound activating the 14273 is contacted with cells containing the 14273 and (ii) when a compound activating the 14273 and a test compound are contacted with cells containing the 14273, the cell stimulating activities mediated by the 14273 are determined and compared.

[86] The screening method according to [1], characterized in that when a compound capable of activating the 14273 is contacted with the 14273 expressed on a cell membrane with a transformant by culturing the transformant transformed with a recombinant vector bearing a DNA comprising the DNA encoding the 14273 and when a compound capable of activating the 14273 and a test compound are contacted with the 14273 expressed on a cell membrane of said transformant, the 14273-mediated cell stimulating activities are determined and compared.

[87] The screening method according to [85] or [86], wherein the compound activating the 14273 is fatty acid or a salt thereof.

[88] The screening kit according to [2], which comprises cells containing the 14273 or a membrane fraction of the cells.

[89] The screening kit according to [2], which comprises the 14273 expressed on a cell membrane of a transformant by culturing the transformant transformed with a recombinant vector bearing a DNA comprising a DNA encoding the 14273.

[90] Described is the method for confirmation according to [69] through [71], characterized in that (1) when (i) a labeled fatty acid or a salt thereof or (ii) a labeled compound or its salt that changes the binding property of the receptor protein or a salt thereof to a fatty acid or a salt thereof, is contacted with the receptor protein, its partial peptide, or a salt thereof, and (2) when each drug and (i) a labeled fatty acid or a salt thereof or (ii) the labeled compound or its salt are contacted with the receptor protein, its partial peptide, or a salt thereof, the binding properties of (i) the labeled fatty acid or a salt thereof or (ii) the labeled compound or its salt, to the receptor protein, its partial peptide, or a salt thereof are determined.

[91] Described is the method for confirmation according to [69] through [71], characterized in that (1) when (i) a labeled fatty acid or a salt thereof or (ii) a labeled compound or its salt that changes the binding property of the receptor protein or a salt thereof to a fatty acid or a salt thereof, is contacted with cells containing the receptor protein or a membrane fraction of the cells, and (2) when each drug and (i) a labeled fatty acid or a salt thereof or (ii) the labeled compound or its salt are contacted with the cells containing the receptor protein or a membrane fraction of the cells, the binding amounts of (i) the labeled fatty acid or a salt thereof or (ii) the labeled compound or its salt, to the cells containing said receptor protein or a membrane fraction of the cells are determined.

[92] Described is the method for confirmation according to [69] through [71], characterized in that (1) when (i) a fatty acid or a salt thereof or (ii) a compound or its salt that changes the binding property of the receptor protein or a salt thereof to a fatty acid or a salt thereof, is contacted with cells containing the receptor protein or a membrane fraction of the cells, and (2) when each drug and (i) a fatty acid or a salt thereof or (ii) the compound or its salt are contacted with the cells containing the receptor protein or a membrane fraction of the cells, the intracellular $Ca^{2+}$ level increasing activities, intracellular cAMP production suppressing activities, MAP kinase phosphorylated or activation, adrenocorticotropic hormone (ACTH) secretion suppressing activities, glycerol production suppressing activities or lipolysis suppressing activities are determined.

[93] Described is a kit for confirming that (i) a drug for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris, myocardial infarction, anorexia or obesity), (ii) a drug for regulating stress, a drug for regulating glycerol production from adipocytes, a drug for regulating blood glycerol, a drug for regulating lipolysis or a drug for regulating insulin resistance, (iii) a drug for regulating adrenocorticotropic hormone (ACTH) secretion, (iv) a drug for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, or (v) a drug for preventing/treating connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome; malignant exophthalmos due to thyroid gland disease, ACTH isolated deficiency), skin diseases (e.g., urticaria, eczema, dermatitis, herpes zoster, psoriasis, drug allergy) or anaphylactic shock, binds to a G protein-coupled receptor protein comprising the same or substantially the same amino acid sequence as the amino acid sequence represented by SEQ ID NO: 1 or SEQ ID NO: 3, or a salt thereof, characterized by comprising said receptor protein, its partial peptide, or a salt thereof.

[94] Described is (i) a drug for preventing/treating, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, diabetes mellitus, hyperlipemia, overweight, arteriosclerosis, angina pectoris, myocardial infarction, anorexia or obesity), (ii) a drug for regulating stress, a drug for regulating glycerol production from adipocytes, a drug for regulating blood glycerol, a drug for regulating lipolysis or a drug for regulating insulin resistance, (iii) a drug for regulating adrenocorticotropic hormone (ACTH) secretion, (iv) a drug for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, or (v) a drug for preventing/treating connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy) or anaphylactic shock, which is an agonist or antagonist to a G protein-coupled receptor protein comprising the same or substantially the same amino acids sequence as the amino acid sequence represented by SEQ ID NO: 1 or SEQ ID NO: 3.

[95] An siRNA for a polynucleotide encoding a G protein-coupled receptor protein comprising the same or substantially the same amino acid sequence as the amino acid sequence represented by SEQ ID NO: 1, SEQ ID NO: 3 or SEQ ID NO: 8, or a partial peptide thereof.

[96] The siRNA according to [95], which is siRNA (m14i561 of EXAMPLE 11) constructed by a sense strand consisting of the base sequence represented by SEQ ID NO: 21 and an antisense strand consisting of the base sequence represented by SEQ ID NO: 22.

[97] Described is a diagnostic agent comprising the siRNA according to [95].

[98] Described is a pharmaceutical comprising the siRNA according to [95].

[99] Described is an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones or circulatory diseases (especially, anorexia and obesity, among others, obesity with visceral fat accumulation), comprising the siRNA according to [95].

[100] Described is an agent for regulating stress, an agent for promoting glycerol production from adipocytes, an agent for increasing blood glycerol, an agent for promoting lipolysis or an agent for promoting insulin resistance, comprising the siRNA according to [95].

[101] Described is an agent for promoting adrenocorticotropic hormone (ACTH) secretion, comprising the siRNA according to [95].

[102] An agent for preventing/treating chronic articular rheumatism, systemic lupus erythematosus, polymyositis, rheumatic fever, scleroderma, kidney disease, bronchial asthma, pulmonary tuberculous pleuritis, sarcoidosis, diffuse interstitial pneumonia, ulcerative colitis, cholestatic acute hepatitis, fulminant hepatitis, chronic hepatitis, cirrhosis, encephalomyelitis, peripheral neuritis, multiple sclerosis, myasthenia gravis, facial paralysis, hemolytic anemia, granulocytosis, purpura, aplastic anemia, leukemia, malignant lymphoma, acute or chronic adrenocortical insufficiency, adrenogenital syndrome, malignant exophthalmos due to thyroid gland disease, ACTH isolated deficiency, urticaria, eczema, dermatitis, herpes zoster, psoriasis, drug allergy or anaphylactic shock, comprising the siRNA according to [95].

BRIEF DESCRIPTION OF THE DRAWINGS

FIG. 1 shows the results obtained by examination of a change in intracellular $Ca^{2+}$ level when 30 μM palmitoleic acid was added. Counts on the ordinate denote fluorescence intensity showing intracellular $Ca^{2+}$ level and Time (sec.) on the abscissa denote the passage of time (second) after sample addition. Symbol ○ (circle) designates human 14273-expressed CHO-K1 cells, Δ. (triangle) designates mouse 14273-expressed CHO-K1 cells and □ (square) designates 14273-not expressed-CHO-K1 cells for control in which the 14273 was not expressed.

FIG. 2 shows the results obtained by examination of a change in intracellular $Ca^{2+}$ level when 30 μM linoleic acid was added. Counts on the ordinate denote fluorescence intensity showing intracellular $Ca^{2+}$ level and Time (sec.) on the abscissa denote the passage of time (second) after sample addition. Symbol ○(circle) designates human 14273-expressed CHO-K1 cells, Δ (triangle) designates mouse 14273-expressed CHO-K1 cells and □ (square) designates 14273-not expressed-CHO-K1 cells for control in which the 14273 was not expressed.

FIG. 3 shows the results obtained by examination of a change in intracellular $Ca^{2+}$ level when 30 μM γ-linolenic acid was added. Counts on the ordinate denote fluorescence intensity showing intracellular $Ca^{2+}$ level and Time (sec.) on the abscissa denote the passage of time (second) after sample addition. Symbol ○(circle) designates human 14273-expressed CHO-K1 cells, Δ (triangle) designates mouse 14273-expressed CHO-K1 cells and □ (square) designates 14273-not expressed-CHO-K1 cells for control in which the 14273 was not expressed.

FIG. 4 shows the results obtained by examination of a change in intracellular $Ca^{2+}$ level when 30 μM arachidonic acid was added. Counts on the ordinate denote fluorescence intensity showing intracellular $Ca^{2+}$ level and Time (sec.) on the abscissa denote the passage of time (second) after sample addition. Symbol ○(circle) designates human 14273-expressed CHO-K1 cells, Δ (triangle) designates mouse 14273-expressed CHO-K1 cells and □ (square) designates 14273-not expressed-CHO-K1 cells for control in which the 14273 was not expressed.

FIG. 5 shows the results obtained by examination of a change in intracellular $Ca^{2+}$ level when 30 μM docosahexaenoic acid (DHA) was added. Counts on the ordinate denote fluorescence intensity showing intracellular $Ca^{2+}$ level and Time (sec.) on the abscissa denote the passage of time (second) after sample addition. Symbol ○(circle) designates human 14273-expressed CHO-K1 cells, Δ (triangle) designates mouse 14273-expressed CHO-K1 cells and □ (square) designates 14273-not expressed-CHO-K1 cells for control in which the 14273 was not expressed.

FIG. 6 shows the expression distribution of 14273 mRNA in various human tissues. The copies/25 ng total RNA designates the number of copies per 25 ng of total RNA.

FIG. 7 shows the expression distribution of 14273 mRNA in various rat tissues. The copies/25 ng poly(A)+RNA designates the number of copies per 25 ng of poly(A)+RNA.

FIG. 8 shows the results obtained by examination of increased expression of 14273 mRNA in the water-immersion restraint stress-loaded rat hypophysis. The abscissa denotes water-immersion time (h). Copies/ng poly(A)+RNA on the ordinate designate the number of copies/ng poly(A)+RNA.

FIG. 9 shows the results obtained by examination of effects of fatty acids on cAMP production in human 14273-expressed CHO cells. "Forskolin" designates the case where any fatty acid was not added in the presence of forskolin, "Butyric acid" designates the case where butyric acid (30 μM) was added in the presence of forskolin, "γ-Linolenic acid" designates the case where γ-linolenic acid (30 μM) was added in the presence of forskolin, "Linoleic acid" designates the case where linoleic acid (30 μM) was added in the presence of forskolin, "DHA" designates the case where DHA (30 μM) was added in the presence of forskolin, and "Oleic acid" designates the case where oleic acid (30 μM) was added in the presence of forskolin. "%" on the ordinate designates the amount of cAMP produced when each fatty acid was added, in which a value obtained by subtracting base from the amount of cAMP produced by forskolin stimulation was made 100%.

FIG. 10 shows the results of detection of MAP kinase activation in human 14273-expressed CHO cells by the addition of a fatty acid. "CHO-h 14273" designates the case where human 14273-expressed CHO cells were used, and "CHO-mock" designates the case of using CHO cells where human 14273 was not expressed. "γ-lino" designates the case where γ-linolenic acid was added and "methyl" designates the case where methyl linoleate was added. Numerical figures (min.) represent the passage of time (min.) after sample addition. p44 represents the bands from phosphorylated and activated ERK1. p42 represents the bands from phosphorylated and activated ERK2.

FIG. 11 show the results obtained by examination of a change in expression of the 14273 receptor, accompanied by differentiation induction of 3T3-L1 cells into adipocytes. The abscissa denotes the number of days (day) after differentiation induction of 3T3-L1 cells into adipocytes. The copies/25 ng total RNA on the ordinate denotes values when the obtained mRNA expression level of the 14273 receptor in the 3T3-L1 cells acquired was calculated as the number of copies per 25 ng of total RNA.

FIG. 12 shows the results obtained by examination of a change in expression of the 14273 receptor, accompanied by differentiation induction of rat primary culture preadipocytes into adipocytes. Day 0 represents the case using cells before the differentiation induction and Day 8 represents the case using cells 8 days after the differentiation induction. The copies/25 ng total RNA on the ordinate denotes values when the obtained mRNA expression level of the 14273 receptor in the primary culture adipocytes was calculated as the number of copies per 25 ng of total RNA.

FIG. 13 shows the results obtained by examination of the effects of suppressing corticotropin-releasing factor (CRF)-inducing ACTH secretion from AtT-20 cells by fatty acids, wherein "base" denotes the case added with no fatty acid in the absence of CRF, CRF denotes the case added with no fatty acid in the presence of CRF (10 nM); and "γ-LA" denotes the case added with γ-linolenic acid, α-LA denotes the case added with α-linolenic acid and BA denotes the case added with butyric acid, all in the presence of CRF (10 nM). ACTH (x 25 pg/ml) denotes the amount of ACTH secreted. Mean + standard error (n=8); **, p<0.01; *, p<0.05 (Student's t test).

FIG. 14 shows the action of suppressing lipolysis of a fatty acid in 3T3-L1 adipose differentiation cells. γ-LA and ML denote γ-linolenic acid and methyl linoleate, respectively base indicates the case added with no fatty acid. Glycerol (μM) denotes the amount of glycerol produced. Symbol * indicates significant suppression when isoproterenol was added to the base (p<0.05). Symbol** indicates significant suppression when isoproterenol was added to the base p < 0.01).

BEST MODE FOR CARRYING OUT THE INVENTION

The G protein-coupled receptor protein of the present invention (hereinafter sometimes briefly referred to as the 14273) is a receptor protein comprising the same or substantially the same amino acid sequence as the amino acid sequence represented by SEQ ID NO: 1, SEQ ID NO: 3, or SEQ ID NO: 8.

The 14273 may be any protein derived from any cell of human and mammals (e.g., guinea pig, rat, mouse, rabbit, swine, sheep, bovine, monkey, etc.); any cell (e.g., splenocyte, nerve cells, glial cells, β cells of pancreas, pancreatic Langerhans' islet, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.) or hematocytes; or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum. substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cell, prostate, testicle, testis, ovary, placenta, uterus, bone, joint, skeletal muscle, etc.; the proteins may also be synthetic proteins. In particular, the 14273 is highly expressed in the hypophysis, gastrointestinal tract and adipose tissue.

The amino acid sequence which has substantially the same amino acid sequence as the amino acid sequence represented by SEQ ID NO: 1, SEQ ID NO: 3 or SEQ ID NO: 8 includes an amino acid sequence having at least about 85% homology, preferably at least about 90% homology and more preferably at least about 95% homology, to the amino acid sequence represented by SEQ ID NO: 1, SEQ ID NO: 3 or SEQ ID NO: 8.

Examples of the protein which contains substantially the same amino acid sequence as the amino acid sequence represented by SEQ ID NO: 1, SEQ ID NO: 3 or SEQ ID NO: 8 include a protein comprising substantially the same amino acid sequence as the amino acid sequence represented by SEQ ID NO: 1, SEQ ID NO: 3 or SEQ ID NO: 8 and preferably having the activity substantially equivalent to the amino acid sequence represented by SEQ ID NO: I, SEQ ID NO: 3 or SEQ ID NO: 8, etc.

Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

Examples of the substantially equivalent activity described above include a ligand binding activity, a signal transduction activity, etc. The term substantially equivalent is used to mean that the activities are the same in nature. Therefore, although it is preferred that activities such as the ligand binding and signal transduction activities, etc. be equivalent (e.g., about 0.01- to about 100-fold, preferably about 0.5- to about 20-fold, more preferably about 0.5- to about 2-fold), quantitative factors such as a level of these activities, a molecular weight of the protein, etc. may differ.

The activities such as ligand binding and signal transduction activities or the like can be determined according to publicly known methods with some modifications thereof, for example, by the screening methods that will be later described.

Also, proteins comprising the following amino acid sequences are used as the 14273: a) amino acid sequences wherein at least I or 2 amino acids (preferably approximately 1 to 30 amino acids, more preferably approximately 1 to 10 amino acids, most preferably several (1 to 5) amino acids) are deleted of the amino acid sequence represented by SEQ ID NO: 1, SEQ ID NO: 3 or SEQ ID NO: 8; b) amino acid sequences wherein at least 1 or 2 amino acids (preferably approximately 1 to 30 amino acids, more preferably approximately 1 to 10 amino acids, and most preferably several (1 to 5) amino acids) are added to the amino acid sequence represented by SEQ ID NO: 1, SEQ ID NO: 3 or SEQ ID NO: 8; c) amino acid sequences wherein at least I or 2 amino acids (preferably approximately 1 to 30 amino acids, more preferably approximately 1 to 10 amino acids, and most preferably several (1 to 5) amino acids) in the amino acid sequence represented by SEQ ID NO: 1, SEQ ID NO: 3 or SEQ ID NO: 8 are substituted by other amino acids; or d) combination of these amino acid sequences described above; and the like.

Throughout the present specification, the 14273 is represented in accordance with the conventional way of describing peptides, that is, the N-terminus (amino terminus) at the left hand and the C-terminus (carboxyl terminus) at the right hand. In the 14273 including a 14273 comprising the amino acid sequence represented by SEQ ID NO: 2, the C-terminus may be in any form of a carboxyl group (-COOH), carboxylate ($-COO^-$), an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$alkyl group, e.g., benzyl, phenethyl, etc., or an α-naphthyl-$C_{1-2}$-alkyl group such as α-naphthylmethyl, etc.; and the like. In addition, pivaloyloxymethyl or the like, which is used widely as an ester for oral administration, may also be used.

Where the 14273 contains a carboxyl group (or a carboxylate) at a position other than the C-temminus, it may be amidated or esterified and such an amide or ester is also included within the 14273. The ester group may be the same group as that described with respect to the C-terminus described above.

Furthermore, examples of the 14273 include variants of the above proteins, wherein the amino group at the N-terminal methionine residue of the protein supra is protected with a protecting group (for example, a $C_{1-6}$ acyl group such as a $C_{2-6}$ alkanoyl group, e.g., formyl goup, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins bound to sugar chains.

Specific examples of the 14273 used include human-derived 14273 consisting of the amino acid sequence represented by SEQ ID NO: 1 and mouse-derived 14273 consisting of the amino acid sequence represented by SEQ ID NO: 3 (WO2002/67868, open sequence database: ACCESSION XP_061208, XP_129252), rat-derived 14273 consisting of the amino acid sequence represented by SEQ ID NO: 8, etc. Rat-derived 14273 consisting of the amino acid sequence represented by SEQ ID NO: 8 is a novel protein.

As partial peptides of the 14273 (hereinafter sometimes referred to as the partial peptides), any peptide can be used so long as it can be a peptide of the aforesaid 14273. Among the 14273 protein molecules, for example, those having a site exposed to the outside of a cell membrane and having substantially the same receptor binding activity can be used.

Specifically, the partial peptide of the 14273 comprising the amino acid sequence represented by SEQ ID NO: 1, SEQ ID NO: 3 or SEQ ID NO: 8 is a peptide containing the parts analyzed to be extracellular domains (hydrophilic domains) in the hydrophobic plotting analysis. A peptide containing a hydrophobic domain in part can be used as well. In addition, the peptide may contain each domain separately or plural domains together.

In the partial peptides described herein, preferred are peptides having at least 20, preferably at least 50, and more preferably at least 100 amino acids, in the amino acid sequence which constitutes the receptor protein of the present invention described above.

The amino acid sequence comprising substantially the same amino acid sequence includes an amino acid sequence having at least about 85% homology, preferably at least about 90% homology and more preferably at least about 95% homology, to these amino acid sequences.

Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

Herein the term "substantially the same receptor activity" has the same significance as described above. The "substantially the same receptor activity" can be assayed by the same manner as described above.

In the amino acid sequence described above, the partial peptide described herein may contain amino acid sequences, of which at least 1 or 2 amino acids (preferably approximately 1 to 10 amino acids, more preferably several (1 to 5) amino acids) are deleted; to which at least 1 or 2 amino acids (preferably approximately I to 20 amino acids, more preferably approximately I to 10 amino acids, and most preferably several (1 to 5) amino acids) are added; or, in which at least 1 or 2 amino acids (preferably approximately 1 to 10 amino acids, more preferably several and most preferably approximately I to 5 amino acids) are substituted with other amino acids.

In the partial peptide described herein the C-terminus may be in any form of a carboxyl group (-COOH), carboxylate (-COO), an amide ($-CONH_2$) or an ester (-COOR). Where the partial peptide described herein contains a carboxyl group (or a carboxylate) at a position other than the C-terminus, it may be amidated or esterified and such an amide or ester is also included within the partial peptide of the present invention. In this case, the ester group may be the same group as that described with respect to the C-terminus described above.

As in the 14273 described above, the partial peptide described herein further includes those in which the amino group of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycopeptides, to which sugar chains are bound, and the like.

For salts of the 14273 or its partial peptides, preferred are physiologically acceptable salts with acids or bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The 14273 or its salts may be produced by a publicly known method used to purify receptor proteins from human or mammalian cells or tissues described above, or by culturing a transformant that contains DNA encoding the 14273, as will be later described.

Furthermore, the 14273 or its salts may also be produced by the methods for synthesizing proteins, which will also be described hereinafter, or by their modifications.

Where the 14273 or its salts are produced from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

To synthesize the 14273 or its partial peptides, or salts or amides thereof according to the present invention, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the protein is cut out from the resin and at the same time, the protecting groups are removed. Then, intramolecular disulfide bond-forming reaction is performed in a highly diluted solution to obtain the objective protein or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylfonnamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20 to 50˚C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl. 4-methoxybenzyloxycarbonyl. Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups used in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids, in which the amino groups are activated in the starting material, for example, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia. The elimination of the protecting group by the acid treatment described above is carried out generally at a temperature of approximately -20 to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Fonnyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups, activation of functional groups involved in the reaction, or the like may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide or its salts of the 14273 can be manufactured by publicly known methods for peptide synthesis, or by cleaving the 14273 with an appropriate peptidase. For the methods for peptide synthesis, for example, either solid phase synthesis or liquid phase synthesis may be used. That is, the partial peptide or amino acids that can construct the 14273 are condensed with the remaining part. Where the product contains protecting groups, these protecting groups are removed to give the desired peptide. Publicly known methods for condensation and elimination of the protecting groups are described in a) - e) below.

a) M. Bodanszky & M.A. Ondetti: Peptide Synthesis, Interscience Publishers, New York (1966)
b) Schroeder & Luebke: The Peptide, Academic Press, New York (1965)
c) Nobuo Izumiya, et al.: Peptide Gosei-no-Kiso to Jikken (Basics and experiments of peptide synthesis), published by Maruzen Co. (1975)
d) Haruaki Yajima & Shunpei Sakakibara: Seikagaku Jikken Koza (Biochemical Experiment) 1, Tanpakushitsu no Kagaku (Chemistry of Proteins) IV, 205 (1977) e) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the methods above is in a free form, the peptide can be converted into an appropriate salt by a publicly known method; conversely when the peptide is obtained in a salt form, it can be converted into a free form by a publicly known method.

The polynucleotide encoding the 14273 may be any polynucleotide so long as it contains the base sequence (DNA or RNA, preferably DNA) encoding the 14273 described above. Such a polynucleotide may also be any one of DNA or RNA such as mRNA, etc. encoding the 14273, and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the 14273, mRNA of the 14273 can be quantified by for example, the publicly known method published in separate volume of Jikken Igaku 15 (7) "New PCR and its application" (1997), or by its modifications.

The DNA encoding the 14273 may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the 14273 may be any DNA, so long as it is, for example, a DNA comprising the base sequence represented by SEQ ID NO: 2,SEQ ID NO: 4 or SEQ ID NO: 9, or any DNA comprising a base sequence hybridizable to the base sequence represented by SEQ ID NO: 2,SEQ ID NO: 4 or SEQ ID NO: 9 under high stringent conditions and encoding a receptor protein which has the activities substantially equivalent to those of the 14273 consisting of the amino acid sequence represented by SEQ ID NO: 1, SEQ ID NO: 3 or SEQ ID NO: 8 (e.g., the ligand-binding activities, the signal transduction activities, etc.).

Examples of the DNA hybridizable to the DNA comprising the base sequence represented by SEQ ID NO: 2, SEQ ID NO: 4 or SEQ ID NO: 9 include a DNA comprising a base sequence having at least about 85% homology, preferably at least about 90% homology and more preferably at least about 95% homology, to the base sequence represented by SEQ ID NO: 2, SEQ ID NO: 4 or SEQ ID NO: 9; and the like.

Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70˚C, preferably about 60 to 65˚C. In particular, hybridization conditions in a sodium concentration of about 19 mM at a temperature of about 65˚C are most preferred.

More specifically, as the DNA encoding human-derived 14273 consisting of the amino acid sequence represented by SEQ ID NO:1, there may be employed a DNA consisting of the base sequence represented by SEQ ID NO: 2; etc.

As the DNA encoding mouse-derived 14273 consisting of the amino acid sequence represented by SEQ ID NO: 3, there may be employed a DNA consisting of the base ' sequence represented by SEQ ID NO: 4; etc.

As the DNA encoding rats-derived 14273 consisting of the amino acid sequence represented by SEQ ID NO: 8, there may be employed a DNA consisting of the base sequence represented by SEQ ID NO: 9; etc.

The term polynucleotide containing a part of the base sequence of the DNA encoding the 14273 or a part of the base sequence complementary to the DNA is used to mean that the polynucleotide embraces not only the DNA encoding the partial peptide described herein described below but also RNA.

Antisense polynucleotides (nucleic acids described herein) that can inhibit the replication or expression of the 14273 gene can be designed and synthesized based on the base sequence information of the cloned or determined DNA encoding the 14273. Such a polynucleotide (nucleic acid) is capable of hybridizing to RNA of the 14273 gene to inhibit the synthesis or function of said RNA or capable of modulating or controlling the expression of the 14273 gene via interaction with the 14273-associated RNA. Polynucleotides complementary to the selected sequences of RNA associated with the 14273 and polynucleotides specifically hybridizable to RNA associated with the 14273 are useful in modulating or controlling the expression of the 14273 gene in vivo and in vitro, and useful for the treatment or diagnosis of disease. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the genes for the 14273, the 5' end hairpin loop, 5' end 6-base-pair repeats, 5' end untranslated region, polypeptide translation initiation codon, protein coding region, ORF translation initiation codon, 3' end untranslated region, 3' end palindrome region, and 3' end hairpin loop, may be selected as preferred target regions, though any other region may be selected as a target in the genes for the 14273.

The relationship between the targeted nucleic acids and the polynucleotides complementary to at least a part of the target, specifically the relationship between the target and the polynucleotides hybridizable to the target, can be denoted to be "antisense." Examples of the antisense polynucleotides include polydeoxyribonucleotide containing 2-deoxy-D-ribose, polyribonucleotide containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid), described herein is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids described herein can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications CRC Press. 1993; etc.

The antisense nucleic acid described herein may contain changed or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant, described herein , the gene expression system described herein in vivo and in vitro, or the translation system of the G protein-coupled receptor protein in vivo and in vitro. The nucleic acid can be applied to cells by a variety of per se known methods.

The siRNA to the polynucleotide of the present invention is a double-stranded RNA containing a part of RNA encoding the 14273 and complementary RNA thereto. Specifically used are siRNA composed of a sense strand consisting of the.base sequence represented by SEQ ID NO:21 and an antisense strand consisting of the base sequence represented by SEQ ID NO: 22 (m14i561 in EXAMPLE 11) and the like.

The siRNA can be designed based on the sequence of the polynucleotide of the present invention and produced by a publicly known method (e.g., Nature, 411, 494, 2001) with its modification.

A ribozyme containing a part of RNA encoding the 14273 can be designed based on the sequence of the polynucleotide of the present invention and produced by a publicly known method (e.g., TRENDS in Molecular Medicine, 7, 221, 2001) with its modification. For example, the ribozyme can be produced by replacing a part of the sequence of a known ribozyme with a part of RNA encoding the 14273. Examples of such a part of RNA encoding the 14273 include sequences proximal to the consensus sequence NUX (wherein N represents all bases and X represents bases other than G), which may be cleaved by a publicly known ribozyme, and the like.

The polynucleotide encoding the partial peptide described herein may be any DNA so long as it contains the base sequence (DNA or RNA, preferably DNA) encoding the partial peptide described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide described herein may be any one of, for example, (1) a DNA containing a partial base sequence of the DNA comprising the base sequence represented by SEQ ID NO: 2, SEQ ID NO: 4 or SEQ ID NO: 9, or (2) any DNA containing a base sequence hybridizable to the base sequence represented by SEQ ID NO: 2, SEQ ID NO: 4 or SEQ ID NO: 9 under highly stringent conditions and containing a part of DNA encoding the receptor protein which has the activities (e.g., the ligand-biding activities, the signal transduction activities, etc.) substantially equivalent to those of the 14273 consisting of the amino acid sequence represented by SEQ ID NO: 1, SEQ ID NO: 3 or SEQ ID NO: 8; and the like.

Examples of the DNA that is hybridizable to the base sequence represented by SEQ ID NO: 1, SEQ ID NO: 3 or SEQ ID NO: 8 include DNA comprising a base sequence having at least about 85% homology, preferably at least about 90% homology and more preferably at least about 95% homology, to the base sequence represented by SEQ ID NO: 1, SEQ ID NO: 3 or SEQ ID NO: 8.

Homology of the amino acid sequences can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool).

The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions. More preferably, the hybridization can be carried out under high stringent conditions.

The highly stringent conditions are, for example, those in a sodium concentration at about 19 to 40 mM, preferably about 19 to 20 mM at a temperature of about 50 to 70˚C, preferably about 60 to 65˚C. In particular, hybridization conditions in a sodium concentration of about 19 mM at a temperature of about 65˚C are most preferred.

For cloning of the DNA that completely encodes the 14273 or its partial peptides (hereinafter sometimes collectively referred to as the 14273), the DNA may be either amplified by PCR using synthetic DNA primers containing a part of the base sequence encoding the 1 4273, or the DNA inserted into an appropriate vector can be selected by hybridization with a labeled DNA fragment or synthetic DNA that encodes a part or entire region of the 14273. The hybridization can be carried out, for example, according to the method described in Molecular Cloning, 2nd. (J. Sambrook et al., Cold Spring Harbor Lab. Press, 1989). The hybridization may also be performed using commercially available library in accordance with the protocol described in the attached instructions.

Conversion of the base sequence of the DNA can be effected by publicly known methods such as the ODA-LA PCR method, the Gapped duplex method or the Kunkel method or its modifications by using publicly know kits available as Mutan™-superExpress Km (manufactured by TaKaRa Shuzo Co., Ltd.), Mutan™-K (manufactured by TaKaRa Shuzo Co., Ltd.), etc.

The cloned DNA encoding the 14273 can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the 14273 can be produced, for example, by (a) excising the desired DNA fragment from the DNA encoding the 14273, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA 1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, $\lambda P_L$ promoter, lpp

promoter, etc. In the case of using bacteria of the genus Bacillus as the host, preferred example of the promoter are SPO1 promoter, SPO2 promoter, penP promoter, etc. When yeast is used as the host, preferred examples of the promoter are PHOS promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker in CHO ($dhfr^-$) cells, selection can also be made on thymidine free media.

If necessary, a signal sequence that matches with a host is added to the N-terminus of the receptor protein of the present invention. Examples of the signal sequence that can be used are PhoA signal sequence, OmpA signal sequence, etc. in the case of using bacteria of the genus Escherichia as the host; $\alpha$-amylase signal sequence, subtilisin signal sequence, etc. in the case of using bacteria of the genus Bacillus as the host; MF$\alpha$ signal sequence, SUC2 signal sequence, etc. in the case of using yeast as the host; and insulin signal sequence, $\alpha$-interferon signal sequence, antibody molecule signal sequence, etc. in the case of using animal cells as the host, respectively.

Using the vector bearing the DNA encoding the 14273 thus constructed, transformants can be produced.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)), JM103 (Nucleic Acids Research, 9, 309 (1981)], JA221 [Journal of Molecular Biology, 120, 517 (1978)], HB101 [Journal of Molecular Biology, 41, 459 (1969)), C600 [Genetics, 39, 440 (1954)], etc.

Examples of the bacteria belonging to the genus Bacillus include Bacillus subtilis M1114 [Gene, 24, 255 (1983)], 207-21 [Journal of Biochemistry, 95, 87 (1984)], etc.

Examples of yeast include Saccharomyces cereviseae AH22, AH22R', NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977)).

As the insect, for example, a larva of Bombyx mori can be used [Maeda, et al., Nature, 315, 592 (1985)].

Examples of animal cells include monkey cells COS-7, Vero, Chinese hamster cells CHO (hereinafter referred to as CHO cells), dhfr gene deficient Chinese hamster cells CHO (hereinafter briefly referred to as CHO ($dhfr^-$) cell), mouse L cells, mouse AtT-20, mouse myeloma cells, rat GH3, human FL cells, human HEK293 cells, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformant transformed with the expression vector bearing the DNA encoding the 14273 can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43˚C for about 3 to 24 hours. If necessary, the culture may be aerated or agitated.

Where the bacteria belonging to the genus Bacillus are used as the host, the transformant is cultivated generally at about 30 to 40˚C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20˚C to about 35˚C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27˚C for about 3 days to about 5 days and, if necessary and desired, the culture can be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30˚C to about 40˚C for about 15 hours to about 60 hours and, if necessary and desired, the culture can be aerated or agitated.

As described above, the 14273 can be produced into the cell, in the cell membrane or out of the cell of the transformant.

The 14273 can be separated and purified from the culture described above by the following procedures.

When the 14273 is extracted from the culture or cells, after cultivation the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the 14273 can be obtained. The buffer used for the procedures may contain a protein modifier such as urea or guanidine hydrochloride, or a surfactant such as Triton X-100™, etc. When the 14273 is secreted in the culture, after completion of the cultivation the supernatant can be separated from the transformants or cells to collect the supernatant by a publicly known method.

The 14273 contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

In the case that the 14273 thus obtained is in a free form, it can be converted into the salt by publicly known methods or modifications thereof. On the other hand, when the 14273 is obtained in the form of a salt, it can be converted into the free form or in the form of a different salt by publicly known methods or modifications thereof.

The 14273 produced by the recombinant can be treated, prior to or after the purification, with an appropriate protein modifying enzyme so that the 14273 can be appropriately modified to partially remove a polypeptide. Examples of the protein-modifying enzyme include trypsin, chymotrypsin, arginyl endopeptidase, protein kinase, glycosidase or the like.

The activity of the thus produced 14273 or salts thereof can be determined by a binding experiment to a labeled ligand, by an enzyme immunoassay using a specific antibody, or the like.

Antibodies to the 14273 may be any of polyclonal antibodies and monoclonal antibodies, as long as they are capable of recognizing the 14273.

The antibodies to the 14273 may be manufactured by publicly known methods for manufacturing antibodies or antisera, using the 14273 as an antigen.

[Preparation of monoclonal antibody]

(a) Preparation of monoclonal antibody-producing cells

The 14273 is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the receptor protein, which will be described later, with the antiserum followed by assaying the binding activity of the labeling agent bound to the antibody. The fusion may be operated, for example, by the known Koehler and Milstein method [Nature, 256, 495 (1975)]. Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40˚C, preferably at about 30 to about 37˚C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with an antigen of the receptor protein directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the receptor protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20˚C to 40˚C, preferably at about 37˚C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody described herein can be manufactured by publicly known methods or modifications thereof. For example, a complex of immunogen (the 14273 as an antigen) and a carrier protein is prepared, and a mammal is immunized with the complex in a manner similar to the method described above for the manufacture of monoclonal antibodies. The product containing the antibody to the 14273 is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

The 14273 is highly expressed in the hypophysis, adipose tissues, colorectal cancer cells, etc. and one of the ligands for the 14273 is a fatty acid or a salt thereof. Fatty acids used are oleic acid, palmitoleic acid, linoleic acid, $\alpha$-linolenic acid, $\gamma$-linolenic acid, arachidonic acid, docosahexaenoic acid (DHA), etc. Among them, palmitoleic acid, linoleic acid, $\alpha$-linolenic acid, $\gamma$-linolenic acid, etc. are preferred.

As salts of the fatty acids, there are used salts with acids (e.g., inorganic acids, organic acids, etc.), bases (e.g., alkali metals such as sodium, potassium, etc.; alkaline earth metals such as calcium, etc.) or the like, with particular preference being bases.

Hereinafter, the fatty acids or salts thereof are merely referred to as "fatty acid" in the specification.

Moreover, the expression level of the 14273 increases in the hypophysis when stress is loaded.

Therefore, the 14273, the DNA encoding the 14273 (hereinafter sometimes referred to briefly as the DNA of the present invention), the antibody to the 14273 (hereinafter sometimes referred to briefly as the antibody of the present invention) and the antisense DNA to the DNA described herein (hereinafter sometimes merely referred to as the antisense DNA described herein) have the following applications.

(1) Agent for preventing/treating diseases associated with dysfunction of the 14273

For example, when the physiological activities of the fatty acid, which is the ligand, cannot be expected in a patient (deficiency of the 14273) due to a decrease of the 14273 in the body, the amount of the 14273- can be increased in the boy of the patient a) by administering the 14273 to the patient thereby to supplement the amount of the 14273; or b) (i) by administering the DNA encoding the 14273 to the patient and expressing the same, or (ii) by inserting and expressing the DNA encoding the 14273 in the objective cells and then transplanting the cells to the patient, thus increasing the amount of the 14273 in the body of the patient, whereby the activities of the ligand can be sufficiently exhibited.

Specifically, the 14273 or DNA described herein has the activity of regulating glycerol production, from adipocytes, the activity of regulating blood glycerol, the activity of regulating lipolysis, the activity of regulating insulin resistance, the activity of regulating stress, the activity of regulating adrenocorticotropic hormone (ACTH) secretion (preferably, activities of suppressing glycerol production from adipocytes, lowering blood glycerol, inhibiting lipolysis, suppressing insulin resistance, regulating stress and suppressing adrenocorticotropic hormone (ACTH) secretion), etc. and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion, an agent for regulating lipolysis, etc. (preferably, an agent for suppressing glycerol production from adipocytes, an agent for lowering blood glycerol, an agent for suppressing lipolysis, an agent for suppressing insulin resistance, an agent for regulating stress, an agent for suppressing adrenocorticotropic hormone (ACTH) secretion).

Also, the 14273 or the DNA described herein can be used as an agent for preventing/treating, e.g., diabetes mellitus, impaired glucose tolerance, ketosis, acidosis, diabetic neuropathy, diabetic nephropathy, diabetic retinopathy, hyperlipemia (e.g., hyper-LDL-cholesterolemia, hyperlipoproteinemia and hypertriglyceridemia, hypo-HDL-cholesterolemia), arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancers (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P. neurotensin, galanin, neuropeptide Y, enkephalins, peptide YY, etc.), diseases including circulatory disease, etc. (especially, diabetes mellitus, hyperlipemia, overweight, arteriosclerosis, angina pectoris and myocardial infarction), or as an agent for regulating stress.

Moreover, the 14273 or DNA described herein can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

Furthermore, the 14273 or DNA described herein acts as an agent for suppressing adrenocorticotropic hormone (ACTH) secretion and can be used as a prophylactic/therapeutic agent for preventing/treating diseases, for example, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose tolerance, leukocytosis), adrenocortical atrophy, etc.

Since the fatty acid binds to the 14273 to exhibit, e.g., an activity of regulating glycerol production from adipocytes, an activity of regulating blood glycerol, an activity of regulating lipolysis, an activity of regulating insulin resistance, an activity, of regulating stress, an activity of regulating adrenocorticotropic hormone (ACTH) secretion (preferably, an activity of suppressing glycerol production from adipocytes, an activity of lowering blood glycerol, an activity of suppressing lipolysis, an activity of suppressing insulin resistance, an activity of regulating stress, an activity of suppressing adrenocorticotropic hormone (ACTH) secretion), etc., the fatty acid is useful as an agent for regulating blood glycerol, an agent for regulating lipolysis, an agent for regulating insulin resistance, an agent for regulating stress, an agent for regulating adrenocorticotropic hormone (ACTH) secretion (preferably, an agent for suppressing glycerol production from adipocytes, an agent for lowering blood glycerol, an agent for suppressing lipolysis, an agent for suppressing insulin resistance, an agent for regulating stress, an agent for suppressing adrenocorticotropic hormones (ACTH) secretion), etc.

Also, the fatty acid can be used as a signal transduction activity potentiator of the 14273, or, based on the signal transduction potentiating activity, can be used as an agent for preventing/treating diseases, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases (especially, diabetes mellitus, hyperlipemia, overweight, arteriosclerosis, angina pectoris or myocardial infarction), or as an agent for regulating stress.

Furthermore, the fatty acid can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, the fatty acid acts as an agent for suppressing adrenocorticotropic hormone (ACTH) secretion and can be used as an agent for preventing/treating diseases, for example, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, etc.

Diabetes mellitus includes insulin dependent (Type I) diabetes and non-insulin dependent (Type II) diabetes.
Where the fatty acid or the 14273 is used as the pharmaceuticals described above, the fatty acid or the 14273 can be prepared into pharmaceutical preparations in a conventional manner.
On the other hand, where the DNA, described herein is used as the pharmaceuticals described above, the DNA itself is administered; alternatively, the DNA is inserted into an appropriate vector such as retrovirus vector, adenovirus vector, adenovirus-associated virus vector, etc. and then administered in a conventional manner. The DNA described herein may also be administered as naked DNA, or with adjuvants to assist its uptake by gene gun or through a catheter such as a catheter with a hydrogel.
For example, a) the 14273 or b) the DNA described herein can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. These preparations can be manufactured by mixing a) the 14273 or b) the DNA described herein with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.
Additives miscible with tablets, capsules, etc. include a binder such as gelatin, cornstarch, tragacanth and gum parable, an excipient such as crystalline cellulose, a swelling agent such as cornstarch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and Eco-50), etc. Examples of the oily medium include sesame oil and soybean oil, which may also be used in combination with a dissolution aid such as benzyl benzoate, benzyl alcohol, etc. '
The pharmaceuticals described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.
Since the thus obtained pharmaceutical preparation is safe and low toxic, the preparation can be administered to human or mammals (e.g., rats, mice, rabbits, sheep, swine, bovine, cats, dogs, monkeys, etc.).
The dose of the 14273 varies depending on subject to be administered, organs to be administered, conditions, methods for administration, etc.; in oral administration, e.g., for the patient with diabetes mellitus, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, methods for administration, etc. but it is advantageous, e.g., for the patient with diabetes mellitus, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.
The dose of the DNA described herein varies depending on subject to be administered, organs to be administered, conditions, methods for administration, etc.; in oral administration, e.g., for the patient with diabetes mellitus, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, methods for administration, etc. but it is advantageous, e.g., for the patient with diabetes mellitus, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

(2) Gene diagnostic agent

By using the DNA and antisense DNA described herein as probes, abnormalities (gene abnormalities) of the DNA or mRNA encoding 14273 or its partial peptides in human or mammals (e.g., rats, mice, rabbits, sheep, swine, bovine, cats, dogs, monkeys, etc.) can be detected, and the DNA and antisense DNA are thus useful as gene diagnostic agents for the damage against the DNA or mRNA, its mutation, or its reduced expression, or increased expression or overexpression of the DNA or mRNA.

The gene diagnosis described above using the DNA or antisense DNA described herein can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc..

Where reduced expression of the 14273 is detected, e.g., by northern hybridization, it can be diagnosed that one suffers from, for example, diseases associated with dysfunction, of the 14273, especially, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases, stress, etc. (especially, diabetes mellitus, hyperlipemia, overweight, arteriosclerosis, angina pectoris or myocardial infarction), or it is highly likely to suffer from these diseases in the future. Further where reduced expression of the 14273 is detected, e.g., by northern hybridization, it can be diagnosed that one suffers from diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc., inappropriate adrenocorticotropic hormone (ACTH) secretion, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, etc., or it is highly likely to suffer from these diseases in the future.

Also, where overexpression of the 14273 is detected, e.g., by northern hybridization, it can be diagnosed that one suffers from, for example, diseases caused by excessive expression of the 14273, especially, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, stress, etc. (especially, anorexia and obesity, among others, obesity with visceral fat accumulation), or it is highly likely to suffer from these diseases in the future. Further where overexpression of the 14273 is detected by northern hybridization, it can be diagnosed that one suffers from diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, inappropriate adrenocorticotropic hormone (ACTH) secretion, connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy) or anaphylactic shock, etc., or it is highly likely to suffer from these diseases in the future.

(3) Pharmaceutical comprising the compound or its salt that changes the expression level of the 14273

By using the DNA described herein as a probe, the DNA can be used for screening the compound or its salt that changes the expression level of the 14273.

That is, the present invention provides a method of screening the compound or its salt that changes the expression level of the 14273, which comprises measuring the amount of mRNA in the 14273 contained, for example, in (i) a) blood, b) particular organs, c) tissues or cells isolated from the organs of non-human mammals or in (ii) transformants, etc.

The amount of mRNA in the 14273 can be specifically measured as follows.

(i) Normal or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., anti-dementia agents, hypotensive agents, anticancer agents, antiobestic agents, etc.) or physical stress (e.g., water-immersion stress, electric shock, light and darkness, low temperature, etc.), and the blood, particular organs (e.g., brain, liver, kidney, etc.), or tissues or cells isolated from the organs are obtained after a specified period of time.
The mRNA of the 14273 contained in the thus obtained cells is extracted from the cells, for example, in a conventional manner and quantified by means of, e.g., TaqManPCR, or may also be analyzed by northern blot technique by publicly known methods.
(ii) Transformants that express the 14273 are prepared according to the methods described above, and the mRNA of the 14273 contained in transformants can be quantified and analyzed, as described above.

The compound or its salt that changes the expression level of the 14273 can be screened by the following procedures.

(i) To normal or disease models of non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA in the 14273 contained in cells are quantified and analyzed.
(ii) Transformants are cultured in a conventional manner and a test compound is mixed in the culture medium. After a specified time (after 1 day to 7 days, preferably after 1 day to 3 days, more preferably after 2 to 3 days), the amount of mRNA in the 14273 contained in the transformants can be quantified and analyzed.

The test compounds include peptides, proteins, non-peptide compounds, synthetic compounds, fermentation products, cell extracts, plant extracts, animal tissue extracts, blood plasma, etc. These compounds may be novel or known compounds.

The compound may form salts and may be used in the form of salts with physiologically acceptable acids (e.g., inorganic acids, etc.) or bases (e.g., organic acids, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt, which is obtained by the screening methods of the present invention, is the compound or its salt that changes the expression level of the 14273. Specifically, it is (a) the compound or its salt that potentiates the cell stimulating activities mediated by the 14273 by increasing the expression level of the 14273; and (b) the compound or its salt that attenuates the cell-stimulating activities by decreasing the expression level of the 14273.

Examples of the cell-stimulating activities include the activities that promote or suppress arachidonic acid release, acetylcholine release, intracellular $Ca^{2+}$ release, intracellular cAMP production, intracellular cGMP production, inositol phosphate production, changes in cell membrane potential, phosphorylation or activation of intracellular proteins (e.g., MAP kinase), activation of c-fos, pH reduction, etc., an adrenocorticotropic hormone (ACTH) secretion suppressing activity, a glycerol production suppressing activity, a lipolysis suppressing activity, etc. Particularly preferred is the intracellular $Ca^{2+}$ level increasing activity, the intracellular cAMP production suppressing activity, phosphorylation or activation of MAP kinase, the adrenocorticotropic hormone (ACTH) secretion suppressing activity, the glycerol production suppressing activity or the lipolysis suppressing activity.

The compounds obtained by using screening method of present invention include peptides, proteins, non-peptide compounds, synthetic compounds, fermentation products, etc. They may be novel or known compounds.

As the salts of the compound which are obtained using the screening methods of the present invention, there are salts with physiologically acceptable acids (e.g., inorganic acids, etc.) or bases (e.g., organic acids, etc.) and the like. Examples of such salts used are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that increases the expression level of the 14273, which is obtained by the screening method described above has, for example, an action of regulating glycerol production from adipocytes, an action of regulating blood glycerol, an action of regulating lipolysis, an action of regulating insulin resistance, an action of regulating stress, an action of regulating adrenocorticotropic hormone (ACTH) secretion (preferably, an action of suppressing glycerol production from adipocytes, an action of lowering blood glycerol, an action of suppressing lipolysis, an action of suppressing insulin resistance, an action of regulating stress, an action of suppressing adrenocorticotropic hormone (ACTH) secretion) etc., and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for suppressing glycerol production from adipocytes, an agent for lowering blood glycerol, an agent for suppressing lipolysis, an agent for suppressing insulin resistance, an agent for regulating stress and an agent for suppressing adrenocorticotropic hormone (ACTH) secretion), etc.

Also, the compound or its salt that is obtained by the screening methods described above and increases the expression level of the 14273 is useful as a safe and low-toxic agent for preventing/treating diseases associated with dysfunction of the 14273. for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases (especially, diabetes mellitus, hyperlipemia, overweight, arteriosclerosis, angina pectoris or myocardial infarction), etc., or as an agent for regulating stress.

Furthermore, the compound or its salt that is obtained by the screening methods described above and increases the expression level of the 14273 can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

Moreover, the compound or its salt that is obtained by the screening methods described above and increases the expression level of the 14273 acts as an agent for suppressing adrenocorticotropic hormone (ACTH) secretion and can be used as an agent for preventing/treating diseases, for example, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, etc.

The compound or its salt that decreases the expression level of the 14273 has, for example, an action of regulating glycerol production from adipocytes, an action of regulating blood glycerol, an action of regulating lipolysis, an action of regulating insulin resistance, an action of regulating stress, an action of regulating adrenocorticotropic hormone (ACTH) secretion (preferably, an action of promoting glycerol production from adipocytes, an action of increasing blood glycerol, an action of promoting lipolysis, an action of promoting insulin resistance, an action of regulating stress and an action of promoting adrenocorticotropic hormone (ACTH) secretion) etc., and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for promoting glycerol production from adipocytes, an agent for increasing blood glycerol, an agent for promoting lipolysis, an agent for promoting insulin resistance, an agent for regulating stress and an agent for promoting adrenocorticotropic hormone (ACTH) secretion), etc.

The compound or its salt that decreases the expression level of the 14273 is useful as a safe and low-toxic agent for preventing/treating diseases caused by overexpression of the 14273, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases (especially, anorexia and obesity, among others, obesity with visceral fat accumulation) and the like, or as an agent for regulating stress.

Also, the compound or its salt that decreases the expression level of the 14273 can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, the compound or its salt that decreases the expression level of the 14273 acts as an agent for promoting adrenocorticotropic hormone (ACTH) secretion and is useful as an agent for preventing/treating, for example, connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy) or anaphylactic shock, etc.

Compounds or salts thereof derived from the compound or its salt obtained by the screening described above can also be used as well.

Where the compound or its salt obtained by using the screening methods of the present invention is used as a pharmaceutical composition, the compound or its salt can be prepared into a pharmaceutical preparation in a conventional manner.

For example, the compound or its salt can be used orally in the form of tablets which may be tablets, if necessary, coated with sugar, capsules, elixirs, microcapsules, etc., or parenterally in the form of injectable preparations such as a sterile solution or a suspension in water or with other pharmaceutically acceptable liquid. These preparations can be manufactured, e.g., by mixing the compound, with a physiologically acceptable know carrier, flavoring agent, excipient, vehicle, antiseptic, stabilizer, binder, etc., in a unit dosage form required in a generally accepted manner applied to making pharmaceutical preparations. The active ingredient in the preparation is controlled in such an amount that an appropriate dose is obtained within the specified range given.

Additives miscible with tablets, capsules, etc. include a binder such as gelatin, cornstarch, tragacanth or gum arabic, an excipient such as crystalline cellulose, a swelling agent such as cornstarch, gelatin, alginic acid, etc., a lubricant such as magnesium stearate, a sweetening agent such as sucrose, lactose or saccharin, a flavoring agent such as peppermint, akamono oil or cherry, and the like. When the unit dosage is in the form of capsules, liquid carriers such as oils and fats may further be used together with the additives described above. A sterile composition for injection may be formulated following a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline, an isotonic solution containing glucose and other auxiliary agents (e.g., D-sorbitol, D-mannitol, sodium chloride, etc.) or the like, which may be used in combination with an appropriate dissolution aid such as an alcohol (e.g., ethanol), a polyalcohol (e.g., propylene glycol, polyethylene glycol), a nonionic surfactant (e.g., polysorbate 80™ and HCO-50), etc. As an oily medium, for example, sesame oil, soybean oil or the like may be used, which can be used in combination with a dissolution aid such as benzyl benzoate, benzyl alcohol, etc.

Furthermore, the pharmaceuticals described above may also be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the compound or its salt that increases the amount of the 14273 may vary depending on subject to be administered, target organ, conditions, methods for administration, etc.; in oral administration, the dose for the patient with diabetes mellitus (as 60 kg body weight) is normally about 0.1 mg to about 100 mg, preferably about 1.0 to about 50 mg, and more preferably about 1.0 to about 20 mg per day. In parenteral administration, the single dose may vary depending on subject to be administered, target organ, conditions, methods for administration, etc. but it is advantageous to administer the active ingredient intravenously to the patient with diabetes mellitus (as 60 kg body weight) in a daily dose of about 0.01 to about 30 mg, preferably about 0.1 to about 20 mg, and more preferably about 0.1 to about 10 mg. For other animal species, the corresponding dose as converted per 60 kg body weight can be administered.

(4) Quantification of the 14273 and a method for diagnosis

The antibodies described herein are capable of specifically recognizing the 14273. Therefore, the antibodies can be used to quantify the 14273 in a test fluid, especially for quantification by the sandwich immunoassay, etc.

Described are, for example, the following quantification methods:

(i) a method for quantification of the 14273 in a test fluid, which comprises competitively reacting the antibody of the present invention with the test fluid and a labeled form of the 14273, and measuring a ratio of the labeled 14273 bound to the antibody; and,

(ii) a method for quantification of the 14273 in a test fluid, which comprises reacting the test fluid with the antibody of the present invention immobilized on a carrier and a labeled form of the antibody of the present invention simultaneously or sequentially, and measuring the activity of the label on the immobilized carrier.

In the quantification method (ii) described above, it is preferred that one antibody recognizes the N-terminal region of the 14273, and another antibody reacts with the C-terminal region of the 14273.

Using the monoclonal antibodies to the 14273, the 14273 can be assayed. The 14273 can also be detected by tissue staining or the like. For this purpose, the antibody molecule itself may be used, or $F(ab')_2$, Fab' or Fab fractions of the antibody molecule may also be used.

The quantification methods of the 14273 using the antibodies described herein are not particularly limited. Any quantification method can be used, so long as the amount of antibody, antigen, or antibody-antigen complex corresponding to the amount of antigen (e.g., the amount of the 14273) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [$^{125}I$], [$^{131}I$], [$^{3}H$], [$^{14}C$] and the like are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used are fluorescamine and fluorescein isothiocyanate are used. For the luminescent substance, there are used, for example, luminol, luminol derivatives, luciferin, and lucigenin. Furthermore, the biotin-avidin system may be used for binding antibody or antigen to the label.

For immobilization of antigen or antibody, physical adsorption may be used. Chemical binding methods conventionally used for insolubilization or immobilization of the 14273, enzymes or the like may also be used. For the carrier, for example, insoluble polysaccharides such as agarose, dextran, cellulose, etc.; synthetic resin such as polystyrene, polyacrylamide, silicon, etc., and glass or the like are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the 14273 in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the 14273 by the sandwich method, antibodies that bind to different sites of the 14273 are preferably used as the monoclonal antibodies of the present invention for the primary and secondary reactions. That is, in the antibodies used for the primary and secondary reactions are, for example, when the antibody used in the secondary reaction recognizes the C-terminal region of the 14273, it is preferable to use the antibody recognizing the region other than the C-terminal region for the primary reaction, e.g., the antibody recognizing the N-terminal region.

The monoclonal antibodies described herein can be used for the assay systems other than the sandwich method, for example, competitive method, immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the 14273 are constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al, ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the 14273 can be quantified with high sensitivity, using the antibodies described herein.

Where a decreased level of the 14273 is detected by quantifying the 14273 level using the antibodies described herein, it can be diagnosed that one suffers from diseases associated with dysfunction of the 14273, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases or stress (especially, diabetes mellitus, hyperlipemia, overweight, arteriosclerosis, angina pectoris or myocardial infarction), etc., or it is highly likely to suffer from these diseases in the future. In addition, where a decreased level of the 14273 is detected by quantifying the 14273 level using the antibodies of the present invention, it can be diagnosed that one suffers from diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, inappropriate adrenocorticotropic hormone (ACTH) secretion, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, etc., or it is highly likely to suffer from these diseases in the future.

Where an increased level of the 14273 is detected, it can be diagnosed that one suffers from diseases caused by overexpression of the 14273, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, stress (especially, anorexia and obesity, among others, obesity with visceral fat accumulation), etc., or it is highly likely to suffer from these diseases in the future. In addition, where an increased level of the 14273 is detected, it can be diagnosed that one suffers from diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, inappropriate adrenocorticotropic hormone (ACTH) secretion, connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy) or anaphylactic shock, etc., or it is highly likely to suffer from these diseases in the future.

(5) Methods for screening agonists to the 14273

Fatty acids bind to the 14273 so that elevation of intracellular $Ca^{2+}$ level, suppression of intracellular cAMP production, activation of MAP kinase, suppression of adrenocorticotropic hormone (ACTH) secretion, suppression of glycerol production from adipocytes, suppression of lipolysis, etc. are observed. Thus, the 14273 is useful as a reagent for searching or determining agonists (including naturally occurring ligands) to the 14273 other than fatty acids, using these intracellular signals as an indicator.

That is, the present invention provides a method of determining an agonist to the 14273, which comprises assaying the 14273-mediated intracellular $Ca^{2+}$ level increasing activity, intracellular cAMP production suppressing activity, phosphorylation or activation of MAP kinase, adrenocorticotropic hormone (ACTH) secretion suppressing activity, glycerol production suppressing activity, lipolysis suppressing activity, etc., when a test compound is brought in contact with cells containing the 14273.

Examples of test compounds include publicly known ligands (for example, angiotensin, bombesin, canavinoid, cholecystokinin, glutamine, serotonin, melatonin, neuropeptide Y, opioids, purines, vasopressin, oxytocin, PACAP (e.g., PACAP27, PACAP38), secretion, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotrienes, pancreastatin, prostaglandins, thromboxane, adenosine, adrenaline, the chemokine superfamily (e.g., the CXC chemokine subfamily such as IL-8, GROα, GROβ, GROγ, NAP-2, ENA-78, GCP-2, PF4, IP-10, Mig, PBSF/SDF-1, etc.; the CC chemokine subfamily such as MCAF/MCP-1, MCP-2, MCP-3, MCP-4. eotaxin, RANTES, MIP-1α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; the C chemokine subfamily such as lymphotactin, etc.; the CX3C chemokine subfamily such as fractalkine, etc.), endothelin, enterogastrin, histamine, neurotensin, TRH, pancreatic polypeptide, galanin, lysophosphatidic acid (LPA), sphingosine 1 -phosphate, etc.) as well as other substances, for example, tissue extracts, cell culture supernatants from humans or mammals (e.g., mice, rats, swine, bovine, sheep, monkey, etc.) or low molecular synthetic compound. For example, the tissue extract, or cell culture supernatant, is added to the 14273 while assaying the cell-stimulating activities and fractionating to finally obtain a single ligand.

The test compound may form salts and may be used in the form of salts with physiologically acceptable acids (e.g., inorganic acids, etc.) or bases (e.g., organic acids, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

Specifically, the agonist determination method of the present invention is a method of determining a compound or its salt having the 14273-mediated intracellular $Ca^{2+}$ level increasing activity, intracellular cAMP production suppressing activity, phosphorylation or activation of MAP kinase, adrenocorticotropic hormone (ACTH) secretion suppressing activity, glycerol production suppressing activity or lipolysis suppressing activity, which involves constructing the expression system of recombinant 14273 of the present invention and using the receptor-binding assay system by the aforesaid expression system.

More specifically, the present invention provides the following determination methods:

(1) a method of determining an agonist to the 14273, which comprises assaying the intracellular $Ca^{2+}$ level increasing activity, intracellular cAMP production suppressing activity, phosphorylation or activation of MAP kinase, adrenocorticotropic hormone (ACTH) secretion suppressing activity, glycerol production suppressing activity or lipolysis suppressing activity, when a test compound is brought in contact with cells (e.g., CHO cells, adipocytes, AtT-20 cells, 3T3-L1 cells) containing the 14273; and

(2) a method of determining an agonist to the 14273, which comprises assaying the intracellular $Ca^{2+}$ level increasing activity, intracellular cAMP production suppressing activity, phosphorylation or activation of MAP kinase, adrenocorticotropic hormone (ACTH) secretion suppressing activity, glycerol production suppressing activity or lipolysis suppressing activity, when a test compound is brought in contact with the 14273 expressed on a cell membrane by culturing transformants containing the 14273.

It is particularly preferred to carry out the test described above after confirming that the test compound binds to the 14273.

Where cells containing the 14273 are used in the agonist determination methods of the present invention, the cells may be fixed using glutaraldehyde, formalin, etc. The fixation can be made by publicly known methods.

The membrane fraction of the cells containing the 14273 means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about I to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the 14273 expressed and membrane components such as cell-derived phospholipids, membrane proteins, etc.

The amount of the 14273 in the cells containing the 14273 or a membrane fraction of the cells is preferably $10^3$ to $10^8$ molecules per cell, more preferably $10^5$ to $10^7$ molecules per cell. As the level of expression increases, the ligand binding activity per unit of membrane fraction (specific activity) increases so that not only the highly sensitive screening system can be constructed but also large quantities of samples can be assayed with the same lot.

To perform the agonist determination method of the present invention, the 14273-mediated intracellular $Ca^{2+}$ level increasing activity, intracellular cAMP production suppressing activity, phosphorylation or activation of MAP kinase, adrenocorticotropic hormone (ACTH) secretion suppressing activity, glycerol production suppressing activity or lipolysis suppressing activity can be assayed by publicly known methods or using assay kits commercially available. Specifically, cells containing the 14273 are first cultured on a multi-well plate, etc. Prior to the agonist determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the indicator substance (e.g., $Ca^{2+}$, cAMP, etc.) for the cell-stimulating activities due to a degrading enzyme contained in the cells, an inhibitor against such a degrading enzyme may be added prior to the assay.

The kit for agonist determination of the present invention contains the cells containing the 14273 or a membrane fraction of the cells.

The agonist to the 14273 thus determined binds to the 14273 to regulate its physiological functions and exerts, for example, an activity of regulating glycerol production from adipocytes, an activity of regulating blood glycerol, an activity of regulating lipolysis, an activity of regulating insulin resistance, an activity of regulating stress, an activity of regulating adrenocorticotropic hormone (ACTH) secretion (preferably, an activity of suppressing glycerol production from adipocytes, an activity of lowering blood glycerol, an activity of suppressing lipolysis, an activity of suppressing insulin resistance, an activity of regulating stress and an activity of suppressing adrenocorticotropic hormone (ACTH) secretion), etc., and is thus useful as a central or peripheral neural function-regulating agent or as a preventive/therapeutic agent for diseases associated with the functions of the 14273. Specifically, the agonist is useful as an agent for regulating blood glycerol, an agent for regulating lipolysis, an agent for regulating insulin resistance, an agent for regulating stress, an agent for regulating adrenocorticotropic hormone (ACTH) secretion (preferably, an agent for suppressing glycerol production from adipocytes, an agent for lowering blood glycerol, an agent for suppressing lipolysis, an agent for suppressing insulin resistance, an agent for regulating stress and an agent for suppressing adrenocorticotropic hormone (ACTH) secretion), etc.

Furthermore, since the agonist to the 14273 binds to the 14273 and regulates the physiological function of 14273, the agonist can be used as a pharmaceutical, for example, as an agent for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases (especially, diabetes mellitus, hyperlipemia, arteriosclerosis, angina pectoris or myocardial infarction), or as an agent for regulating stress, etc.

Also, the agonist to the 14273 can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

Furthermore, the agonist to the 14273 acts as an agent for suppressing adrenocorticotropic hormone (ACTH) secretion and can be used as an agent for preventing/treating diseases, for example, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, etc.

(6) Method of screening a compound (agonist, antagonist, etc.) or its salt that changes the binding property of the 14273 to its ligand and pharmaceuticals comprising the compound or its salt that changes the binding property of the 14273 to its ligand

By using the 14273, or by constructing a recombinant of the 14273 expression system and using the receptor-binding assay system via the expression system, the compound (e.g., peptide, protein, a non-peptide compound, a synthetic compound, a fermentation product, etc.) or its salt that changes the binding property of a fatty acid as a ligand to the 14273 can be screened efficiently.

Examples of these compounds include (a) a compound showing the cell stimulating activities mediated by the 14273 (a so-called agonist to the 14273). (b) a compound having no such cell stimulating activities (a so-called antagonist to the 14273), (c) a compound that potentiates the binding affinity of a fatty acid to the 14273, or (d) a compound that decreases the binding affinity of a fatty acid to the 14273, and the like.

That is, the present invention provides a method of screening a compound or its salt that changes the binding property of the 14273 to a fatty acid, which comprises comparing (i) the case wherein the 14273 is brought in contact with the fatty acid; and (ii) the case wherein the 14273 is brought in contact with the fatty acid and a test compound.

According to the screening method of the present invention, the method is characterized by assaying and comparing, e.g., the binding amounts of a fatty acid to the 14273, the cell-stimulating activities, etc. in the cases (i) and (ii).

Examples of the cell-stimulating activities include the activities that promote or suppress arachidonic acid release, acetylcholine release, intracellular $Ca^{2+}$ release, intracellular cAMP production, intracellular cGMP production, inositol phosphate production, changes in cell membrane potential, phosphorylation or activation of intracellular proteins (e.g., MAP kinase), activation of c-fos, pH reduction, etc., an adrenocorticotropic hormone (ACTH) secretion suppressing activity, a glycerol production suppressing activity, a lipolysis suppressing activity, etc. Particularly preferred are the intracellular $Ca^{2+}$ level increasing activity, the intracellular cAMP production suppressing activity, phosphorylation or activation of MAP kinase, the adrenocorticotropic hormone (ACTH) secretion suppressing activity, the glycerol production suppressing activity, the lipolysis suppressing activity, etc.

More specifically, the present invention provides the following methods.

a) A method of screening a compound or a salt thereof that changes the binding property of a fatty acid to the 14273, characterized in that which when a labeled fatty acid is brought in contact with the 14273 and when a labeled fatty acid and a test compound are brought in contact with the 14273, the binding amounts of a labeled fatty acid to the 14273 are determined and compared.

b) A method of screening a compound or a salt thereof that changes the binding property of a fatty acid to the 14273, characterized in that when a labeled fatty acid is contacted with cells containing the 14273 or a membrane fraction of the cells and when a labeled fatty acid and a test compound are contacted cells containing the 14273 or a membrane fraction of the cells, the binding amounts of the labeled fatty acid to the said cells or membrane fraction are determined and compared.

c) A method of screening a compound or a salt thereof that changes the binding property of a fatty acid to the 14273, characterized in that when a labeled fatty acid is brought in contact with the 14273 expressed on a cell membrane by culturing transformants containing the DNA of the present invention and when a labeled fatty acid and a test compound are brought in contact with the 14273 expressed on the cell membrane by culturing transformants containing the DNA of the present invention, the amounts of labeled fatty acid bound to the 14273 are determined and compared.

d) A method of screening a compound or its salt that changes the binding property of a ligand to the 14273, characterized in that when a compound capable of activating the 14273 (e.g., a fatty acid, etc.) is brought in contact with cells (e.g., CHO cells, adipocytes, AtT-20 cells or 3T3-L1 cells) containing the 14273 and when a compound capable of activating the 14273 and a test compound are brought in contact with cells containing the 14273, the 14273-mediated cell stimulating activities are determined and compared.

e) A method of screening a compound or its salt that changes the binding property of a ligand to the 14273, characterized in that when a compound capable of activating the 14273 (e.g., a fatty acid, etc.) is brought in contact with the 14273 expressed on a cell membrane by culturing transformants containing the DNA of the present invention and when a compound capable of activating the 14273 and a test compound are brought in contact with the 14273 expressed on a cell membrane by culturing transformants containing the DNA of the present invention, the receptor-mediated cell stimulating activities are determined and compared.

Further as the ligand, a compound or its salt (preferably, an agonist, more preferably, a synthetic agonist) that changes the binding property of a fatty acid to the 14273 can also be used, in place of the fatty acid. The compound or its salt that changes the binding property of a fatty acid to the 14273 can be obtained by carrying out the screening methods later described, using, e.g., a fatty acid as the ligand.

In the following screening methods, the ligand is briefly referred to as the fatty acid, including the compound or its salt that changes the binding property of the fatty acid to the 14273.

Hereinafter the screening method of the present invention will be described more specifically.

First, the 14273, which is used for the screening method of the present invention, may be any one so long as it contains the 14273 described above, though cell membrane fractions from mammalian organs are preferably employed. Since it is very difficult to acquire human-derived organs especially, human-derived 14273, etc. expressed abundantly by use of recombinants are suitable for use in the screening.

In producing the 14273, the methods described above can be used, and the DNA of the present invention is preferably expressed on mammalian cells or insect cells. As the DNA fragment encoding the target protein region, a complementary DNA may be used but is not limited thereto. For example, gene fragments or a synthetic DNA may also be used. In order to introduce the DNA fragment encoding the 14273 into host animal cells and express the same efficiently, the DNA fragment is preferably incorporated into a polyhedron promoter of nuclear polyhedrosis virus (NPV) belonging to the Baculovirus, an SV40-derived promoter, a promoter of retrovirus, a metallothionein promoter, a human heat shock promoter, a cytomegalovirus promoter, SR$\alpha$ promoter, etc. at the downstream thereof. The quantity and quality of the thus expressed receptors can be examined by a publicly known method, for example, by the method described in the literature [Nambi, P. et al., J. Biol. Chem., 267, 19555-19559, 1992].

Accordingly, in the screening method of the present invention, the substance containing the 14273 may be any 14273 purified by publicly known methods, or cells containing the 14273 or a membrane fraction of cells containing the 14273 may be used as well.

Where cells containing the 14273 are used in the screening method of the present invention, the cells may be fixed with glutaraldehyde, formalin, etc. The fixation may be carried out by a publicly known method.

The cells containing the 14273 refer to host cells in which the 14273 has been expressed. Examples of such host cells include Escherichia coli, Bacillus subtilis, yeast, insect cells, animal cells, etc.

The membrane fraction of the cells means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the 14273 expressed and membrane components such as cell-derived phospholipids, membrane proteins, etc.

The amount of the 14273 in the cells containing the 14273 or the cell membrane fraction is preferably $10^3$ to $10^8$ molecules per cell, more preferably $10^5$ to $10^7$ molecules per cell. As the level of expression increases, the ligand binding activity per unit of membrane fraction (specific activity) increases so that not only the highly sensitive screening system can be constructed but also large quantities of samples can be assayed with the same lot.

To perform a) through c) above for screening the compound that changes the binding property of a fatty acid to the 14273, for example, an appropriate fraction of the 14273 and a labeled fatty acid are required.

The 14273 fraction is preferably a fraction of naturally occurring type 14273 or a fraction of recombinant type 14273 having an activity equivalent thereto. Herein, the equivalent activity is intended to mean the ligand binding activity or the signal transduction activity, which is equivalent.

As the labeled fatty acid, a labeled fatty acid, a labeled $\beta$-alanine analogue or labeled L-camosine and the like are employed. For example, there are used fatty acids labeled with [$^3$H], [$^{125}$I], [$^{14}$C] [$^{35}$S], etc.

Specifically, the compound that changes the binding property of a fatty acid to the 14273 is screened by the following procedures. First, a preparation of the 14273 is prepared by suspending a cell containing the 14273 or a membrane fraction of the cell in a buffer appropriate for use in the screening method. Any buffer can be used so long as it does not interfere the binding affinity of a fatty acid to the 14273, including a phosphate buffer or a Tris-HCl buffer, having pH of 4 to 10 (preferably pH of 6 to 8), etc. For the purpose of minimizing non-specific binding, a surfactant such as CHAPS, Tween-80™ (Kao-Atlas Inc.), digitonin, deoxycholate, etc., may optionally be added to the buffer. Further for the purpose of suppressing the degradation of the receptor protein or ligand by a protease, a protease inhibitor such as PMSF, leupeptin, E-64 (manufactured by Peptide Institute, Inc.), pepstatin, etc. may also be added. A given amount (5,000 cpm to 500,000 cpm) of labeled fatty acid is added to 0.01 ml to 10 ml of the receptor protein solution, in which $10^{-4}$ M to $10^{-10}$ M of a test compound is co-present. To determine the amount of non-specific binding (NSB), a reaction tube containing an unlabeled fatty acid in a large excess is also provided. The reaction is carried out at approximately 0˚C to 50˚C, preferably approximately 4˚C to 37˚C for about 20 minutes to about 24 hours, preferably about 30 minutes to 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or $\gamma$-counter. When nonspecific binding (NSB) is subtracted from the count ($B_0$) where any competitive substance is absent and the resulting count ($B_0$ minus NSB) is made 100%, the test compound showing the specific binding amount (B minus NSB) of, e.g., 50% or less may be selected as a candidate compound capable of competitive inhibition.

The method d) or e) described above for screening the compound that changes the binding property of a fatty acid to the 14273 can be performed as follows. For example, the cell-stimulating activities mediated by the 14273 can be determined by a publicly known method, or using an assay kit commercially available.

Specifically, the cells containing the 14273 are first cultured in a multiwell plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the cell-stimulating activity indicator (e.g., $Ca^{2+}$, cAMP, arachidonic acid, etc.) due to a degrading enzyme contained in the cells, an inhibitor against such as a degrading enzyme may be added prior to the assay. For detecting the activities such as the cAMP production suppression activity, the baseline production in the cells is increased by forskolin or the like and the activity can be detected in terms of the suppressing effect on the cells in which the baseline production is increased.

For screening through the assay for the cell stimulating activities, cells where an appropriate type of the 14273 has been expressed are necessary. Preferred cells where the 14273 has been expressed are a cell line containing naturally occurring type 14273 and the aforesaid cell line wherein the 14273 of recombinant type has been expressed.

Examples of the test compounds include peptides, proteins, non-peptide compounds, synthetic compounds, fermentation products, cell extracts, plant extracts, animal tissue extracts, blood plasma, etc. These compounds may be either novel or publicly known compounds.

The test compound may form salts and may be used in the form of salts with physiologically acceptable acids (e.g., inorganic acids, etc.) or bases (e.g., organic acids, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The test compound which is preferably used is a compound designed to bind to the ligand-binding pocket, based on the atomic coordinate and the position of the ligand-binding pocket in the active site of the 14273. The atomic coordinate and the position of the ligand-binding pocket in the active site of the 14273 can be determined by publicly known methods or modifications thereof.

Following the method of screening the agonist to the 14273 described above, it can be confirmed whether the compound that changes the binding property of a fatty acid to the 14273 is either an agonist or an antagonist.

The kit for screening the compound or its salt that changes the binding property of a fatty acid to the 14273 is a kit comprising the 14273, cells containing the 14273, or a membrane fraction of cells containing the 14273, and the like.

Examples of the screening kit of the present invention include the following.

1. Reagent for screening

a) Assay buffer and wash buffer

Hanks' balanced salt solution (manufactured by Gibco, Inc.) supplemented with 0.05% bovine serum albumin (manufactured by Sigma, Inc.)

The solution is sterilized by filtration through a 0.45 μm filter, and stored at 4°C or may be prepared at use.

b) The 14273 sample

CHO cells wherein the 14273 has been expressed are passaged in a 12-well plate at a density of 5 x $10^5$ cells/well followed by culturing at 37°C under 5% $CO_2$ and 95% air for 2 days.

c) Labeled fatty acid

A fatty acid labeled with [$^3$H], [$^{125}$I], [$^{14}$C), [$^{35}$S], etc., commercially available.

The labeled fatty acid is stored at 4°C or -20°C in the state of an aqueous solution and diluted to 1 μM with the assay buffer upon use.

d) Standard fatty acid solution

A fatty acid is dissolved in and adjusted to 1 mM with PBS containing 0.1 % bovine serum albumin (manufactured by Sigma, Inc.) and stored at -20°C.

2. Assay method

a) CHO cells wherein the 14273 has been expressed are cultured in a 12-well culture plate and washed twice with 1 ml of the assay buffer, and 490 μl of the assay buffer is added to each well.
b) After adding 5 μl of $10^{-3}$ - $10^{-10}$ M solution of a test compound. 5 μl of the labeled fatty acid is added to the mixture, and the cells are incubated at room temperature for an hour. To determine the amount of the non-specific binding, 5 μl of $10^{-3}$ M fatty acid is added in place of the test compound.
c) The reaction solution is removed, and the wells are washed 3 times with I ml of the wash buffer. The labeled ligand bound to the cells is dissolved in 0.2N NaOH-1% SDS, and mixed with 4 ml of liquid scintillator A (manufactured by Wako Pure Chemical Industries, Ltd.)
d) The radioactivity is measured using a liquid scintillation counter (manufactured by Beckman Co.), and the percent maximum binding (PMB) is calculated by the equation below.

$$PMB = [(B - NSB)/(B_0 - NSB)] \times 100$$

PMB : Percent maximum binding
B : Value obtained in the presence of a test compound
NSB : Non-specific binding
$B_0$ : Maximum binding

The compound or its salt, which is obtained by using the screening methods or the screening kits of the present invention, is a compound or its salt that changes the binding property of a fatty acid to the 14273. Specifically, the compound is: (a) a compound or its salt having the cell-stimulating activities mediated by the G protein-coupled receptor (a so-called agonist to the 14273); (b) a compound or its salt having no cell stimulating activity (a so-called antagonist to the 14273); (c) a compound or its salt that potentiates the binding affinity of a fatty acid to the 14273; or (d) a compound or its salt that reduces the binding affinity of a fatty acid to the 14273. The compound obtained by using the screening methods or screening kits of the present invention may be fatty acids other than the ligand fatty acid.
These compounds obtained by using the screening methods or the screening kits of the present invention may be peptides, proteins, non-peptide compounds, synthetic compounds, fermentation products, and may be novel or known compounds.
As salts of the compounds obtained by using the screening methods or the screening kits of the present invention, there are employed salts with physiologically acceptable acids (e.g., inorganic acids, etc.) or bases (e.g., organic acids, etc.), with particular preference in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.
Since the agonists to the 14273 have the same physiological activities as the fatty acid, which is a ligand to the 14273, the agonists are useful as safe and low toxic pharmaceuticals, correspondingly to the physiological activities possessed by the fatty acid.
Since the antagonists to the 14273 can suppress the physiological activities possessed by the fatty acid, which is a ligand to the 14273, the antagonists are useful as safe and low toxic pharmaceuticals to suppress the physiological activities of the fatty acid.
The compound or its salt that potentiates the binding affinity of the fatty acid to the 14273 can potentiate the physiological activities possessed by the fatty acid as a ligand to the 14273, and is thus useful as a safe and low toxic pharmaceutical correspondingly to the physiological activities the fatty acid possesses.
The compound or its salt that reduces the binding affinity of the fatty acid to the 14273 can reduce the physiological activities possessed by the fatty acid as a ligand to the 14273, and is thus useful as a safe and low toxic pharmaceutical to suppress the physiological activities the fatty acid possesses.
Specifically, (i) the agonist to the 14273 or (ii) the compound or its salt that potentiates the binding affinity of the fatty acid to the 14273, which is obtained by using the screening methods or screening kits of the present invention, has, for example, an activity of regulating glycerol production from adipocytes, an activity of regulating blood glycerol, an activity of regulating lipolysis, an activity of regulating insulin resistance, an activity of regulating stress, an activity of regulating adrenocorticotropic hormone (ACTH) secretion (preferably, an activity of suppressing glycerol production from adipocytes, an activity of lowering blood glycerol, an activity of suppressing lipolysis, an activity of suppressing insulin resistance, an activity of regulating stress and an activity of suppressing adrenocorticotropic hormone (ACTH) secretion), etc., and is thus useful as an agent for suppressing glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for suppressing glycerol production from adipocytes, an agent for lowering blood glycerol, an agent for suppressing lipolysis, an agent for suppressing insulin resistance and an agent for regulating stress, an agent for suppressing adrenocorticotropic hormone (ACTH) secretion), etc.

In addition, (i) the agonist to the 14273 or (ii) the compound or its salt that potentiates the binding affinity of the fatty acid to the 14273, which is obtained by using the screening methods or screening kits of the present invention, is useful as an agent for preventing/treating, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of antidiuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases (especially, diabetes mellitus, hyperlipemia, overweight, arteriosclerosis, angina pectoris or myocardial infarction), or as an agent for regulating stress.

Moreover, (i) the agonist to the 14273 or (ii) the compound or its salt that potentiates the binding affinity of the fatty acid to the 14273, which is obtained by using the screening methods or screening kits of the present invention, can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

Furthermore, (i) the agonist to the 14273 or (ii) the compound or its salt that potentiates the binding affinity of the fatty acid to the 14273, which is obtained by using the screening methods or screening kits of the present invention, acts as an agent for suppressing adrenocorticotropic hormone (ACTH) secretion and can be used as an agent for preventing/treating diseases, for example, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis) or adrenocortical atrophy, etc.

(i) The antagonist to the 14273 or (ii) the compound or its salt that reduces the binding affinity of the fatty acid to the 14273, which is obtained by using the screening methods or screening kits of the present invention, has, for example, an action of regulating glycerol production from adipocytes, an action of regulating blood glycerol, an action of regulating lipolysis, an action of regulating insulin resistance, an action of regulating stress, an action of regulating adrenocorticotropic hormone (ACTH) secretion (preferably, an action of promoting glycerol production from adipocytes, an action of increasing blood glycerol, an action of promoting lipolysis, an action of promoting insulin resistance, an action of regulating stress and an action of promoting adrenocorticotropic hormone (ACTH) secretion) etc., and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for promoting glycerol production from adipocytes, an agent for increasing blood glycerol, an agent for promoting lipolysis, an agent for promoting insulin resistance, an agent for regulating stress and an agent for promoting adrenocorticotropic hormone (ACTH) secretion), etc.

Furthermore, (i) the antagonist to the 14273 or (ii) the compound or its salt that reduces the binding affinity of the fatty acid to the 14273, which is obtained by using the screening methods or screening kits of the present invention, is useful as an agent for preventing/treating, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of antidiuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, etc. (especially, anorexia and obesity, among others, obesity with visceral fat accumulation), etc., or as an agent for regulating stress.

Also, (i) the antagonist to the 14273 or (ii) the compound or its salt that reduces the binding affinity of the fatty acid to the 14273, which is obtained by using the screening methods or screening kits of the present invention, can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, (i) the antagonist to the 14273 or (ii) the compound or its salt that reduces the binding affinity of the fatty acid to the 14273, which is obtained by using the screening methods or screening kits of the present invention, acts as an agent for promoting adrenocorticotropic hormone (ACTH) secretion and is useful as a pharmaceutical such as an agent for preventing/treating, e.g., connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy) or anaphylactic shock, etc.

Compounds or salts thereof derived from the compound or its salt obtained by the screening described above can also be used as well.

The compound or its salt obtained by using the screening methods or screening kits of the present invention can be used in combination with the compound or its salt that changes the expression level of the 14273 later described, pharmaceutical drugs such as other drugs for the diseases described above, other antidiabetics, therapeutic agents for diabetic complications, antihyperlipidemic agents, hypotensive agents, antiobestic agents, diuretic agents, chemotherapeutic agents, immunotherapeutic agents and the like (hereinafter sometimes referred to briefly as the concomitant drug). In this case, the time of administration of the compound or its salt obtained using the screening methods or screening kits of the present invention and that of the concomitant drug are not limited, and they may be administered simultaneously or with time intervals to the subject to be administered. The dose of the concomitant drug can be appropriately chosen, based on the dose clinically employed. The ratio of the compound or its salt obtained using the screening methods or screening kits of the present invention to the concomitant drug can be appropriately chosen according to the subject to be administered, administration route, target disease, clinical conditions, combination, and other factors. In the case where the subject to be administered is human, for instance, the concomitant drug may be used in an amount of 0.01 to 100 parts by weight per 1 part by weight of, e.g., the agonist.

Examples of the other antidiabetics include insulin preparations (e.g., animal insulin preparations obtained by extraction from the bovine or porcine pancreas; human insulin preparations synthesized by a genetic engineering technique using Escherichia coli or yeast; insulin-zinc; protamine-insulin-zinc; insulin fragments or derivatives (e.g., INS-1, etc.), insulin sensitivity enhancers (e.g., pioglitazone hydrochloride, troglitazone, rosiglitazone or its maleate, JTT-501, MCC-555, YM-440, GI-262570, KRP-297, FK-614, CS-011, (γE)-γ-[[[4-[(5-methyl-2-phenyl-4-oxazolyl)methoxy]phenyl]methoxy]imino]benzenebutanoi c acid, etc.), α-glucosidase inhibitors (e.g., voglibose, acarbose, miglitol, emiglitate, etc.), biguanides (e.g., phenformin, metformin, buformin, etc.), sulfonylureas (e.g., tolbutamide, glibenclamide, gliclazide, chlorpropamide, tolazamide, acetohexamide, glyclopyramide, glimepiride, etc.), and other insulin secretagogues (e.g., repaglinide, senaglinide, mitiglinide or its calcium salt hydrate, GLP-1, nateglinide, etc.), dipeptidylpeptidase IV inhibitors (e.g., NVP-DPP-278, PT-100, P32/98, etc.), β3 agonists (e.g., CL-316243, SR-5861 1-A, UL-TG-307, AJ-9677, AZ40140, etc.), amylin agonists (e.g., pramlintide, etc.), phosphotyrosine phosphatase inhibitors (e.g., vanadic acid, etc.), gluconeogenesis inhibitors (e.g., glycogen phosphorylase inhibitors, glucose-6-phosphatse inhibitors, glucagon antagonists, etc.), SGLT (sodium-glucose cotransporter) inhibitors (e.g., T-1095, etc.), and the like.

Examples of the therapeutic agents for diabetic complications include aldose reductase inhibitors (e.g., tolrestat, epalrestat, zenarestat, zopolrestat, fidarestat (SNK-860), minalrestat (ARI-509), CT-112, etc.), neurotrophic factors (e.g.,

NGF, NT-3. etc.), AGE inhibitors (e.g., ALT-945, pimagedine, pyradoxamine, N-phenacylthiazolinium bromide (ALT-766), EXO-226, etc.), active oxygen scavengers (e.g., thioctic acid, etc.), cerebral vasodilators (e.g., tioplide, etc.), and the like.

Examples of the antihyperlipidemic agents include statin compounds which are cholesterol synthesis inhibitors (e.g., pravastatin, simvastatin, lovastatin, atorvastatin, fluvastatin, cerivastatin or their salts (e.g., sodium salt, etc.), etc.), squalene synthase inhibitors or fibrate compounds having a triglyceride lowering action (e.g., bezafibrate, clofibrate, simfibrate, clinofibrate, etc.), and the like.

Examples of the hypotensive agents include angiotensin convertase inhibitors (e.g., captopril, enalapril, delapril, etc.), angiotensin II antagonists (e.g., losartan, candesartan cilexetil, etc.), calcium antagonists (e.g., manidipine, nifedipine, amlodipine, efonidipine, nicardipine, etc.), clonidine, and the like.

Examples of the antiobestic agents include antiobestic drugs acting on the central nervous system (e.g., dexfenfluramine, fenfluramine, phentermine, sibutramine, amfepramon, dexamphetamine, mazindol, phenylpropanolamine, clobenzorex, etc.), pancreatic lipase inhibitors (e.g., orlistat, etc.), β3 agonists (e.g., CL-316243, SR-58611-A, UL-TG-307, AJ-9677, AZ40140, etc.), anorectic peptides (e.g., leptin, CNTF (ciliary neurotrophic factor), etc.), cholecystokinin agonists (e.g., lintitript, FPL-15849, etc.), and the like.

Examples of the diuretic agents include xanthine derivatives (e.g., theobromine sodium salicylate, theobromine calcium salicylate, etc.), thiazide preparations (e.g., ethiazide, cyclopenthiazide, trichlormethiazide, hydrochlorothiazide, hydroflumethiazide, benzylhydrochlorothiazide, penflutizide, polythiazide, methyclothiazide, etc.), antialdosterone preparations (e.g., spironolactone, triamterene, etc.), carbonic anhydrase inhibitors (e.g., acetazolamide, etc.), chlorobenzenesulfonamide preparations (e.g., chlorthalidone, mefruside, indapamide, etc.), azosemide, isosorbide, ethacrynic acid, piretanide, bumetanide, furosemide, and the like.

Examples of the chemotherapeutic agents include alkylating agents (e.g., cyclophosphamide, ifosamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, taxol, etc.), cisplatin, carboplatin, etopoxide, etc. Among others, 5-fluorouracil derivatives such as Furtulon and Neo-Furtulon are preferable.

Examples of the immunotherapeutic agents include microorganism- or bacterium-derived components (e.g., muramyl dipeptide derivatives, picibanil, etc.), immunopotentiator polysaccharides (e.g., lentinan, schizophyllan, krestin, etc.), genetically engineered cytokines (e.g., interferons, interleukins (IL), etc.), colony stimulating agents (e.g., granulocyte colony stimulating factor, erythropoietin, etc.), and the like. Among others, IL-1, IL-2, IL-12, etc. are preferable.

In addition, agents whose effects of ameliorating cachexia have been confirmed in animal models or clinically, namely, cyclooxygenase inhibitors (e.g., indomethacin, etc.) (Cancer Research, 49, 5935-5939, 1989), progesterone derivatives (e.g., megestrol acetate, etc.) (Journal of Clinical Oncology, 12, 213-225, 1994), glucocorticoids (e.g., dexamethasone, etc.), metoclopramide preparations, tetrahydrocannabinol preparations (the above references are applied to both), fat metabolism ameliorating agents (e.g., eicosapentaenoic acid, etc.) [British Journal of Cancer, 68, 314-318, 1993), growth hormones, IGF-1, and antibodies to the cachexia-inducing factor TNF-α, LIF, IL-6 or oncostatin M, can also be used in combination with the preparation of the present invention.

Furthermore, glycation inhibitors (e.g., ALT-711, etc.), nerve regeneration stimulators (e.g., Y-128, VX853, prosaptide, etc.), antidepressants (e.g., desipramine, amitriptyline, imipramine), anticonvulsants (e.g., lamotrigine), antiarrhythmics (e.g., mexiletine), acetylcholine receptor ligands (e.g., ABT-594), endothelin receptor antagonists (e.g., ABT-627), monoamine reuptake inhibitors (e.g., tramadol), narcotic analgesics (e.g., morphine), GABA receptor agonists (e.g., gabapentin),α2 receptor agonists (e.g., clonidine), topical analgesics (e.g., capsaicin), protein kinase C inhibitors (e.g., LY-333531), antianxiety drugs (e.g., benzothiazepine), phosphodiesterase inhibitors (e.g., sildenafil), dopamine receptor agonists (e.g., apomorphine), etc. can also be used in combination with the preparation of the present invention.

Where the compound or its salt, which is obtained by using the screening methods or screening kits of the present invention, is used as the pharmaceutical composition described above, the compound or its salt can be prepared into a pharmaceutical preparation in a conventional manner.

For example, the compound or its salt can be used orally in the form of tablets which may be tablets, if necessary, coated with sugar, capsules, elixirs, microcapsules, etc., or parenterally in the form of injectable preparations such as a sterile solution or a suspension in water or with other pharmaceutically acceptable liquid. These preparations can be manufactured, e.g., by mixing the compound, with a physiologically acceptable known carrier, flavoring agent, excipient, vehicle, antiseptic, stabilizer, binder, etc., in a unit dosage form required in a generally accepted manner applied to making pharmaceutical preparations. The active ingredient in the preparation is controlled in such an amount that an appropriate dose is obtained within the specified range given.

Additives miscible with tablets, capsules, etc. include a binder such as gelatin, cornstarch, tragacanth or gum arabic, an excipient such as crystalline cellulose, a swelling agent such as cornstarch, gelatin, alginic acid, etc., a lubricant such as magnesium stearate, a sweetening agent such as sucrose, lactose or saccharin, a flavoring agent such as peppermint, akamono oil or cherry, and the like. When the unit dosage is in the form of capsules, liquid carriers such as oils and fats may further be used together with the additives described above. A sterile composition for injection may be formulated following a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline, an isotonic solution containing glucose and other auxiliary agents (e.g., D-sorbitol, D-mannitol, sodium chloride, etc.) or the like, which may be used in combination with an appropriate dissolution aid such as an alcohol (e.g., ethanol), a polyalcohol (e.g., propylene glycol, polyethylene glycol), a nonionic surfactant (e.g., polysorbate 80™ and HCO-50), etc. As an oily medium, for example, sesame oil, soybean oil or the like may be used, and may be used in combination with a dissolution aid such as benzyl benzoate, benzyl alcohol, etc.

Furthermore, the drugs described above may also be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the agonist to the 14273 may vary depending on the subject to be administered, target organ, conditions, route for administration, etc.; in oral administration, the dose for the patient with diabetes mellitus (as 60 kg body weight) is normally about 0.1 mg to about 100 mg, preferably about 1.0 to about 50 mg, and more preferably about 1.0 to about 20 mg per day. In parenteral administration, the single dose may vary depending on subject to be administered, target organ, conditions, method for administration, etc. but it is advantageous to administer the active ingredient intravenously to the patient with diabetes mellitus (as 60 kg) in a daily dose of about 0.01 to about 30 mg, preferably about 0.1 to about 20 mg, and more preferably about 0.1 to about 10 mg. For other animal species, the corresponding dose as converted per 60 kg body weight can be administered.

(7) Methods for elucidation of the action mechanism of various drugs

By using the 14273, it can be confirmed whether or not various drugs exhibit their pharmacological effects mediated by the 14273.

Thus, described are also the following methods.

(1) A method for confirmation that (i) a drug for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunction, cancer, deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases (especially, diabetes mellitus, hyperlipemia, overweight, arteriosclerosis, angina pectoris, myocardial infarction, anorexia or obesity), (ii) a drug for regulating stress, a drug for regulating glycerol production from adipocytes, a drug for regulating blood glycerol, a drug for regulating lipolysis or a drug for regulating insulin resistance, (iii) a drug for preventing/treating diseases such as arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc., (iv) a drug for regulating adrenocorticotropic hormone (ACTH) secretion (e.g., a secretion suppressing drug, a secretagogue), (v) a drug for preventing/treating ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis), adrenocortical atrophy, etc., or (vi) a drug for preventing/treating connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy), anaphylactic shock, etc. binds to the 14273 or a salt thereof, which comprises using said receptor protein.

(2) A method for confirmation that a drug for preventing/treating diabetes mellitus, impaired glucose tolerance, ketosis, acidosis, diabetic neuropathy, diabetic nephropathy, (i) diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormone (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases (especially, diabetes mellitus, hyperlipemia, overweight, arteriosclerosis, angina pectoris or myocardial infarction), (ii) a drug for regulating stress, a drug for suppressing glycerol production from adipocytes, a drug for lowering blood glycerol, a drug for suppressing lipolysis or a drug for suppressing insulin resistance, (iii) a drug for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc., (iv) a drug for suppressing adrenocorticotropic hormone (ACTH) secretion, or (v) a drug for preventing/treating diseases such as ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis), adrenocortical atrophy, etc. is an agonist to the 14273 or a salt thereof, which comprises using said receptor protein.

(3) A method of confirmation that (i) a drug for preventing/treating diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, stress, etc. (especially, anorexia and obesity, among others, obesity with visceral fat accumulation), (ii) drug for regulating stress, a drug for promoting glycerol production from adipocytes, a drug for increasing blood glycerol, a drug for promoting lipolysis or a drug for promoting insulin resistance, (iii) a drug for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc., (iv) a drug for promoting adrenocorticotropic hormone (ACTH) secretion, or (v) a drug for preventing/treating connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy) or anaphylactic shock, etc. is an antagonist to the 14273 or a salt thereof, which comprises using said receptor protein.

(4) The method for confirmation according to (1) to (3), wherein the binding amount of each drug to the 14273 is measured when the drug is brought in contact with the 14273.

This confirmation method can be performed by using the drug described above in place of the test compound in the aforesaid screening methods for the compound or its salt that changes the binding property of the ligand to the 14273.

The kit used for the confirmation method of the present invention comprises the drug described above in place of the test compound, in the aforesaid screening kits for the compound that changes the binding properties of the ligand to the 14273.

By using the confirmation method of the present invention as such, it can be confirmed that various drugs commercially available or under development exhibit their pharmacological effects mediated by the 14273.

(8) Pharmaceutical comprising the compound or its salt that changes the amount of the 14273 or its partial peptide in cell membrane

The antibody of the present invention is capable of specifically recognizing the 14273 and can be used for screening the compound or its salt that changes the amount of the 14273 in the cell membrane.

That is, described are further , for example, the following methods:

(i) a method of screening the compound or its salt that changes the amount of the 14273 in the cell membrane, which comprises measuring the amount of the 14273 contained in a) blood, b) particular organs or c) a cell membrane fraction isolated after disrupting tissues or cells isolated from the organs of non-human mammals;

(ii) a method of screening the compound or its salt that changes the amount of the 14273 in the cell membrane, which comprises disrupting transformants, etc. expressing the 14273, isolating the cell membrane fraction and quantifying the 14273 contained in the cell membrane fraction;

(iii) a method of screening the compound or its salt that changes the amount of the 14273 in the cell membrane, which comprises preparing a slice of a) blood, b) particular organs or c) tissues, cells, etc. isolated from organs of non-human mammals and quantifying the stained receptor protein on the cell surface using immunostaining assay thereby to confirm the protein on the cell membrane; and,

(iv) a method of screening the compound or its salt that changes the amount of the 14273 in the cell membrane, which comprises preparing a slice of a transformant expressing the 14273 and quantifying the stained receptor protein on the cell surface using immunostaining assay thereby to confirm the protein on the cell membrane.

Specifically, the 1.4273 contained in the cell membrane fraction can be quantified as follows.

(i) Normal or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., an anti-dementia drug, a hypotensive drug, an anticancer agent, an antiobestic drug, etc.) or physical stress (e.g., water-immersion stress, electric shock, light and darkness, low temperature, etc.), and the blood, particular organs (e.g., brain, liver, kidney, etc.), or tissues or cells isolated from the organs are obtained after a specified period of time. The obtained organs, tissues, cells or the like are suspended in, for example, an appropriate buffer (e.g., Tris hydrochloride buffer, phosphate buffer, HEPES buffer, etc.), and the organs, tissues or cells are disrupted, and the cell membrane fraction is obtained using surfactants (e.g., Triton-X 100™, Tween 20™) and further using techniques such as centrifugal separation, filtration, column fractionation, etc. The cell membrane fraction means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours.

The precipitate thus obtained is used as the membrane fraction. The membrane fraction is rich in the 14273 expressed and membrane components such as cell-derived phospholipids, membrane proteins, etc.
The 14273 contained in the cell membrane fraction can be quantified by, for example, the sandwich immunoassay, western blot analysis, etc. using the antibody of the present invention.
The sandwich immunoassay can be performed as described above, and western blotting can be performed by publicly known methods.
(ii) Transformants expressing the 14273 are prepared by the method described above, and the 14273 contained in the cell membrane fraction can be quantified.

The compound or its salt that changes the amount of the 14273 in cell membranes can be screened as follows.

(i) Normal non-human mammals or disease models of non-human mammals are administered with a test compound at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of the 14273 in the cell membranes can be quantified.
(ii) Transformants are cultured in a conventional manner and a test compound is mixed in the culture medium. After a specified time (after 1 day to 7 days, preferably after 1 day to 3 days, more preferably after 2 to 3 days), the amount of the 14273 in the cell membranes can be quantified.
Specifically, the 14273 contained in cell membrane fractions is confirmed as follows.
(iii) Normal non-human mammals or disease models of non-human mammals (e.g., mice, rats, rabbits, sheep, swine, bovine, cats, dogs, monkeys, etc., more specifically, rat with dementia, obese mouse, rabbit with arteriosclerosis, tumor-bearing mouse, etc.) are administered with drugs (e.g., anti-dementia agents, hypotensive agents, anticancer agents, antiobestic agents, etc.) or physical stress (e.g., water-immersion stress, electric shock, light and darkness, low temperature, etc.) or the like, and blood or particular organ (e.g., brain, liver, kidney, etc.), or the tissues or cells isolated from the organ are obtained after a specified period of time. Tissue sections are prepared from the thus obtained organs, tissues, cells, etc. in a conventional manner followed by immunostaining with the antibody of the present invention. The staining intensity of the receptor protein on the cell surface is quantified to confirm the protein on the cell membrane, whereby the amount of the 14273 in the cell membranes can be confirmed quantitatively or qualitatively.
(iv) The confirmation can also be made by the similar method, using transformants expressing the 14273.

The compounds include peptides, proteins, non-peptide compounds, synthetic compounds, fermentation products, cell extracts, plant extracts, animal tissue extracts, plasma, etc. These compounds may be novel or known compounds.
The test compound may form salts and may be used in the form of salts with physiologically acceptable acids (e.g., inorganic acids, etc.) or bases (e.g., organic acids, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.
The compounds or their salts obtained by the screening methods of the present invention are compounds or their salts that have the action of changing the amount of the 14273 in the cell membranes. Specifically, these compounds are: (a) compounds or their salts that increase the amount of the 14273 in the cell membranes thereby to potentiate the cell stimulating activities mediated by the 14273 and (b) compounds or their salts that decrease the amount of the 14273 in the cell membranes thereby to attenuate the cell stimulating activities.
The compounds obtained by using the screening methods of the present invention may be peptides, proteins, non-peptide compounds, synthetic compounds, fermentation products, and these compounds may be novel or publicly known compounds.
The compounds obtained by using the screening methods of the present invention may form salts and may be used in the form of salts with physiologically acceptable acids (e.g., inorganic acids, etc.) or bases (e.g., organic acids, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.
The compound or its salt that increases the amount of the 14273 in the cell membranes is useful as a safe and low toxic preventive/therapeutic drug for diseases associated with dysfunction of the 14273.

The compound or its salt that decreases the amount of the 14273 in the cell membranes is useful as a safe and low toxic preventive/therapeutic drug for diseases caused by overexpression of the 14273.

Specifically, the compound or its salt that increases the amount of the 14273 has an activity of regulating glycerol production from adipocytes, an activity of regulating blood glycerol, an activity of regulating lipolysis, an activity of regulating insulin resistance, an activity of regulating stress, an activity of regulating adrenocorticotropic hormone (ACTH) secretion (preferably, an activity of suppressing glycerol production from adipocytes, an activity of lowering blood glycerol, an activity of suppressing lipolysis, an activity of suppressing insulin resistance, an activity of regulating stress and an activity of suppressing adrenocorticotropic hormone (ACTH) secretion), etc., and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for suppressing glycerol production from adipocytes, an agent for lowering blood glycerol, an agent for suppressing lipolysis, an agent for suppressing insulin resistance, an agent for regulating stress and an agent for suppressing adrenocorticotropic hormone (ACTH) secretion), etc.

Also, the compound or its salt that increases the amount of the 14273 can be used an agent for preventing/treating diseases, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases, etc. (especially, diabetes mellitus, hyperlipemia, overweight, arteriosclerosis, angina pectoris or myocardial infarction), or as an agent for regulating stress.

Furthermore, the compound or its salt that increases the amount of the 14273 can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

Moreover, the compound or its salt that increases the amount of the 14273 acts as an agent for suppressing adrenocorticotropic hormone (ACTH) secretion and can be used as an agent for preventing/treating diseases, for example, ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis), adrenocortical atrophy, etc.

The compound or its salt that decreases the amount of the 14273 has, for example, an action of regulating glycerol production from adipocytes, an action of regulating blood glycerol, an action of regulating lipolysis, an action of regulating insulin resistance, an action of regulating stress, an action of regulating adrenocorticotropic hormone (ACTH) secretion (preferably, an action of promoting glycerol production from adipocytes, an action of increasing blood glycerol, an action of promoting lipolysis, an action of promoting insulin resistance, an action of regulating stress and an action of promoting adrenocorticotropic hormone (ACTH) secretion) etc., and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for promoting glycerol production from adipocytes, an agent for increasing blood glycerol, an agent for promoting lipolysis, an agent for promoting insulin resistance, an agent for regulating stress and an agent for promoting adrenocorticotropic hormone (ACTH) secretion), etc.

Also, the compound or its salt that decreases the amount of the 14273 can be used as an agent for preventing/treating, e.g., diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, etc. (especially, anorexia and obesity, among others, obesity with visceral fat accumulation), or as an agent for regulating stress.

Furthermore, the compound or its salt that decreases the amount of the 14273 can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, the compound or its salt that decreases the amount of the 14273 acts as an agent for promoting adrenocorticotropic hormone (ACTH) secretion and is thus useful as pharmaceutical such as an agent for preventing/treating, e.g., connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy), anaphylactic shock, etc.

Compounds or salts thereof derived from the compound or its salt obtained by the screening described above can also be used as well.

Where the compound or its salt obtained by using the screening methods of the present invention is used in the form of a pharmaceutical composition, the compound or its salt can be prepared into a pharmaceutical preparation in a conventional manner.

For example, the compound or its salt can be used orally in the form of tablets which may be tablets, if necessary, coated with sugar, capsules, elixirs, microcapsules, etc., or parenterally in the form of injectable preparations such as a sterile solution or a suspension in water or with other pharmaceutically acceptable liquid. These preparations can be manufactured, e.g., by mixing the compound or its salt, with a physiologically acceptable known carrier, flavoring agent, excipient, vehicle, antiseptic, stabilizer, binder, etc., in a unit dosage form required in a generally accepted manner applied to making pharmaceutical preparations. The active ingredient in the preparation is controlled in such an amount that an appropriate dose is obtained within the specified range given.

Additives miscible with tablets, capsules, etc. include a binder such as gelatin, cornstarch, tragacanth or gum arable, an excipient such as crystalline cellulose, a swelling agent such as cornstarch, gelatin, alginic acid, etc., a lubricant such as magnesium stearate, a sweetening agent such as sucrose, lactose or saccharin, a flavoring agent such as peppermint, akamono oil or cherry, and the like. When the unit dosage is in the form of capsules, liquid carriers such as oils and fats may further be used together with the additives described above. A sterile composition for injection may be formulated following a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline, an isotonic solution containing glucose and other auxiliary agents (e.g., D-sorbitol, D-mannitol, sodium chloride, etc.) or the like, which may be used in combination with an appropriate dissolution aid such as an alcohol (e.g., ethanol), a polyalcohol (e.g., propylene glycol, polyethylene glycol), a nonionic surfactant (e.g., polysorbate 80™ and HCO-50), etc. As an oily medium, for example, sesame oil, soybean oil or the like, may be used and they can be used in combination with a dissolution aid such as benzyl benzoate, benzyl alcohol, etc.

Furthermore, the preventive/therapeutic drug described above may also be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the compound or its salt that increases the amount of the 14273 in the cell membranes may vary depending on subject to be administered, target organ, conditions, methods for administration, etc.; in oral administration, the dose for the patient with diabetes mellitus (as 60 kg body weight) is normally about 0.1 mg to about 100 mg, preferably about 1.0 to about 50 mg, and more preferably about 1.0 to about 20 mg per day. In parenteral administration, the single dose may vary depending on subject to be administered, target organ, conditions, methods for administration, etc. but it is advantageous to administer the active ingredient intravenously to the patient with diabetes mellitus (as 60 kg body weight) in a daily dose of about 0.01 to about 30 mg, preferably about 0.1 to about 20 mg, and more preferably about 0.1 to about 10 mg. For other animal species, the corresponding dose as converted per 60 kg body weight can be administered.

(9) Pharmaceutical comprising the antibody to the 14273

The neutralizing activity of the antibody to the 14273 means the activity of inactivating the signal transduction function in which the 14273 is involved. Thus, when the antibody has the neutralizing activity, the antibody can inactivate signal transduction in which the 14273 is involved, for example, the cell stimulating activities mediated by the 14273.

Therefore, the antibody (e.g., the neutralizing antibody) to the 14273 has, for example, an action of regulating glycerol production from adipocytes, an action of regulating blood glycerol, an action of regulating lipolysis, an action of regulating insulin resistance, an action of regulating stress, an action of regulating adrenocorticotropic hormone (ACTH) secretion (preferably, an action of promoting glycerol production from adipocytes, an action of increasing blood glycerol, an action of promoting lipolysis, an action of promoting insulin resistance, an action of regulating stress and an action of promoting adrenocorticotropic hormone (ACTH) secretion) etc., and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for promoting glycerol production from adipocytes, an agent for increasing blood glycerol, an agent for promoting lipolysis, an agent for promoting insulin resistance, an agent for regulating stress and an agent for promoting adrenocorticotropic hormone (ACTH) secretion), etc.

Also, the antibody (e.g., the neutralizing antibody) to the 14273 can be used as an agent for preventing/treating, e.g., diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, etc. (especially, anorexia and obesity, among others, obesity with visceral fat accumulation), or as an agent for regulating stress.

Furthermore, the antibody (e.g., the neutralizing antibody) to the 14273 can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, the antibody (e.g., the neutralizing antibody) to the 14273 acts as an agent for promoting adrenocorticotropic hormone (ACTH) secretion and is thus useful as a drug such as an agent for preventing/treating, e.g., connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy), anaphylactic shock, etc.

(10) Pharmaceutical comprising the antisense DNA or siRNA of the present invention

The antisense DNA or siRNA described herein has, for example, an action of regulating glycerol production from adipocytes, an action of regulating blood glycerol, an action of regulating lipolysis, an action of regulating insulin resistance, an action of regulating stress, an action of regulating adrenocorticotropic hormone (ACTH) secretion (preferably, an action of promoting glycerol production from adipocytes, an action of increasing blood glycerol, an action of promoting lipolysis, an action of promoting insulin resistance, an action of regulating stress and an action of promoting adrenocorticotropic hormone (ACTH) secretion) etc., and is thus useful as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion (preferably, an agent for promoting glycerol production from adipocytes, an agent for increasing blood glycerol, an agent for promoting lipolysis, an agent for promoting insulin resistance, an agent for regulating stress and an agent for promoting adrenocorticotropic hormone (ACTH) secretion), etc.

Also, the antisense DNA or siRNA described herein can be used as an agent for preventing/treating, e.g., diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, etc. (especially, anorexia and obesity, among others, obesity with visceral fat accumulation), or as an agent for regulating stress.

Furthermore, the antisense DNA or siRNA described herein can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, the antisense DNA or siRNA described herein acts as an agent for promoting adrenocorticotropic hormone (ACTH) secretion and is thus useful as a drug such as an agent for preventing/treating, e.g., connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy), anaphylactic shock, etc.

For example, where the antisense DNA or siRNA is used, the antisense DNA itself is administered; alternatively, the antisense DNA is inserted into an appropriate vector such as retrovirus vector, adenovirus vector, adenovirus-associated virus vector, etc. and then administered in a conventional manner. The antisense DNA or siRNA may also be administered as its intact form, or with adjuvants to assist its uptake by gene gun or through a catheter such as a catheter with a hydrogel.

In addition, the antisense DNA or siRNA may also be used as an oligonucleotide probe for diagnosis to investigate the presence of the DNA described herein or the state of its expression in tissues or cells.

(11) Preparation of animal bearing the DNA of the present invention

Described is also a non-human mammal bearing DNA which is exogenous (hereinafter briefly referred to as the exogenous DNA described wherein or its variant DNA (sometimes briefly referred to as the exogenous variant DNA described herein).

That is, described is further:

[1] A non-human mammal bearing the exogenous DNA of the present invention or its variant DNA;
[2] The mammal according to [1], wherein the non-human mammal is a rodent;
[3] The mammal according to [2], wherein the rodent is mouse or rat; and,
[4] A recombinant vector containing the exogenous DNA described herein or its variant DNA and capable of expressing in a mammal; etc.

The non-human mammal bearing the exogenous DNA described herein or its variant DNA (hereinafter briefly referred to as the DNA transgenic animal described herein) can be produced by transferring a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfer the exogenous DNA of the present invention into a somatic cell, a living organ, a tissue cell, or the like by the DNA transfer, and utilize the transformant for cell culture, tissue culture, etc. In addition, these cells may be fused with the above-described germinal cell bv a publicly known cell fusion method to prepare the DNA transgenic animal described herein.
Examples of the non-human mammal that can be used include bovine, swine, sheep, goats, rabbits, dogs, cats, guinea pigs, hamsters, mice, rats, etc. Above all, preferred are rodents, especially mice (e.g., C57B1/6 strain, DBA2 strain, etc. for a pure line and for a cross line, B6C3F$_1$ strain, BDF$_1$ strain B6D2F$_1$ strain, BALB/c strain, ICR strain, etc.), rats (Wistar, SD, etc.) or the like, since they are relatively short in ontogeny and life cycle from a standpoint of producing model animals for human disease.
"Mammals" in a recombinant vector that can be expressed in the mammals include the aforesaid non-human mammals and human.
The exogenous DNA described herein refers to the DNA described herein that is once isolated and extracted from mammals, not the DNA described herein inherently possessed by the non-human mammals.
The mutant DNA described herein includes mutants resulting from variation (e.g., mutation, etc.) in the base sequence of the original DNA described herein specifically DNAs resulting from base addition, deletion, substitution with other bases, etc. and further including abnormal DNA.
The abnormal DNA is intended to mean DNA that expresses abnormal the 14273 and exemplified by the DNA that expresses the 14273 for suppressing the function of normal the 14273.
The exogenous DNA described herein may be any one of those derived from a mammal of the same species as, or a different species from, the mammal as the target animal. In transferring the DNA described herein, it is generally advantageous to use the DNA as a DNA construct in which the DNA is ligated downstream a promoter capable of expressing the DNA in the target animal. For example, in the case of transferring the human DNA described herein a DNA transgenic mammal that expresses the DNA of the present invention to a high lever, can be prepared by microiniecting a DNA construct (e.g., vector, etc.) ligated with the human DNA described herein into a fertilized egg of the target non-human mammal downstream various promoters which are capable of expressing the DNA derived from various mammals (e.g., rabbits, dogs, cats, guinea pigs, hamsters, rats, mice, etc.) bearing the DNA described herein highly homologous to the human DNA.
As expression vectors for the 14273, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.
Examples of these promoters for regulating the DNA expression include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, a and β myosin heavy chains, -myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide elongation factor 1α (EF-1α) promoters, human and chicken β actin promoters, etc., which are capable of high expression in the whole body are preferred.
Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for normal 14273 can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal 14273 obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA described herein is transferred at the fertilized egg cell stage in a manner such that the DNA is certainly present in all the terminal cells and somatic cells of the target mammal. The fact that the exogenous DNA described herein is present in the germinal cells of the animal prepared by DNA transfer means that all offspring of the prepared animal will maintain the exogenous DNA described herein in all of the germinal cells and somatic cells thereof. The offspring of the animal that inherits the exogenous DNA described herein also have the exogenous DNA described herein in all of the germinal cells and somatic cells thereof.

The non-human mammal in which the normal exogenous DNA described herein has been transferred can be passaged as the DNA-bearing animal under ordinary rearing environment, by confirming that the exogenous DNA is stably retained by crossing.

By transfer of the exogenous DNA described herein at the fertilized egg cell stage, the DNA is retained to be excess in all of the germinal and somatic cells. The fact that the exogenous DNA described herein is excessively present in the germinal cells of the prepared animal after transfer means that the DNA described herein is excessively present in all of the germinal cells and somatic cells thereof. The offspring of the animal that inherits the exogenous DNA described herein have excessively the DNA of the present invention in all of the germinal cells and somatic cells thereof.

It is possible to obtain homozygous animals having the transferred DNA in both homologous chromosomes and breed male and female of the animal so that all the progeny have this DNA in excess.

In a non-human mammal bearing the normal DNA described herein , the normal DNA described herein has expressed at a high level, and may eventually develop hyperfunction in the function of the 14273 by promoting the function of endogenous normal DNA. Therefore, the animal can be utilized as a pathologic model animal for such a disease. For example, using the normal DNA transgenic animal described herein , it is possible to elucidate the mechanism of hyperfunction of the 14273 and the pathological mechanism of diseases associated with the 14273 and to investigate how to treat these diseases.

Furthermore, since a mammal transferred with the exogenous normal DNA described herein exhibits an increasing symptom of the 14273 liberated, the animal is usable for screening of a drug for the treatment of diseases associated with the 14273.

On the other hand, a non-human mammal having the exogenous abnormal DNA described herein can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfer of the abnormal DNA described herein at the fertilized egg cell stage is preserved to be present in all of the germinal and somatic cells of the target mammal. The fact that the abnormal DNA described herein is present in the germinal cells of the animal after DNA transfer means that all of the offspring of the prepared animal have the abnormal DNA described herein in all of the germinal and somatic cells. Such an offspring that passaged the exogenous DNA described herein will have the abnormal DNA described herein in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA described herein, the abnormal DNA described herein has expressed to a high level, and may eventually develop the function inactive type inadaptability to the 14273 by inhibiting the functions of endogenous normal DNA. Therefore, the animal can be utilized as a pathologic model animal for such a disease. For example, using the abnormal DNA transgenic animal described herein, it is possible to elucidate the mechanism of the function inactive type inadaptability to the 14273 and the pathological mechanism of diseases and to investigate how to treat the disease.

More specifically, the transgenic animal described herein expressing the abnormal DNA described herein at a high level is expected to serve as an experimental model to elucidate the mechanism of the functional inhibition (dominant negative effect) of the normal 14273 by the abnormal 14273 in the function inactive type inadaptability to the 14273.

A mammal bearing the abnormal exogenous DNA described herein is also expected to serve for screening a candidate drug for the treatment of the function inactive type inadaptability to the 14273, since the 14273 liberated is increased in such an animal.

Other potential applications of two kinds of the DNA transgenic animas described herein described above further include, for example:

(i) use as a cell source for tissue culture;
(ii) elucidation of the relation to the 14273 that is specifically expressed or activated by the 14273, by direct analysis of DNA or RNA in tissues of the DNA transgenic animal of the present invention or by analysis of the 14273 expressed by the DNA;
(iii) research on the function of cells derived from tissues that are usually cultured only with difficulty, using cells in tissues bearing the DNA cultured by a standard tissue culture technique;
(iv) screening a drug that enhances the functions of cells using the cells described in (iii) above; and,
(v) Isolation and purification of the variant 14273 of the present invention and preparation of its antibody, etc..

Furthermore, clinical conditions of a disease associated wit the 14273, including the function inactive type inadaptability to the 14273 can be determined by using the DNA transgenic animal described herein Also, pathological findings on each organ in a disease model associated with the 14273 can be obtained in more detail, leading to the development of a new method for treatment as well as the research and therapy of any secondary disease associated with the disease.

It is also possible to obtain a free DNA-transfened cell by withdrawing each organ from the DNA transgenic animal described herein , mincing the organ and degrading with a proteinase such as trypsin, etc., followed by establishing the line nf culturing or cultured cells. Furthermore, the DNA transgenic animal described herein can serve to identify cells capable of producing the 14273, and to study in association with apoptosis, differentiation or propagation or on the mechanism of signal transduction in these properties to inspect any abnormality therein. Thus, the DNA transgenic animal can provide an effective research material for the 14273 and for investigation of the function and effect thereof.

To develop a drug for the treatment of diseases associated with the 14273, including the function inactive type inadaptability to the 14273, using the DNA transgenic animal described herein, an effective and rapid method for screening can be provided by using the method for inspection and the method for quantification, etc. described above. It is also possible to investigate and develop a method for DNA therapy for the treatment of diseases associated with the 14273, using the DNA transgenic animal described herein or a vector capable of expressing the exogenous DNA described herein

(12) Knockout animal

Further described is a non-human mammalian embryonic stem cell bearing the DNA described herein inactivated and a non-human mammal deficient in expressing the DNA described herein.

Thus, described is:

[1] a non-human mammalian embryonic stem cell in which the DNA of the present invention is inactivated;
[2] the embryonic stem cell according to [1], wherein the DNA is inactivated by introducing a reporter gene (e.g., β-galactosidase gene derived from *Escherichia coli);*
[3] the embryonic stem cell according to [1], which is resistant to neomycin;
[4] the embryonic stem cell according to [1], wherein the non-human mammal is a rodent;
[5] the embryonic stem cell according to [4], wherein the rodent is mouse
[6] a non-human mammal deficient in expressing the DNA described herein, wherein the DNA is inactivated;
[7] the non-human mammal according to [6], wherein the DNA is inactivated by inserting a reporter gene (e.g., β-galactosidase derived from *Escherichia coli*) therein and the reporter gene is capable of being expressed under control of a promoter for the DNA described herein
[8] the non-human mammal according to [6], which is a rodent;
[9] the non-human mammal according to [8], wherein the rodent is mouse; and,
[10] a method of screening a compound or its salt that promotes or inhibits the promoter activity to the DNA described herein which comprises administering a test compound to the mammal of [7] and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA described herein, or the DNA has no substantial ability to express the 14273 (hereinafter sometimes referred to as the knockout DNA described herein by substantially inactivating the activities of the 14273 encoded by the DNA (hereinafter merely referred to as ES cell).

As the non-human mammal, the same examples as described above apply. Techniques for artificially mutating the DNA described herein include deletion of a part or all of the DNA sequence and insertion of or substitution with other DNA, by genetic engineering. By these variations, the knockout DNA described herein may be prepared, for example, by shifting the reading frame of a codon or by disrupting the function of a promoter or exon.

Specifically, the non-human mammal embryonic stem cell in which the DNA described herein is inactivated (hereinafter merely referred to as the ES cell with the DNA described herein inactivated or the knockout ES cell described herein) can be obtained by, for example, isolating the DNA described herein that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon region thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter briefly referred to as a targeting vector). The thus-obtained ES cells to the southern hybridization analysis with a DNA sequence on or near the DNA described herein as a probe, or to PCR analysis with a DNA sequence on the targeting vector and another DNA sequence near the DNA of the present invention which is not included in the targeting vector as primers, to select the knockout ES cell described herein.

The parent ES cells to inactivate the DNA described herein by homologous recombination, etc. may be of a strain already established as described above, or may originally be established in accordance with a modification of the known method by Evans and Kaufman described above. For examples, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cells aggregates in suspension under appropriate conditions, it is possible to differentiate the ES cells to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA described herein which are obtained from the differentiated ES cells described herein are useful for studying the 14273 in vitro or the 14273 cytologically.

The non-human mammal deficient in expression of the DNA. described herein can be identified from a normal animal by measuring the mRNA level in the subject animal by a publicly known method, and indirectly comparing the degrees of expression.

As the non-human mammal, the same examples supra apply.

With respect to the non-human mammal deficient in expression of the DNA of the present invention, the DNA of the present invention can be made knockout by transferring a targeting vector, prepared as described above, to mouse embryonic stem cells or mouse oocytes, and conducting homologous recombination in which a targeting vector DNA sequence, wherein the DNA described herein is inactivated by the transfer, is replaced with the DNA described herein on a chromosome of a mouse embryonic stem cell or mouse oocyte.

The knockout cells with the disrupted DNA described herein can be identified by the southern hybridization analysis using as a probe a DNA fragment on or near the DNA described herein or by the PCR analysis using as primers a DNA sequence on the targeting vector and another DNA sequence at the proximal region of other than the DNA described herein derived from mouse used in the targeting vector. When non-human mammal stem cells are used, a cell line wherein the DNA described herein is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA described herein and those having an artificially mutated locus of the DNA described herein .

When some germ cells of the chimeric animal have a mutated locus of the DNA described herein A an individual, which entire tissue is composed of cells having a mutated locus of the DNA described herein can be selected from a series of offspring obtained by crossing between such a chimeric animal and a normal animal, e.g., by coat color identification, etc. The individuals thus obtained are normally deficient in heterozygous expression of the 14273. The individuals deficient in homozygous expression of the 14273 can be obtained from offspring of the intercross between those deficient in heterozygous expression of the 14273.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA described herein can be obtained by selection based on homologous recombination.

As described above, the individuals in which the DNA described herein is rendered knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammalian embryonic stem cell, in which the DNA described herein is inactivated, is very useful for preparing a non-human mammal deficient in expression of the DNA described herein

Since the non-human mammal, in which the DNA described herein is inactivated, lacks various biological activities derived from the 14273, such an animal can be a disease model suspected of inactivated biological activities of the 14273 and thus, offers an effective study to investigate the causes for and therapy for these disease.

(12a) Method of screening a compound having therapeutic/preventive effects on diseases caused by deficiency, damages, etc. of the DNA described herein.

The non-human mammal deficient in expression of the DNA described herein can be employed for screening of a compound having therapeutic/preventive effects on diseases caused by deficiency, damages, etc. of the DNA described herein

That is, the present invention provides a method of screening a compound having a therapeutic/preventive effect on diseases caused by deficiency, damages, etc. of the DNA described herein, which comprises administering a test compound to a non-human mammal deficient in expression of the DNA described herein and, observing and measuring a change occurred in the animal.

As the non-human mammal deficient in expression of the DNA described herein which can be employed for the screening method, the same examples as given hereinabove apply.

Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.

The test compound may form salts and may be used in the form of salts with physiologically acceptable acids (e.g., inorganic acids, etc.) or bases (e.g., organic acids, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

Specifically, the non-human mammal deficient in expression of the DNA described herein is treated with a test compound, comparison is made with an intact animal for control and a change in each organ, tissue, disease conditions, etc. of the animal is used as an indicator to assess the therapeutic/preventive effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately selected depending on the administration methods, nature of the test compound, etc.

When a test compound is administered to a test animal in the screening method and blood sugar level or blood glycerol level of the test animal or the disease condition described above diminishes by at least about 10%, preferably at least 30%, more preferably at least about 50%, the test compound can be selected to be a compound or its salt having a therapeutic/preventive effect on the diseases described above.

The compound or its salt, which is obtained using the above screening method, is a compound or its salt selected from the test compounds described above and can be used as a drug for treating/preventing diseases caused by deficiencies, damages, etc. of the 14273 (e.g., diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of antidiuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases, etc., especially, diabetes mellitus, hyperlipemia, overweight, arteriosclerosis, angina pectoris or myocardial infarction; an agent for regulating stress, an agent for regulating glycerol production from adipocytes, an agent for regulating blood glycerol, an agent for regulating lipolysis, an agent for regulating insulin resistance, an agent for regulating adrenocorticotropic hormone (ACTH) secretion (preferably, an agent for regulating stress, an agent for suppressing glycerol production from adipocytes, an agent for lowering blood glycerol, an agent for suppressing lipolysis, an agent for suppressing insulin resistance and an agent for suppressing adrenocorticotropic hormone (ACTH) secretion), etc.

Also, the compound or its salt, which is obtained using the above screening method, can be used as an agent for preventing/treating diseases, e.g., arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis, and their secondary diseases [e.g., coronary heart disease (CHD), cerebral ischemia, etc.], lipid dysbolism and its secondary diseases, etc.

Furthermore, the compound or its salt, which is obtained using the above screening method, acts as an agent for suppressing adrenocorticotropic hormone (ACTH) secretion and can be used as an agent for preventing/treating disease, e.g., ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis), adrenocortical atrophy, etc.

Compounds or their salts derived from the compounds or their salts obtained by the screening described above can be used as well.

The compound obtained by the screening method may form salts and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The pharmaceutical comprising the compound or its salt obtained by the above screening method can be manufactured in a manner similar to the above-described method for preparing the pharmaceutical comprising the compound or its salt that changes the binding property of the 14273 to the ligand.

Since the pharmaceutical preparation thus obtained is safe and low toxic, it can be administered to human or non-human mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound or its salt is orally administered, the compound is generally administered to the patient (as 60 kg body weight) with diabetes mellitus in a daily dose of about 0.1 to about 100 mg, preferably about 1.0 to about 50 mg and, more preferably about 1.0 to about 20 mg. In parenteral administration, a single dose of said compound or its salt may vary depending upon subject to be administered, target organs, conditions, method of administration, etc. When it is administered to the patient (as 60 kg body weight) with diabetes mellitus in the form of an injectable preparation, it is advantageous to administer intravenously to the patient in a daily dose of about 0.01 to about 30 mg, preferably about 0.1 to about 20 mg, and more preferably about 0.1 to about 10 mg. For other animal species, the corresponding dose as converted per 60 kg body weight can be administered.

(12b) Method of screening a compound or its salt that promotes or inhibits the activity of a promoter to the DNA described herein.

Further described is a method of screening a compound or its salt that promotes or inhibits the activity of a promoter to the DNA described herein, which comprises administering a test compound to a non-human mammal deficient in expression of the DNA described herein and detecting the expression of the reporter gene.

In the screening method described above, an animal in which the DNA described herein is inactivated by introducing a reporter gene and the reporter gene is expressed under control of a promoter to the DNA described herein is used as the non-human mammal deficient in expression of the DNA described herein , which is selected from the aforesaid non-human mammals deficient in expression of the DNA described herein .

The same examples of the test compound apply to specific compounds used for the screening.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA described herein in the non-human mammal deficient in expression of the DNA described herein wherein the DNA described herein is substituted with the reporter gene, the activity of the promoter can be detected by tracing the expression of a substance encoded by the reporter gene.

When a part of the DNA region encoding the 14273 is substituted with, e.g., β-galactosidase gene (lacZ) derived from *Escherichia coli,* (β-galactosidase is expressed in a tissue where the 14273 should originally be expressed, instead of the 14273. Thus, the expression state of the 14273 can be readily observed in vivo of an animal by staining with a reagent, e.g., 5-bromo-4-chloro-3-indolyl-β-galactopyzanoside (X-gal) which is substrate for β-galactosidase. Specifically, a mouse deficient in the 14273, or its tissue section is fixed with glutaraldehyde, etc. After washing with phosphate buffered saline (PBS), the system is reacted with a staining solution containing X-gal at room temperature or about 37˚C for approximately 30 minutes to an hour. After the β-galaciosidase reaction is terminated by washing the tissue preparation with 1 mM EDTA/PBS solution, the color formed is observed. Alternatively, mRNA encoding lacZ may be detected in a conventional manner.

The compounds or their salts obtained using the screening method described above are compounds or their salts that are selected from the test compounds described above and that promote or inhibit the promoter activity to the DNA described herein

The compound obtained by the screening method may form salts and may be used in the form of salts with physiologically acceptable acids (e.g., inorganic acids, etc.) or bases (e.g., organic acids, etc), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that promotes the promoter activity to the DNA described herein is useful as a central or peripheral nerve function-regulating agent.

The compound or its salt that promotes or inhibits the promoter activity to the DNA described herein can regulate the expression of the 14273 and can regulate the function of the 14273, and is thus useful, e.g., as an agent for regulating glycerol production from adipocytes, an agent for regulating blood glycerol, an agent for regulating lipolysis, an agent for regulating insulin resistance, an agent for regulating stress, an agent for regulating adrenocorticotropic hormone (ACTH) secretion, etc.

The compound or its salt that promotes the promoter activity to the DNA described herein can promote the expression of the 14273 and can promote the function of the 14273, and is thus useful, e.g., as an agent for suppressing glycerol production from adipocytes, an agent for lowering blood glycerol, an agent for suppressing lipolysis, an agent for suppressing insulin resistance, an agent for regulating stress, an agent for suppressing adrenocorticotropic hormone (ACTH) secretion), etc.

Also, the compound or its salt that promotes the promoter activity to the DNA described herein is useful as a drug such as an agent for preventing/treating diseases associated with dysfunction of the 14273, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.) or circulatory diseases, stress (especially, diabetes mellitus, hyperlipemia, overweight, arteriosclerosis, angina pectoris or myocardial infarction), etc.

Furthermore, the compound or its salt that promotes the promoter activity to the DNA described herein can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, the compound or its salt that promotes the promoter activity to the DNA described herein acts as an agent for suppressing adrenocorticotropic hormone (ACTH) secretion and can be used as an agent for preventing/treating disease, e.g., ACTH-producing tumor, Cushing's disease, infectious disease, secondary adrenocortical insufficiency, peptic ulcer, diabetes mellitus, mental disorder, cataract, glaucoma, tuberculous disease, hypertension, Cushing's syndrome (e.g., central obesity, edema, hypertension, menstrual disorder, extensive stretch mark, hirsutism, diabetes mellitus, full moon face, osteoporosis, hemorrhagic diathesis, mental disorder, muscular atrophy, loss of muscle strength, hypokalemia, hypercholesterolemia, impaired glucose resistance, leukocytosis), adrenocortical atrophy, etc.

The compound or its salt that inhibits the promoter activity to the DNA described herein can inhibit the expression of the 14273 and can inhibit the function of the 14273, and is thus useful as an agent for promoting glycerol production from adipocytes, an agent for increasing blood glycerol, an agent for promoting lipolysis, an agent for promoting insulin resistance, an agent for regulating stress, an agent for promoting adrenocorticotropic hormone (ACTH) secretion, etc.

Also, the compound or its salt that inhibits the promoter activity to the DNA described herein is useful as a drug such as an agent for preventing/treating diseases associated with overexpression of the 14273, for example, diabetes mellitus, impaired glucose tolerance, ketosis, acidosis, diabetic neuropathy, diabetic nephropathy, diabetic retinopathy, hyperlipemia, arteriosclerosis, angina pectoris, myocardial infarction, sexual dysfunction, overweight, obesity, pituitary dysfunctions (e.g., hypopituitarism, pituitary dwarfism, diabetes insipidus, acromegaly, Cushing's disease, hyperprolactinemia, syndrome of inappropriate secretion of anti-diuretic hormone), cancer (e.g., colorectal cancer), deficits in memory and learning, pancreatic exhaustion, hypoglycemia, insulin allergy, lipotoxicity, fatty atrophy, cancerous cachexia, hyperinsulinemia, hyperglycemia, disorder caused by high FFA flux, hypertriglyceridemia, fatty liver, dysfunction of heat production, cholelithiasis, eating disorder, anorexia, secretion disorders of intestinal hormones (e.g., cholecystokinin (CCK), gastric inhibitory peptide (GIP), gastrin, glucagon-like peptide-1 (GLP-1), somatostatin, gastrin-releasing peptide, secretin, vasoactive intestinal peptide, motilin, substance P, neurotensin, galanin, neuropeptide Y, enkephalins, peptide YY, etc.), circulatory diseases, stress (especially, anorexia and obesity, among others, obesity with visceral fat accumulation), etc.

Furthermore, the compound or its salt that inhibits the promoter activity to the DNA described herein can be used as an agent for preventing/treating diseases, for example, arteriosclerosis, arteriosclerotic diseases and their secondary diseases [e.g., acute coronary syndrome such as atherosclerosis, peripheral arterial disease, acute myocardial infarction, unstable angina, etc., ischemic heart diseases such as restenosis after percutaneous transluminal coronary angioplasty (PTCA), myocardial infarction, angina pectoris, etc., arteriosclerosis including angiocalcinosis, etc., intermittent claudication, apoplexy (cerebral infarction, cerebral embolism, brain hemorrhage, etc.), lacunar infarction, cerebrovascular dementia, gangrene, glomerulosclerosis, nephropathy, Tangier disease, etc.], vascular lesions in atherosclerosis and their secondary diseases [e.g., coronary heart disease (CHD), cerebral ischemia, etc.], lipid dysbolism and its secondary diseases, etc.

In addition, the compound or its salt that inhibits the promoter activity to the DNA described herein acts as an agent for promoting adrenocorticotropic hormone (ACTH) secretion and is thus useful as a drug such as an agent for preventing/treating, e.g., connective tissue diseases (e.g., chronic articular rheumatism, systemic lupus erythematosus, polymyositis, rheumatic fever, scleroderma), kidney diseases (e.g., nephrosis), respiratory diseases (e.g., bronchial asthma, pulmonary tuberculous pleuritis, sarcoidosis, diffuse interstitial pneumonia), alimentary diseases (e.g., ulcerative colitis, cholestatic acute hepatitis, fulminant hepatitis, chronic hepatitis, cirrhosis), neuromuscular diseases (e.g., encephalomyelitis, peripheral neuritis, multiple sclerosis, myasthenia gravis, facial paralysis), blood diseases (e.g., hemolytic anemia, granulocytosis, purpura, aplastic anemia, leukemia, malignant lymphoma), endocrine-metabolic diseases (e.g., acute or chronic adrenocortical insufficiency, adrenogenital syndrome, malignant exophthalmos due to thyroid gland disease, ACTH isolated deficiency), skin diseases (e.g., urticaria, eczema, dermatitis, herpes zoster, psoriasis, drug allergy), anaphylactic shock, etc.

Compounds or their salts derived from the compounds or their salts obtained by the screening described above can be used as well.

The pharmaceutical comprising the compound or its salt obtained by the above screening method can be produced in a manner similar to the above-described method for preparing the pharmaceutical comprising the compound or its salt that changes the binding property of the 14273 or its salt to the ligand.

Since the pharmaceutical preparation thus obtained is safe and low toxic, it can be administered to, for example, human or non-human mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound or its salt that promotes the promoter activity to the DNA described herein is orally administered, it is generally administered to the patient (as 60 kg body weight) with diabetes mellitus in a daily dose of about 0.1 to about 100 mg, preferably about 1.0 to about 50 mg and, more preferably about 1.0 to about 20 mg. In parenteral administration, its single dose may vary depending upon target subject, target disease, conditions, method for administration, etc. When the compound or its salt is administered, e.g., in the form of an injectable preparation, it is advantageous to administer intravenously to the patient (as 60 kg body weight) with diabetes mellitus generally in a daily dose of about 0.01 to about 30 mg, preferably about 0.1 to about 20 mg, and more preferably about 0.1 to about 10 mg. For other animal species, the corresponding dose as converted per 60 kg body weight can be administered.

As stated above, the non-human mammal deficient in expression of the DNA described herein is extremely useful for screening the compound or its salt that promotes or inhibits the promoter activity to the DNA described herein and can greatly contribute to elucidation of causes for various diseases suspected of deficiency in expression of the DNA described herein and for the development of preventive/therapeutic drug for these disease.

Also, a so-called transgenic animal (gene transferred animal) can be prepared by using a DNA containing the promoter region of the 14273, ligating genes encoding various proteins at the downstream and injecting the same into oocyte of an animal. It is thus possible to synthesize the 14273 therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the 14273 itself.

In the specification and drawings, where bases, amino acids, etc. are indicated by abbreviations, these abbreviations are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| RNA | : ribonucleic acid |
| mRNA | : messenger ribonucleic acid |
| dATP | : deoxyadenosine triphosphate |
| dTTP | : deoxythymidine triphosphate |
| dGTP | : deoxyguanosine triphosphate |
| dCTP | : deoxycytidine triphosphate |

ATP : adenosine triphosphate
EDTA : ethylenediaminetetraacetic acid
SDS : sodium dodecyl sulfate
Gly : glycine
Ala : alanine
Val : valine
Leu : leucine
Ile : isoleucine
Ser : serine
Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gin : glutamine
pGlu : pyroglutamic acid
* : corresponding to termination codon
Me : methyl group
Et : ethyl group
Bu : butyl group
Ph : phenyl group
TC : thiazolidine-4(R)-carboxamido group

Substituents, protecting groups and reagents generally used in this specification are presented as the codes below.

Tos : p-toluenesulfonyl
CHO : formyl
Bzl : benzyl
$Cl_2$-Bzl : 2,6-dichlorobenzyl
Bom : benzyloxymethyl
Z : benzyloxycarbonyl
Cl-Z : 2-chlorobenzyloxycarbonyl
Br-Z : 2-bromobenzyl oxycarbonyl
Boc : t-butoxycarbonyl
DNP : dinitrophenol
Trt : trityl
Bum : t-butoxymethyl
Fmoc : N-9-fluorenyl methoxycarbonyl
HOBt : 1-hydroxybenztriazole
HOOBt : 3,4-dihydro-3-hydroxy-4-oxo-1.2,3-benzotriazine
HONB : 1-hydroxy-5-norbornene-2,3-dicarboxyimide
DCC : N,N'-dicyclohexylcarbodiimide

The sequence identification numbers in the sequence listing of the specification indicates the following sequence, respectively.

SEQ ID NO: 1
This shows the amino acid sequence of human-derived 14273.
SEQ ID NO: 2

This shows the base sequence of cDNA encoding human-derived 14273.
SEQ ID NO: 3
This shows the amino acid sequences mouse-derived 14273.
SEQ ID NO: 4
This shows the base sequence of cDNA encoding mouse-derived 14273.
SEQ ID NO: 5
This shows the base sequence of the primer used for PCR in EXAMPLE 2.
SEQ ID NO: 6
This shows the base sequence of the primer used for PCR in EXAMPLE 2.
SEQ ID NO: 7
This shows the base sequence of the primer used for PCR in EXAMPLE 2.
SEQ ID NO: 8
This shows the amino acid sequences rat-derived 14273.
SEQ ID NO: 9
This shows the base sequence of cDNA encoding rat-derived 14273. SEQ ID NO: 10
This shows the base sequence of the primer used for PCR in EXAMPLE 3.
SEQ ID NO: 11
This shows the base sequence of the primer used for PCR in EXAMPLE 3.
SEQ ID NO: 12
This shows the base sequence of the probe used for PCR in EXAMPLE 3.
SEQ ID NO: 13
This shows the base sequence of primer I used for PCR in EXAMPLE 4.
SEQ ID NO: 14
This shows the base sequence of primer 2 used for PCR in EXAMPLE 4.
SEQ ID NO: 15
This shows the base sequence of the primer used for PCR in EXAMPLE 7.
SEQ ID NO: 16
This shows the base sequence of the primer used for PCR in EXAMPLE 7.
SEQ ID NO: 17
This shows the base sequence of the probe used for PCR in EXAMPLE 7.
SEQ ID NO: 18
This shows the base sequence of the primer used for PCR in EXAMPLE 8.
SEQ ID NO: 19
This shows the base sequence of the primer used for PCR in EXAMPLE 8.
SEQ ID NO: 20
This shows the base sequence of the probe used for PCR in EXAMPLE 8.
SEQ ID NO: 21
This shows the base sequence of the sense strand of siRNA m14i561.
SEQ ID NO: 22
This shows the base sequence of the antisense strand of siRNA m 14i561.

The transformant, Escherichia coli JM109/pTArat14273 obtained in EXAMPLE 4 later described has been on deposit since April 18, 2003 under the Accession Number FERM BP-8361 at the National Institute of Advanced Industrial Science and Technology, International Patent Organism Depositary, located at Central 6, 1-1-1 Higashi, Tsukuba, Ibaraki, Japan (postal code 305-8566).

EXAMPLES

Hereinafter, the present invention will be described in more detail, by referring to REFERENCE EXAMPLE and EXAMPLES, but the scope of the invention is not deemed to be limited thereto. The genetic manipulation using Escherichia coli was performed according to the methods described in the Molecular Cloning.

REFERENCE EXAMPLE I

Construction of expression vectors for human and mouse 14273

The DNA fragments encoding human and mouse 14273 were obtained, respectively, by cloning from MTC panels (Clontech) using PCR in accordance with the sequences described in WO 2002/67868 and WO 2000/00611.

The resulting DNA fragments were introduced into pAKKO-111 vector at the SalI and SpeI sites to construct the respective expression plasmids. Subsequently, these expression plasmids were transfected to CHO (dhfr$^-$) by a per se known method. The expression plasmid-transfected cells were selected in a thymidine-free medium to acquire the cells stably expressing the respective receptors.

EXAMPLE 1

Confirmation of the reactivity of fatty acids with human and mouse 14273

CHO-K1 cells were incubated in Ham F-12 medium (Invitrogen) containing 1-% calf fetal serum, unless otherwise indicated. On the day before transfection, the cells were plated in 4.5 x $10^5$/10 $cm^2$ and incubated at 37˚C for 15 hours or longer in a $CO_2$ incubator adjusted to a 5% $CO_2$ concentration. Transfection was carried out by a modification of the procedure described in the protocol attached to the reagent, using Lipofectamine reagent (Invitrogen). Where a 6-well plate was used for the incubator, the procedure was performed as follows. First, 2 tubes each having a 1.5 ml volume were prepared and 100 μl each of Opti-MEM-I medium (Invitrogen) were dispensed in each tube. Next, after1 μg of the expression vector was charged in one tube and in another tube 6 μl of the Lipofectamine reagent was charged, they were mixed and settled at room temperature for 20 minutes. A mixture for transfection of The resulting solution was added with 800 μl of Opti-MEM-I medium and the resulting mixture for transfection was added to CHO-K1 cells previously washed with Opti-MEM-1 medium, followed by incubation in a $CO_2$ incubator for 6 hours. The cells after incubation were rinsed with PBS (Invitrogen), then scraped off using 0.05% trypsin-EDTA solution (Invitrogen) and recovered by centrifugation. The cells collected were counted and diluted to contain 5 x $10^4$ cells per 100 μl of the medium. The dilution was dispensed in a black walled 96-well plate (Costar) at a density of 100 μl/well, followed by incubation overnight in a $CO_2$ incubator. Various samples were added to the CHO-K1 cells, which transiently expressed the receptor by the transfection procedure described above, and a change in intracellular calcium level was measured using FLIPR (Molecular Device). To measure the change in intracellular calcium level on FILPR, the cells were pretreated as follows. First, an assay buffer was prepared to add fluorescent dye Fluo3-AM (DOJIN) to the cells, or to wash the cells immediately before the FLIPR assay. A solution was prepared by adding 20 ml of 1M HEPES (pH 7.4) (DOJIN) to 1000 ml of HBSS (Invitrogen) (hereinafter, HBSS/HEPES solution). After 710 mg of probenecid (Sigma) was dissolved in 5 ml of 1N NaOH, 5 ml of the HBSS/HEPES solution was further added to the solution and the two solutions were mixed with each other. Then, 10 ml of the solution mixture was added to the HBSS/HEPES solution and the resulting solution was used as the assay buffer. Next, K 50 μg of Fluo3-AM was added to 21 μl of DMSO (DOJIN) and an equal volume of 20% pluronic acid (Molecular Probes) was added thereto, followed by mixing them. The mixture was added to 10.6 ml of the assay buffer supplemented with 105 μl of calf fetal serum to prepare the fluorescent dye solution. Immediately after the medium of the transfection-treated CHO-K1 cells was removed, the fluorescent dye solution was dispensed in 100 μl each per well. Incubation was performed in a $CO_2$ incubator for an hour to incorporate the fluorescent dye into the cells. The cells after incubation were washed with the assay buffer described above and then set on FLIPR. Test samples to be added to the receptor-expressed CHO-Klcells were prepared using the assay buffer and set on FLIPR at the same time. Following the pretreatment above, a change in intracellular calcium level was determined with FLIPR, after various test samples were added. The results reveal that the CHO-K1 cells expressing human and mouse 14273 specifically responded (increased the intracellular calcium level), when Palmitoleic acid (FIG. 1), (linoleic acid) (FIG. 2), (γ-linolenic acid) (FIG. 3), arachidonic acid (FIG. 4), (docosahexaenoic acid, DHA) (FIG. 5), etc. With the expression vector alone-transfected CHO-K1 cells for control, such response was not observed. That is, it became clear that intrinsic ligands for human and mouse 14273 were fatty acids.

EXAMPLE 2

Expression distribution of human 14273 mRNA

To quantify the expression level of mRNA, ABI PRISM 7700 Sequence Detector (Applied Biosystems) was used. The primers [5'-GCTGTGGCATGCTTTTAAAC-3' (SEQ ID NO: 5), 5'-CGCTGTGGATGTCTATTTGC-3' (SEQ ID NO: 6)] and the probe [5'-AGTTCATTTCCAGTACCCTCCATCAGTGGC-3' (SEQ ID NO: 7)] used to quantify the expression level were designed based on the base sequence (SEQ ID NO: 2) of human 14273, using software Primer Express (Applied Biosystems) purpose-built for ABI PRISM Sequence Detector. The cDNA, which was synthesized from 1 μg of total RNA (Clontech) derived from various human tissues by reverse transcription using random primers, was used as a template. The reverse transcription was carried out according to the protocol attached, using SuperScriptII (GIBCO BRL) as a reverse transcriptase. The reaction solution for ABI PRISM 7700 Sequence Detector was prepared by mixing 12.5 μl of TaqMan Universal PCR Master Mix (Applied Biosystems), 0.9 μM each primer, 0.25 μM the probe and the cDNA solution together, to which distilled water was added to make the whole volume 25 μl. The reaction on

ABI PRISM 7700 Sequence Detector was carried out at 50°C for 2 minutes and 95°C for 10 minutes, followed by repeating 40 times the cycle set to include 95°C for 15 seconds and 60°C for 1 minute. The expression distribution of mRNA in various human tissues is shown in FIG. 6. It was observed that the mRNA was highly expressed in the hypophysis, adipose tissue and colon.

EXAMPLE 3

Expression distribution of rat 14273 mRNA

To quantify the expression level of mRNA, ABI PRISM 7700 Sequence Detector (Applied Biosystems) was used. The primers [5'-GTGGTGGCCTTCACGTTTG-3' (SEQ ID NO: 10), 5'-CGCTCCTGAACAGCGACAT-3' (SEQ ID NO: 11)] and the probe [5'-CAACTCCGCCCTAAACCCCATTCTGT-3' (SEQ ID NO: 12)] used to quantify the expression level were designed based on the base sequence (SEQ ID NO: 9) of rat 14273, using software Primer Express (Applied Biosystems) purpose-built for ABI PRISM Sequence Detector. The cDNA used as a template was prepared as follows. Various organs were removed from normal rats and rats with water immersion restraint stress and total RNA and poly $(A)^+$RNA were prepared using Isogen (Nippon Gene) and mRNA Purification Kit (Pharmacia), respectively, according to the respective manuals. After 1 μg of poly$(A)^+$RNA obtained was treated with DNaseI (Amplification Grade, GIBCO BRL), 160 ng was treated at 42°C in accordance with the manual using RNA PCR Kit (Takara) to synthesize cDNA. The reaction solution for ABI PRISM 7700 Sequence Detector was prepared by mixing 12.5 μl of TaqMan Universal PCR Master Mix (Applied Biosystems), 0.9 μM each primer, 0.25 μM the probe and the cDNA solution together, to which distilled water was added to make the whole volume 25 μl. The reaction on ABI PRIM 7700 Sequence Detector was carried out at 50°C for 2 minutes and 95°C for 10 minutes, followed by repeating 40 times the cycle set to include 95°C for 15 seconds and 60°C for 1 minute. The expression distribution of mRNA in various tissues is shown in FIG. 7. High expression was detected in the hypophysis, lung, adipose tissue and intestinal tract. Also, an increased expression level of 14273 mRNA was detected in the hypophysis of rats loaded with water immersion restraint stress, clearly indicating that 14273 is involved in regulation of stress (FIG. 8).

EXAMPLE 4

Cloning of cDNA encoding rat-derived 14273 and determination of its base sequence

Using as a template rat brain DNA, PCR was carried out using primer 1 (SEQ ID NO: 13) and primer 2 (SEQ ID NO: 14). Klentaq DNA Polymerase (Clontech) was used for PCR, followed by (i) reacting at 95°C for 2 minute, (2) repeating the reaction at 98°C for 10 seconds, 63°C for 20 seconds and 72°C for 1 minute 35 times, and then extension at 72°C for 7 minutes. After the reaction, the amplified product was cloned to a plasmid vector, pCR2.1 TOPO (Invitrogen) in accordance with the protocol of TOPO TA Cloning Kit (Invitrogen). The plasmid was transfected to Escherichia coli JM109 (Takara Shuzo) and the plasmid-bearing clones were selected in LB agar medium containing ampicillin. Analysis of the individual clones on base sequences gave the cDNA sequence (SEQ ID NO: 9) encoding a novel G protein-coupled receptor protein. A novel protein containing the amino acid sequence (SEQ ID NO: 8) deduced from this cDNA was named rat14273. Also, the transformant was named Escherichia coli JM109/pTArat 14273.

EXAMPLE 5

Effects of fatty acids on cAMP production in human 14273-expressed CHO cells

Human 14273-expressed CHO cells were incubated for 20 hours in a 96-well plate at a density of 1 x $10^5$/well. After the cells were washed twice with 100 μl of Assay Buffer (DMEM (Invitrogen) containing 0.1 mM IBMX (Wako Pure Chemical) and 0.1 mM Ro-20-1724 (Biomol)), solutions of butyric acid, γ-linolenic acid, linoleic acid, DHA and oleic acid (all by SIGMA) in Assay Buffer in the presence or absence of 2 μM forskolin (Wako Pure Chemical) were added to the cells, followed by reacting at 37°C for 10 minutes. After the reaction, the cells were lysed in accordance with the formulation of cAMP Screen (ABI) and the intracellular cAMP level was assayed. As a result, the respective fatty acids did not show any cAMP production activity on the human 14273-expressed CHO cells under the condition containing no forskolin. On the other hand, γ-linolenic acid, linoleic acid, DHA and oleic acid showed the cAMP production suppressing activity on the human 14273-expressed CHO cells in the presence of forskolin (2 μM), whereas butyric acid did not show any suppressing activity (FIG. 9). In the CHO cells for control where human 14273 was not expressed, such cAMP production suppressing activity was not observed.

EXAMPLE 6

MAP kinase activation in human 14273-expressed CHO cells by addition of the fatty acid

Human 14273-expressed CHO cells (CHO-h14273) prepared in by a per se publicly known method using the human 14273 expression vector prepared in REFERENCE EXAMPLE 1 or CHO-mock cells were plated on a 6-well plate at a density of 3 x $10^5$/well. The cells were incubated overnight in low serum medium (nucleic acid-free MEMα medium supplemented with 0.5% dialyzed fetal calf serum). The medium was replaced by a serum-free medium (nucleic acid-free MEMα medium) followed by incubation overnight. The medium was replaced by a fresh serum-free medium and after incubation for 4 hours, 30 μM various fatty acids were added thereto. After incubation for 10 minutes, the cells were lysed and extracted with a sample buffer (TEFCO) and separated on SDS-PAGE. Thereafter, western blotting was performed using PhosphoPlus p44/42 MAP kinase (Thr202/Tyr204) Antibody Kit (Cell Signaling Technology, Inc.). As shown in FIG. 10, the results revealed that activation of the protein shown by phosphorylation of MAP kinase after addition of the fatty acids occurred only in human 14273-expressed CHO cells.

EXAMPLE 7

Change in expression of the 14273 receptor accompanied by the induction of differentiation of 3T3-L1 cells into adipocytes

A change in expression of the 14273 receptor accompanied by the induction of differentiation of mouse fibroblast-like cell line 3T3-L1 cells into adipocytes was analyzed by the following procedures.

As a medium, there was used Dulbecco's Modified Eagle Medium (DMEM, Invitrogen) containing 4.5 g/l of glucose and 1.5 g/l of $Na_2HCO_3$ and supplemented with 10% calf fetal serum (ThermoTrace), 100 U/ml of penicillin and 100 μg/ml of streptomycin. Differentiation-inducing stimulation was given to the 3T3-L1 cells, which were incubated in a 75 $cm^2$ flask to become confluent, for 2 days using the aforesaid medium containing 2.5 μM dexamethasone, 0.5 mM 3-isobutyl-1-methylxanthine and 10 μg/ml of insulin, followed by incubation in the medium containing 10 μg/ml of insulin. The cells were washed twice with PBS(-) at each stage of the incubation and stored at -80˚C until RNA was extracted.

Total RNA was extracted using ISOGEN (Nippon Gene). From 1 μg of RNA cDNA was prepared using random primer and SuperScript II reverse transferase (GIBCO BRL) as a reverse transferase. The reaction was carried out at 42˚C according to the manual attached. After completion of the reaction, cDNA was precipitated in ethanol and dissolved in 100 μl. In RT-PCR, Sequence Detection System Prism 7700 (Applied Biosystems) was used; primers 5'-TCCGAGTGTCCCAACAAGACTAC-3' (SEQ ID NO: 13) and 5'-GACTCCACATGATGAAGAAGGAAA-3' (SEQ ID NO: 14) were used as the primers for amplification and detection and as TaqMan probe, 5'-(Fam)CCGCACGCTCTTCCTGCTCATG-(Tamra)-3' (SEQ ID NO: 15) was used. The reaction solution for RT-PCR was prepared by adding 0.05 μl each of 100 μM primer solutions, 0.5 μl of 5 μM TaqMan probe and 0.5 μl of the cDNA solution prepared above to 12.5 μl of TaqMan Universal PCR Master Mix (PE Biosystems) and further adding distilled water to make the whole volume of the reaction solution 25 μl. PCR was carried out, after reacting at 50˚C for 2 minutes and 95˚C for 10 minutes, by repeating 40 cycles of one cycle set at 95˚C for 15 seconds and 60˚C for 1 minute. The mRNA expression level of the 14273 receptor in the 3T3-L1 cells obtained was calculated as the copy number per 25 ng of total RNA (FIG. 11).

The results indicate that before the differentiation induction into adipose, only a slight expression level of the 14273 receptor was observed in the 3T3-L1 cells, whereas the expression level was drastically increased after the differentiation induction and reached 136027copies/25 ng total RNA on Day 14. This suggests that the 14273 receptor plays an important role in adipocytes.

EXAMPLE 8

Change in expression of the 14273 receptor accompanied by the induction of differentiation of rat primary culture preadipocytes into adipocytes

With respect to S.D. rat subcutaneous adipose-derived white preadipocytes and scapular brown adipose-derived brown preadipocytes (HOKUDO), a change in expression of the 14273 receptor accompanied by the induction of differentiation of the primary culture preadipocytes into adipocytes was analyzed by the following procedures, respectively.

As a medium, there was used Dulbecco's Modified Eagle Medium (D-MEM, Invitrogen) containing 4.5 g/l of glucose and 1.5 g/l of $Na_2HCO_3$ and supplemented with 10% calf fetal serum (Hyclone), 100 U/ml of penicillin and 100 μg/ml of streptomycin. The cells, which were incubated in a 25 $cm^2$ flask to become confluent, were incubated for 2 days in the above medium containing 2.5 μM dexamethasone, 0.5 mM 3-isobutyl-1-methylxanthine and 10 μ/ml of

insulin thereby to stimulate the induction of differentiation of the cells, followed by incubation for further 8 days in the aforesaid medium containing 10 μg/ml of insulin. Using ISOGEN (Nippon Gene), total RNA was extracted from the cells prior to differentiation induction and from the cells incubated for 8 days after the differentiation induction. From 1 μg of the total RNA cDNA was prepared using SuperScript™ II reverse transferase (Invitrogen) as a reverse transferase. The reaction was carried out at 42°C according to the manual attached. After completion of the reaction, cDNA was precipitated in ethanol and dissolved in 40 μl of Tris-EDTA buffer. For RT-PCR, Sequence Detection System Prism 7700 (Applied Biosystems) was used; primers 5'-GTGGTGGCCTTCACGTTTG-3' (SEQ ID NO: 16) and 5'-CGCTCCTGAACAGCGACAT-3' (SEQ ID NO: 17) were used as the primers for amplification and detection and as a TaqMan probe, 5'-(Fam)CAACTCCGCCCTAAACCCCATTCTGT-(Tamra)-3' (SEQ ID NO: 18) was used. The reaction solution for RT-PCR was prepared by adding 0.225 μl each of 100 μM primer solutions, 1.25 μl of 5 μM TaqMan probe and 1 μl of the cDNA solution prepared above to 12.5 μl of TaqMan Universal PCR Master Mix (Applied Biosystem) and further adding distilled water to make the whole volume of the reaction solution 25 μl. PCR was carried out, after reacting at 50°C for 2 minutes and 95°C for 10 minutes, by repeating 40 cycles of one cycle set at 95°C for 15 seconds and 60°C for 1 minute. The mRNA expression level of the 14273 receptor in the primary culture adipocytes obtained was calculated as the copy number per 25 ng of total RNA (FIG. 12).

The results indicate that before the differentiation induction into adipocytes, only a slight expression level of the 14273 receptor was observed both in the primary culture white preadipocytes and in the primary culture brown preadipocytes, whereas the expression level was drastically increased after the differentiation induction. This suggests that the 14273 receptor plays an important role in adipocytes.

EXAMPLE 9

Activity of suppressing adrenocorticotropic hormone (ACTH)-secretion from AtT-20 cells by fatty acids

Effects of free fatty acids on ACTH secretion from mouse pituitary corticotrophic cell line AtT-20 cells were analyzed by the following procedures.

First, adherent substrain was cloned from AtT/20 cell line (suspension type) by the contact selection tecynique using a poly-D-lysine-coated flask. The medium used was composed of Dulbecco's Modified Eagle Medium (DMEM, Invitrogen) containing 4.5 g/l of glucose and supplemented with 10% fetal calf serum (Thermo Trace), 100 U/ml of penicillin and 100 μ/ml of streptomycin. This adherent AtT/20 cell substrain was plated on a poly-D-lysine-coated 96-well plate at a density of 4 x 10°cells/well/100 μl for 2 nights, which was provided for assay. As a buffer for the assay, there was used Dulbecco's Modified Eagle Medium (DMEM, Invitrogen) containing 25 mM HEPES (pH 7.1) and I g/l of glucose. After washing twice with this buffer, 100 ml of buffer containing a given concentration of fatty acid was added, followed by preincubation at 37°C for an hour in a $CO_2$ incubator. Next, the medium as exchanged for a buffer containing a fatty acid in the co-presence of 10 nM corticotropin-releasing factor (CRF), followed by incubation at 37°C for 90 minutes in a $CO_2$ incubator. Ninety minutes after, the plate was gently agitated, then centrifuged at 1200 rpm for 5 minutes at room temperature and a sample (50 ml) was recovered from the intermediate layer. During the experiment process above, the fatty acid was allowed to be present always together with bovine serum albumin (BSA, Sigma) in a molar ratio of BSA : FFA = 4:1. BSA incorporated in the group added with the fatty acid was added in an equimolar amount also to the group added with buffer alone and to the group added with CRF alone. In the sample recovered, the ACTH level was determined using an ACTH assay kit (Mitsubishi Medical, ACTH IRMA "Yuka").

As shown in FIG. 13, suppression of ACTH secretion induced by CRF was observed more significantly with γ-linolenic acid (γ-LA) and α-linoleic acid (α-LA). On the other hand, butyric acid (BA) showing no agonist activity on the 14273 receptor did not show any significant suppression in CRF-induced ACTH secretion.

EXAMPLE 10

Lipolysis suppressing action of a fatty acid in 3T3-L1 adipose differentiation cells

Effects on lipolysis were examined using mouse fibroblast cell line 3T3-L1 cells capable of differentiating into adipocytes. The medium used was DMEM (Dulbecco's Modified Eagle Medium, Invitrogen) containing 4.5 g/l of glucose and supplemented with 10% fetal bovine serum (FBS, Invitrogen), 100 units/ml of penicillin and 100 μg/ml of streptomycin. The 3T3-L1 cells were plated on a plate and incubated to become confluent. The cells were then treated for 48 hours in the aforesaid medium containing 10 μg/ml of insulin, 0.5 mM 3-isobutyl-1-methylxanthine (IBMX), 2.5 μM dexamethasone to induce differentiation into adipose. Incubation was continued for further I days. After the 3T3-L1 cells were differentiated into adipose, a change in the amount of glycerol produced by lipolysis was assayed. After the cells were washed with modified Krebs-Ringer buffer, 1 x $10^{-9}$ M isoproterenol was added to the cell and simultaneously a fatty acid was added thereto, followed by treatment for 2 hours. The supernatant was then recovered. The glycerol content in the supernatant was determined using Free Glycerol Determination Kit (Sigma). As a result, it was noted that addition of γ-linolenic acid (γ-LA), which was a fatty acid shaving an agonist activity on 14273, resulted in lowering the glycerol production increased by the isoproterenol stimulation. On the other hand, such an activity was not observed with methyl linoleate (ML) having no agonist activity (FIG. 14).

EXAMPLE 11

Suppressed expression of mouse 14273-GFP-fused protein by introducing siRNA specific to the sequence of mouse 14273

Fused protein expression plasmid (pAKKO-14273-GFP) (0.2 μg/well) of mouse 14273 and GFP, which was produced by a per se known method, and m 14i561 (Dharmacon) (20 nM), which was mouse 14273-specific siRNA, were cotransfected to CHO cells (4 x $10^4$/well: 96-well plate) using Lipofectamine 2000 reagent (Invitrogen), followed by incubation for a day. The expression level of mouse 14273-GFP was detected by enzyme immunoassay shown below. After the culture supernatant was discarded and the cells were washed with HBSS (Invitrogen), the cells were fixed with 0.01% glutaraldehyde (Wako Pure Chemical) for 5 minutes, followed by blocking with 2% BSA-containing PBS (Takara Shuzo). Anti-GFP monoclonal antibody 3E6 (Nippon Gene) diluted to 500-fold was added to the cells. After incubation at room temperature for 2 hours, the cells were washed and HRP-labeled anti-mouse IgG antibody (ICN) diluted to 500-fold was added thereto, followed by incubation at room temperature for 2 hours. After washing, TMB microwell peroxidase substrate (Funakoshi) was added to the cells. After incubation for 30 minutes, sulfuric acid was added to discontinue the color-forming reaction and absorbance was measured at 450 nm. The results indicate that the expression level of pAKKO-14273-GFP was suppressed by 97% through transfection of m14i561. On the other hand, any remarkable suppression of the expression of pAKKO-14273-GFP was not observed with transfection of ScrambleII (Dharmacon) as negative control siRNA, confirming that ml 41561 was siRNA specifically suppressing mouse 14273.
Sequence of m14i561 (the upper is sense strand and the lower is antisense strand)
5' GGACCAGGAAAUUCCGAUUdTdT 3' (SEQ ID NO: 21)
3' dTdTCCUGGUCCUUUAAGGCUAA 5' (SEQ ID NO: 22)

INDUSTRIAL APPLICABILITY

It has become clear that the 14273 functions as the receptor of fatty acids or salts thereof. Therefore, the compound or its salt that changes the binding property of fatty acids or salts thereof to the 14273, its partial peptide, or a salt thereof can be efficiently screened and the compounds thus screened are useful as drugs for preventing/treating diseases including diabetes mellitus, hyperlipemia, obesity, pituitary dysfunction, etc, or as drugs for regulating stress.

## Claims

**1.** 1. A method of screening a compound or its salt that changes the binding property of a G protein-coupled receptor protein or a salt thereof to a fatty acid or a salt thereof,
wherein said receptor protein comprises:

the amino acid sequence represented by SEQ ID NO: 1, SEQ ID NO: 3 or SEQ ID NO: 8, and said method comprises using (1) the receptor protein or a salt thereof and (2) the fatty acid or a salt thereof.

**2.** A kit for screening a compound or its salt that changes the binding property of a G protein-coupled receptor protein or a salt thereof to a fatty acid or a salt thereof,
wherein said receptor protein comprises:

the amino acid sequence represented by SEQ ID NO: 1, SEQ ID NO: 3 or SEQ ID NO: 8, and said kit comprises (1) the receptor protein or a salt thereof and (2) the fatty acid or a salt thereof.

**3.** A method according to claim 1, wherein the compound is an agonist or an antagonist to the G protein-coupled receptor protein or a salt thereof.

**4.** A method of screening an agonist to a G protein-coupled receptor protein or a salt thereof,
wherein said receptor protein comprises:

the amino acid sequence represented by SEQ ID NO: 1, SEQ ID NO: 3 or SEQ ID NO: 8, and said method comprises assaying MAP kinase phosphorylation or activation, or the adrenocorticotropic hormone (ACTH) secretion suppressing activity in the case where a test compound is contacted with cells containing said G protein-coupled receptor protein or a salt thereof.

## Patentansprüche

**1.** Verfahren zum Screenen einer Verbindung, oder ihres Salzes, die die Bindungseigenschaft eines G-Protein-gekoppelten Rezeptorproteins, oder eines Salzes davon, zu einer Fettsäure, oder einem Salz davon, verändert,
wobei das Rezeptorprotein die Aminosäuresequenz, die dargestellt wird durch SEQ ID NO: 1, SEQ ID NO: 3 oder SEQ ID NO: 8, umfasst,
und wobei das Vefahren die Verwendung (1) des Rezeptorproteins, oder eines Salzes davon, und (2) der Fettsäure, oder eines Salzes davon, umfasst.

**2.** Kit zum Screenen einer Verbindung, oder ihres Salzes, die die Bindungseigenschaft eines G-Protein-gekoppelten Rezeptorproteins, oder eines Salzes davon, zu einer Fettsäure, oder einem Salzes davon, verändert,
wobei das Rezeptorprotein die Aminosäuresequenz, die dargestellt wird durch SEQ ID NO: 1, SEQ ID NO: 3 oder SEQ ID NO: 8, umfasst,
und der Kit (1) das Rezeptorprotein, oder ein Salz davon, und (2) die Fettsäure, oder ein Salz davon, umfasst.

**3.** Verfahren nach Anspruch 1, wobei die Verbindung ein Agonist oder ein Antagonist des G-Protein-gekoppelten Rezeptorproteins, oder eines Salzes davon, ist.

**4.** Verfahren zum Screenen eines Agonisten eines G-Protein-gekoppelten Rezeptorproteins, oder eines Salzes davon,
wobei das Rezeptorprotein die Aminosäuresequenz, die dargestellt wird durch SEQ ID NO: 1, SEQ ID NO: 3 oder SEQ ID NO: 8, umfasst,
und wobei das Verfahren Untersuchen von MAP Kinase Phosphorylierung oder Aktivierung, oder von Aktivität, die die adrenocorticotropes Hormon (ACTH) Sekretion unterdrückt, für den Fall, dass die Testverbindung mit Zellen in Kontakt gebracht wird, die dieses G-Protein-gekoppelte Rezeptorprotein, oder ein Salz davon, enthalten, umfasst.

## Revendications

**1.** Procédé de criblage d'un composé ou de son sel modifiant la propriété de liaison d'une protéine réceptrice couplée à une protéine G ou d'un sel de celle-ci à un acide gras ou à un sel de celui-ci,
dans lequel ladite protéine réceptrice comprend :

la séquence d'acide aminé représentée par SEQ ID NO : 1, SEQ ID NO : 3 ou SEQ ID NO : 8, et où ledit procédé comprend d'utilisation (1) de la protéine réceptrice ou d'un sel de celle-ci et (2) de l'acide gras ou d'un sel de celui-ci.

**2.** Kit de criblage d'un composé ou de son sel, qui modifie la propriété de liaison d'une protéine réceptrice couplée à une protéine G ou à un sel de celle-ci à un acide gras ou à un sel de celui-ci,
dans lequel ladite protéine réceptrice comprend :

la séquence d'acides aminés représentée par SEQ ID NO : 1, SEQ ID NO : 3 ou SEQ ID NO : 8, et où ledit kit comprend (1) la protéine réceptrice ou un sel de celle-ci et (2) l'acide gras ou un sel de celui-ci.

**3.** Procédé selon la revendication 1, dans lequel le composé est un agoniste ou un antagoniste de la protéine réceptrice couplée à une protéine G ou d'un sel de celle-ci.

**4.** Procédé de criblage d'un agoniste vers une protéine réceptrice couplée à une protéine G ou vers un sel de celle-ci, dans lequel ladite protéine réceptrice comprend :

la séquence d'acides aminés représentée par SEQ ID NO : 1, SEQ ID NO : 3 ou SEQ ID NO : 8, et où ledit procédé comprend l'essai de la phosphorylation ou de l'activation de kinase MAP, ou la sécrétion d'hormone adrénocorticotrope (ACTH) supprimant l'activité dans le cas où un composé d'essai vient en contact avec des cellules contenant ladite protéine réceptrice couplée à une protéine G ou avec un sel de celle-ci.

FIG 1

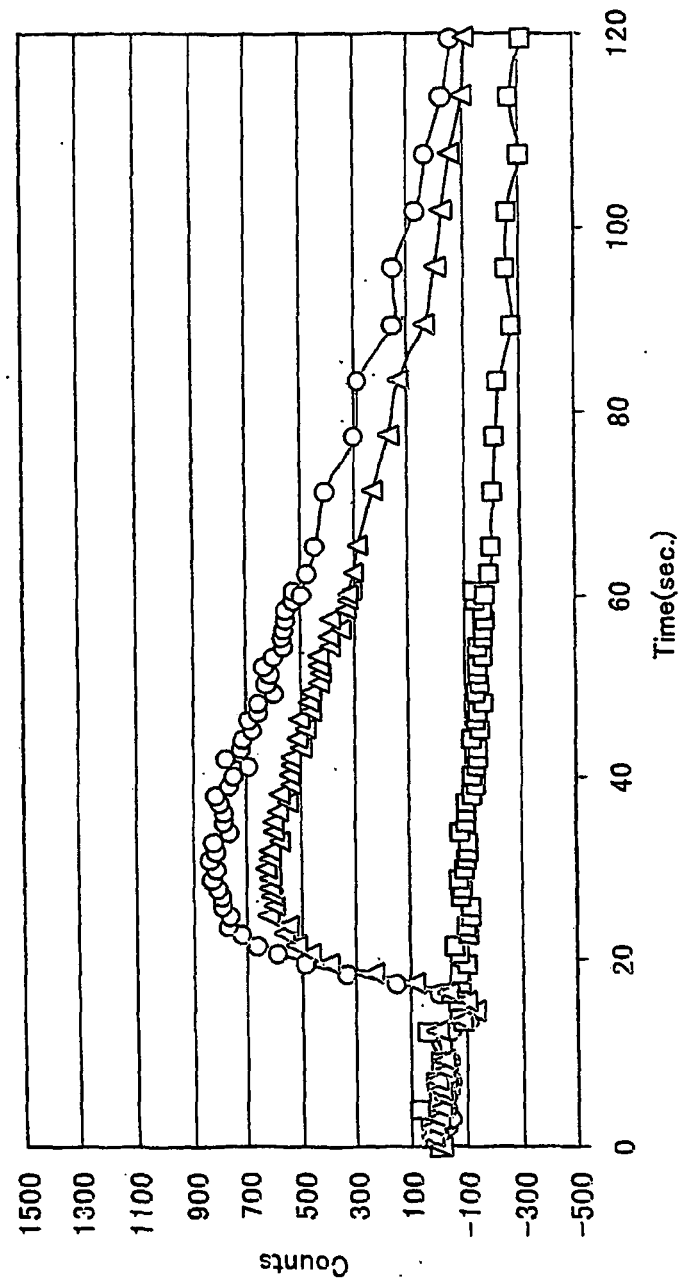
Counts
1500
1300
1100
900
700
500
300
100
-100
-300
-500
Time(sec.)
0
20
40
60
80
100
120

FIG 2

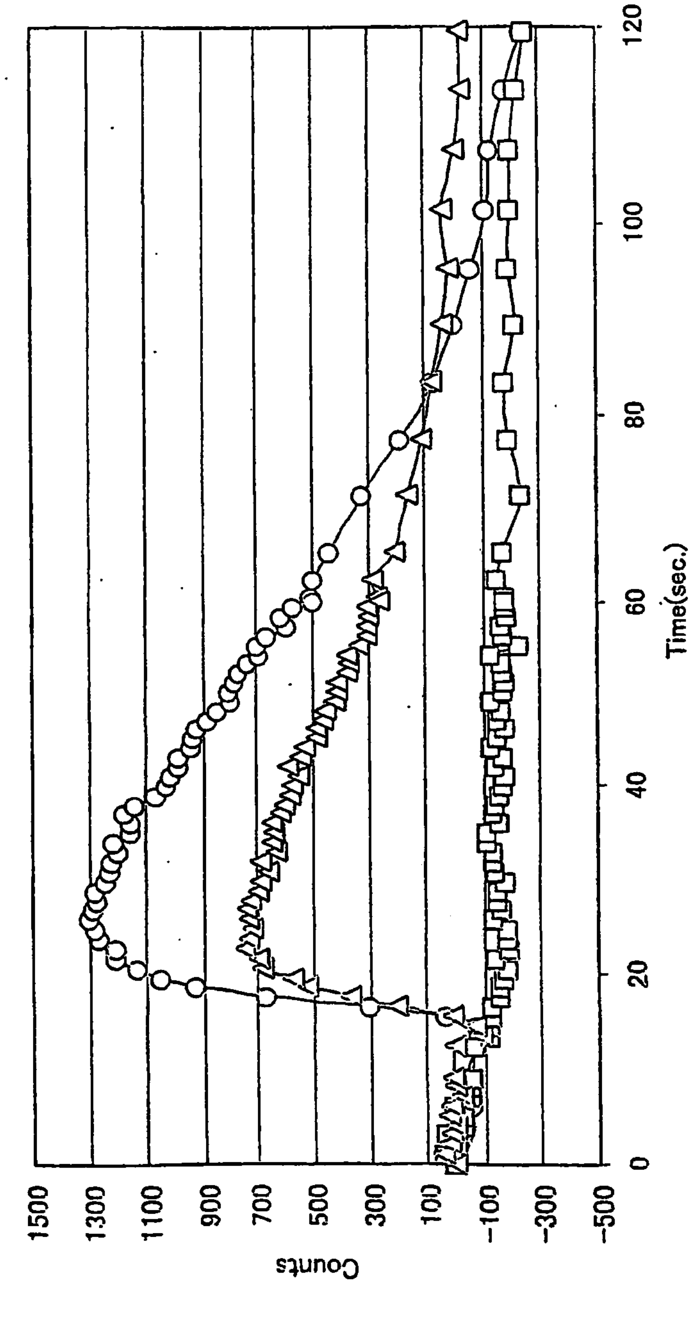
Counts
1500
1300
1100
900
700
500
300
100
-100
-300
-500
Time(sec.)
0
20
40
60
80
100
120

FIG 3

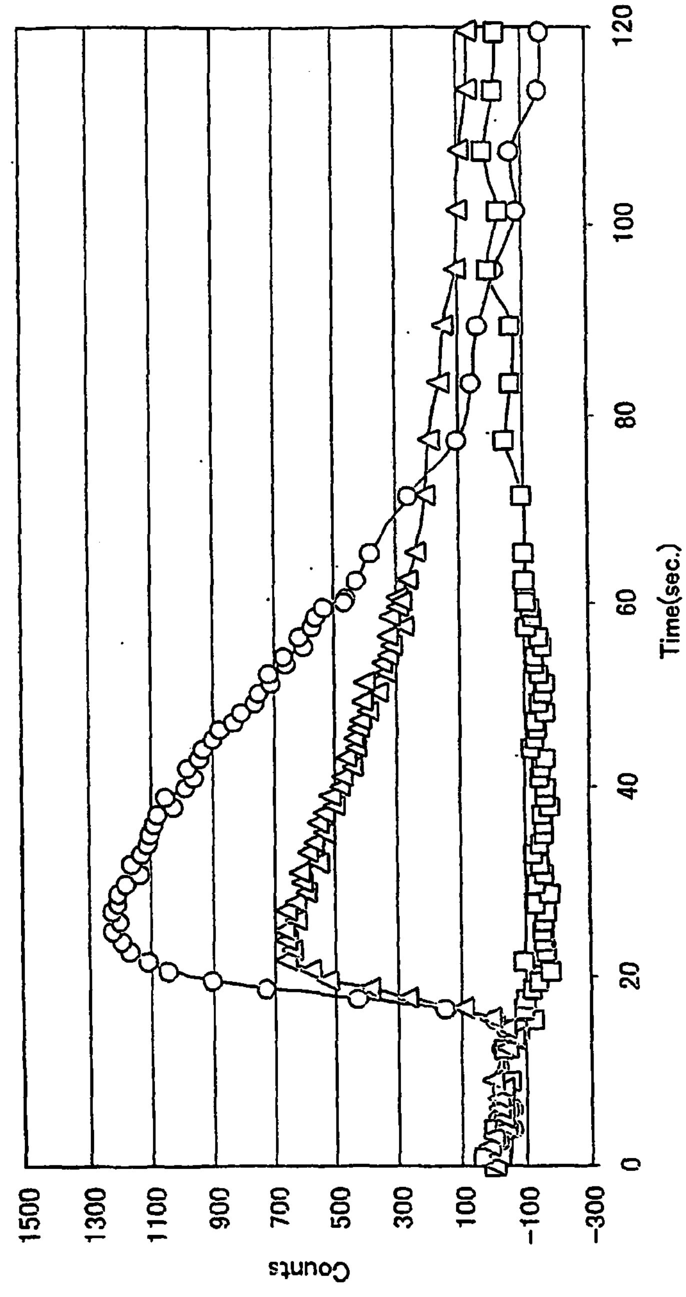
Counts
1500
1300
1100
900
700
500
300
100
-100
-300
Time(sec.)
0
20
40
60
80
100
120

FIG 4

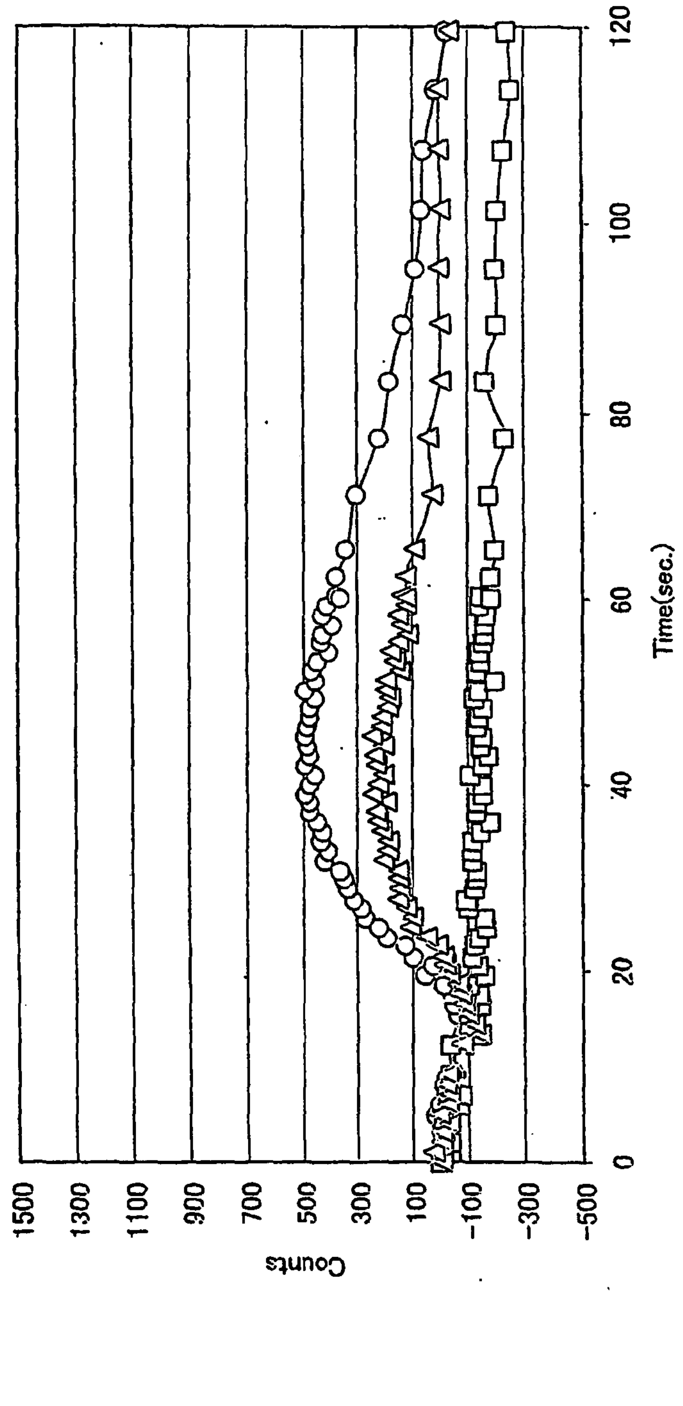
Counts
1500
1300
1100
900
700
500
300
100
-100
-300
-500
0
20
40
60
80
100
120
Time(sec.)

FIG 5

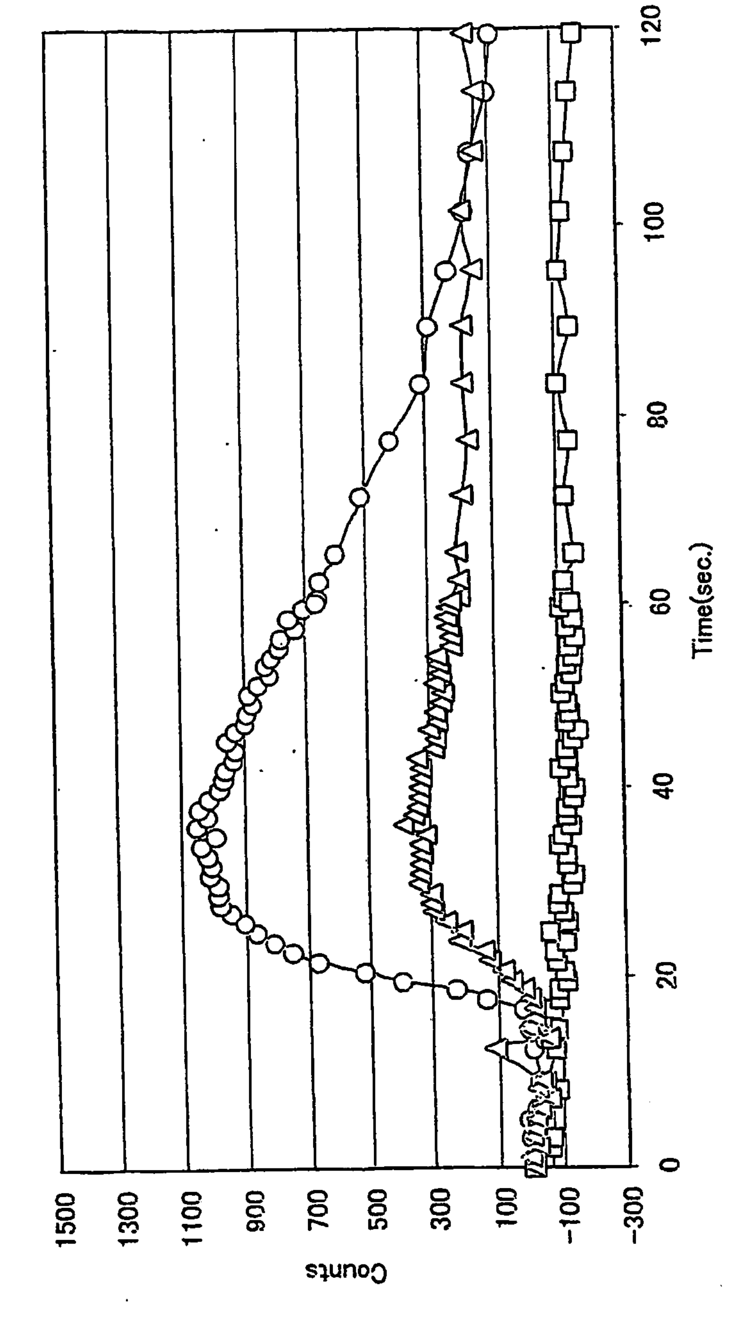
Counts
1500
1300
1100
900
700
500
300
100
-100
-300
Time(sec.)
0
20
40
60
80
100
120

FIG 6

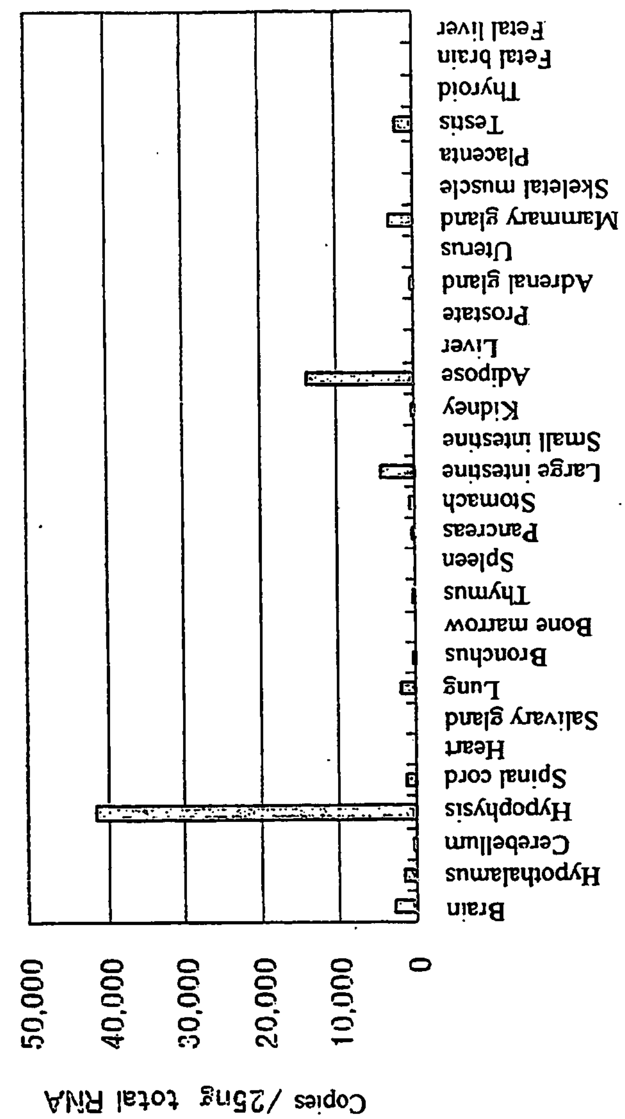
Brain
Hypothalamus
Cerebellum
Hypophysis
Spinal cord
Heart
Salivary gland
Lung
Bronchus
Bone marrow
Thymus
Spleen
Pancreas
Stomach
Large intestine
Small intestine
Kidney
Adipose
Liver
Prostate
Adrenal gland
Uterus
Mammary gland
Skeletal muscle
Placenta
Testis
Thyroid
Fetal brain
Fetal liver
0
10,000
20,000
30,000
40,000
50,000
Copies /25ng total RNA

FIG 7

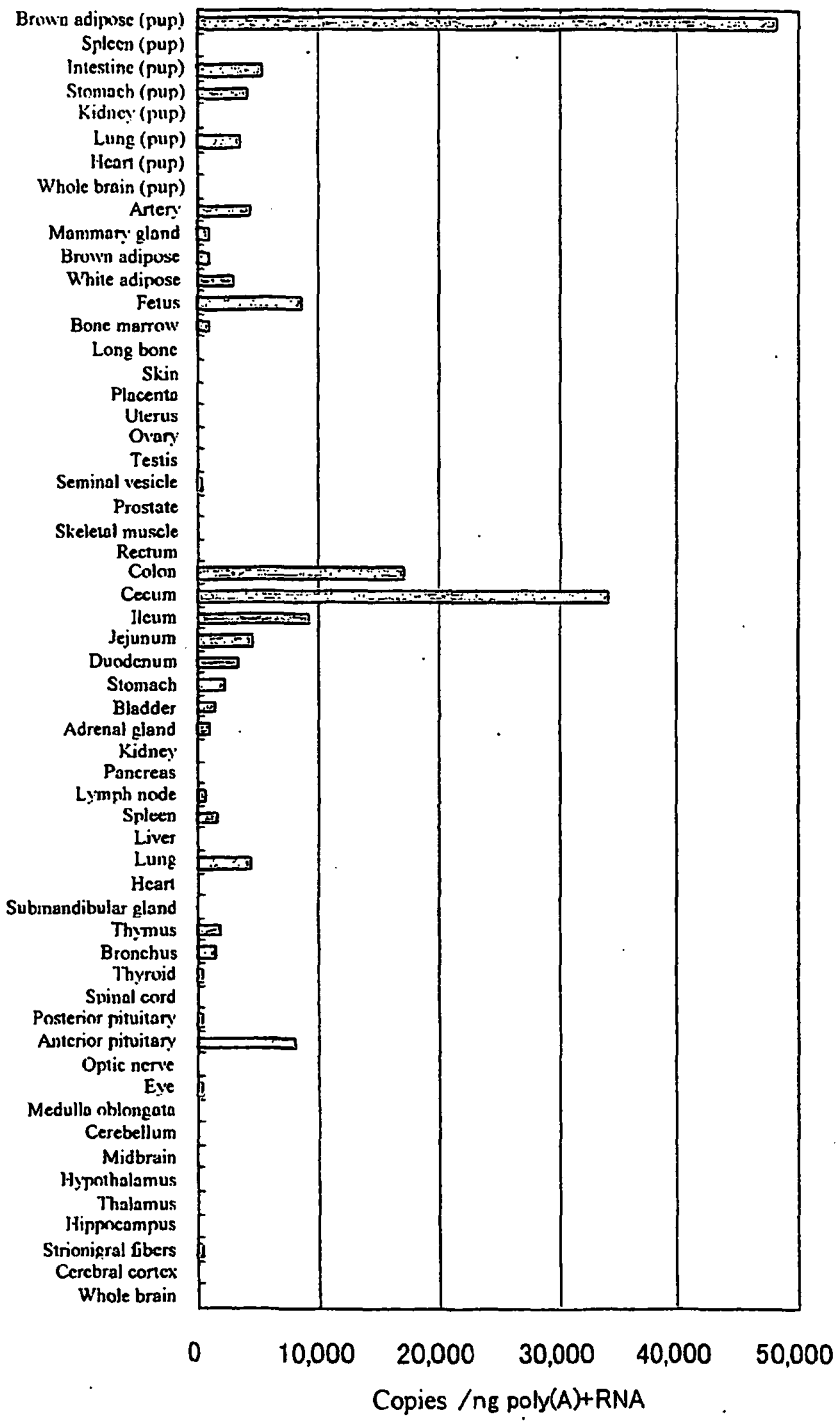
Brown adipose (pup)
Spleen (pup)
Intestine (pup)
Stomach (pup)
Kidney (pup)
Lung (pup)
Heart (pup)
Whole brain (pup)
Artery
Mammary gland
Brown adipose
White adipose
Fetus
Bone marrow
Long bone
Skin
Placenta
Uterus
Ovary
Testis
Seminal vesicle
Prostate
Skeletal muscle
Rectum
Colon
Cecum
Ileum
Jejunum
Duodenum
Stomach
Bladder
Adrenal gland
Kidney
Pancreas
Lymph node
Spleen
Liver
Lung
Heart
Submandibular gland
Thymus
Bronchus
Thyroid
Spinal cord
Posterior pituitary
Anterior pituitary
Optic nerve
Eye
Medulla oblongata
Cerebellum
Midbrain
Hypothalamus
Thalamus
Hippocampus
Strionigral fibers
Cerebral cortex
Whole brain
0
10,000
20,000
30,000
40,000
50,000
Copies /ng poly(A)+RNA

FIG 8

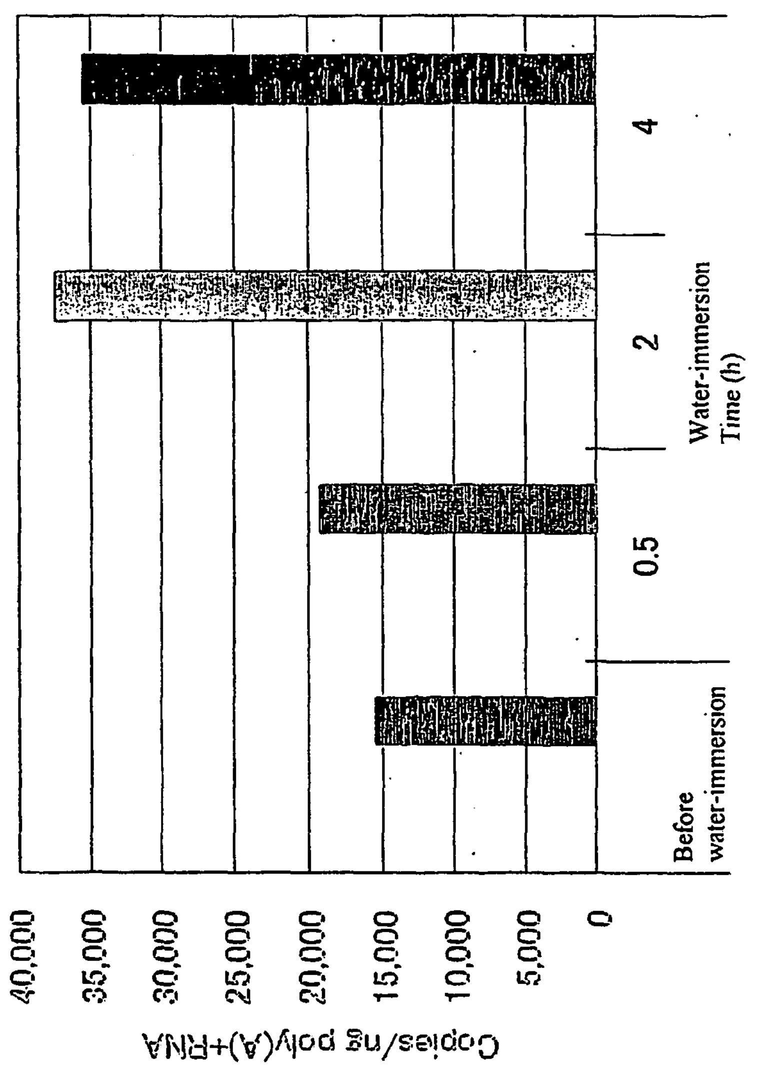
Copies/ng poly(A)+RNA
40,000
35,000
30,000
25,000
20,000
15,000
10,000
5,000
0
Before water-immersion
0.5
2
4
Water-immersion Time (h)

FIG 9

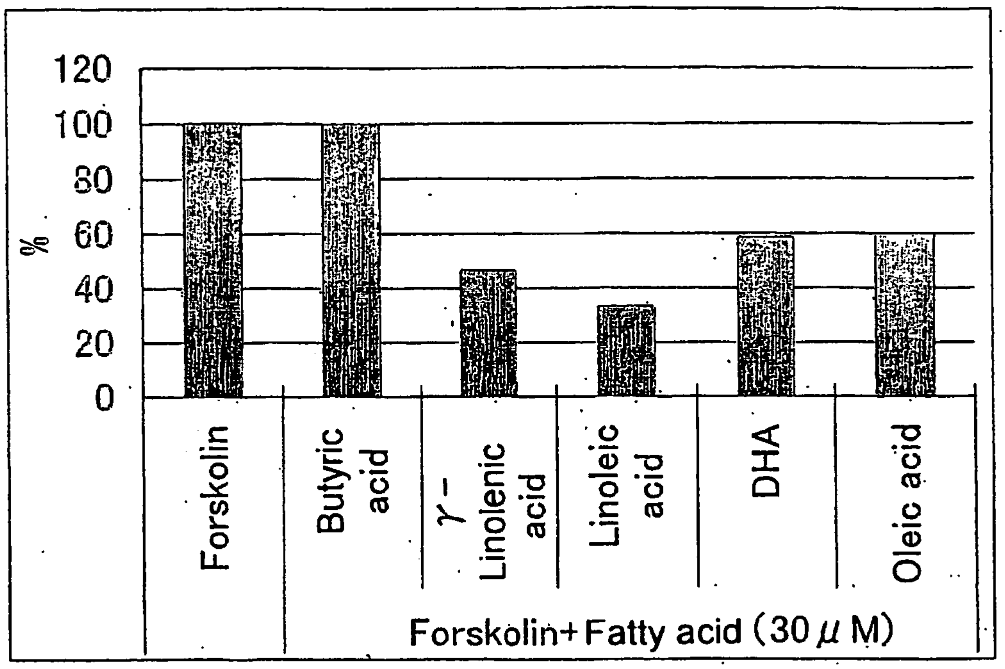
120
100
80
60
40
20
0
%
Forskolin
Butyric acid
γ-Linolenic acid
Linoleic acid
DHA
Oleic acid
Forskolin+Fatty acid (30 μM)

FIG 10

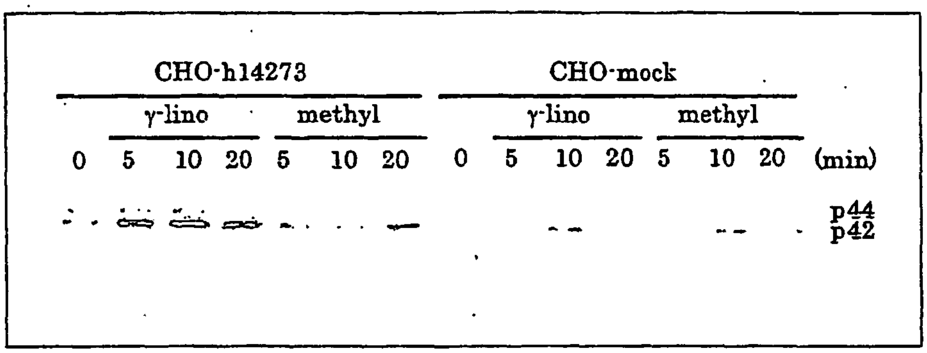
CHO-h14273
CHO-mock
γ-lino
methyl
γ-lino
methyl
0 5 10 20 5 10 20 0 5 10 20 5 10 20 (min)
p44
p42

FIG 11

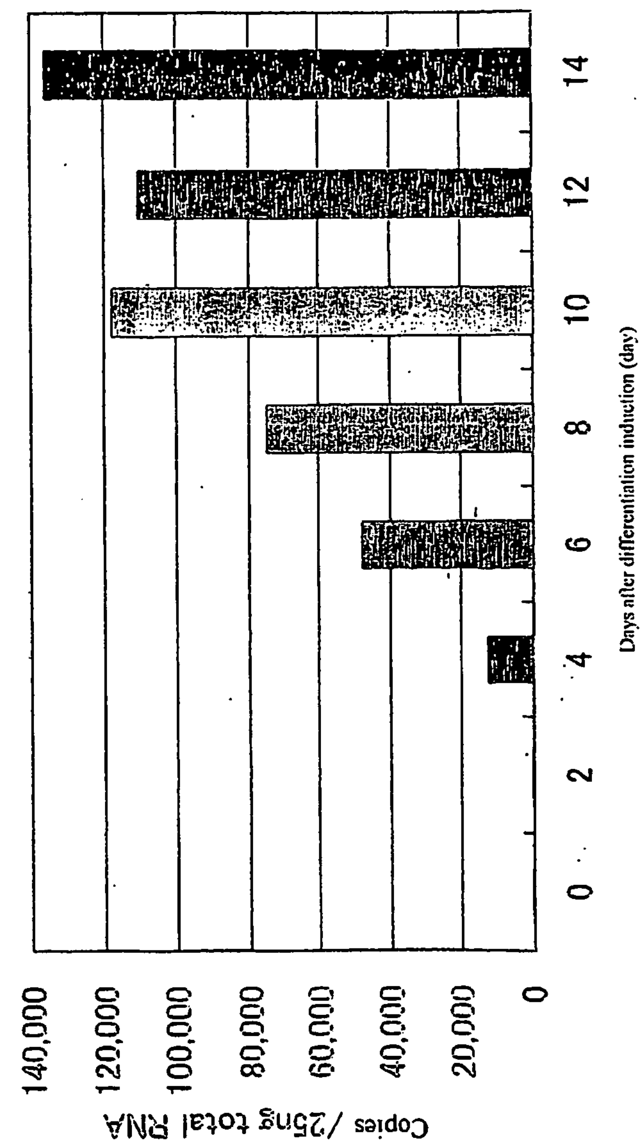
140,000
120,000
100,000
80,000
60,000
40,000
20,000
0
Copies /25ng total RNA
0
2
4
6
8
10
12
14
Days after differentiation induction (day)

FIG 12

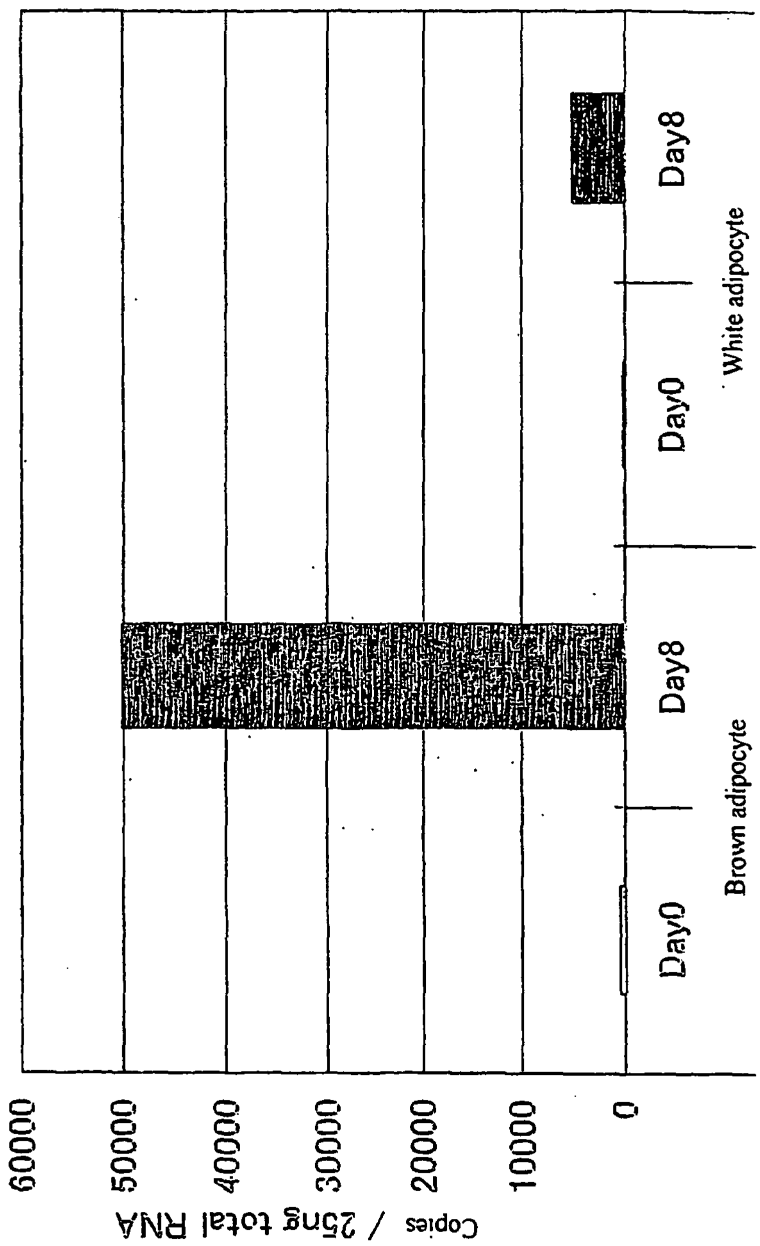
Copies / 25ng total RNA
60000
50000
40000
30000
20000
10000
0
Day0
Day8
Brown adipocyte
Day0
Day8
White adipocyte

FIG 13

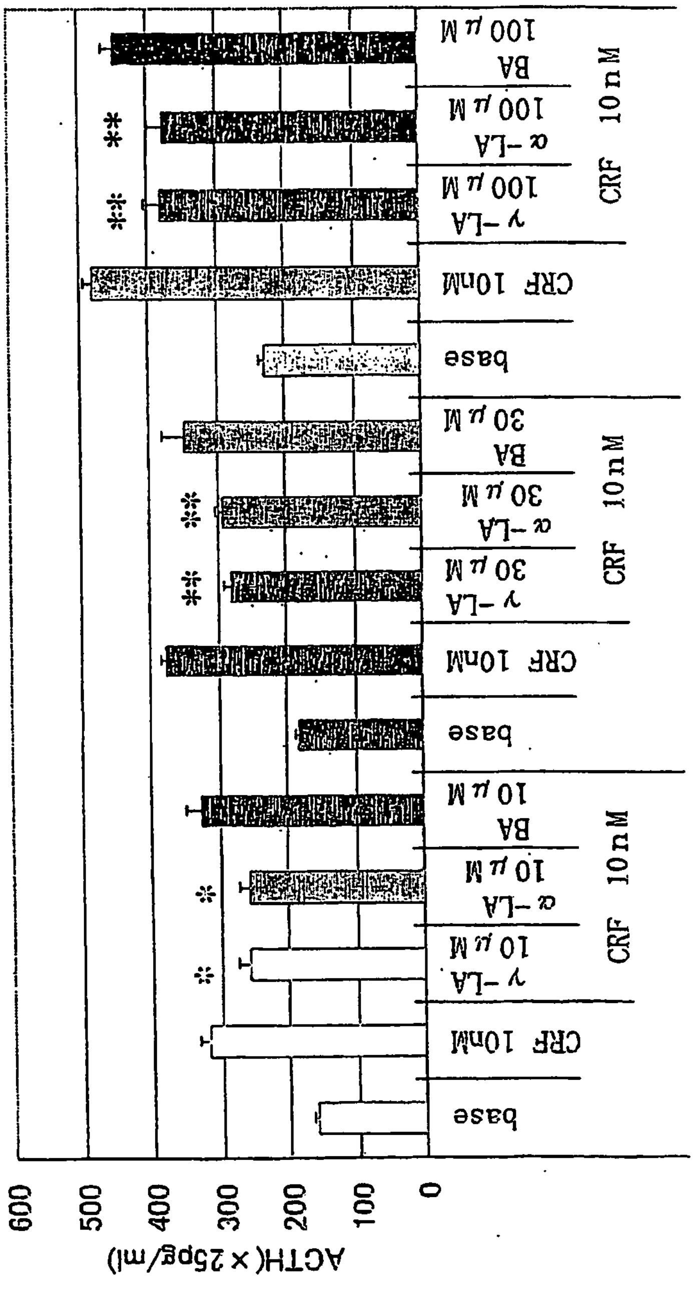
ACTH( × 25pg/ml)
0
100
200
300
400
500
600
base
CRF 10nM
γ-LA 10 μM
α-LA 10 μM
BA 10 μM
CRF 10 n M
base
CRF 10nM
γ-LA 30 μM
α-LA 30 μM
BA 30 μM
CRF 10 n M
base
CRF 10nM
γ-LA 100 μM
α-LA 100 μM
BA 100 μM
CRF 10 n M
*
**

FIG 14

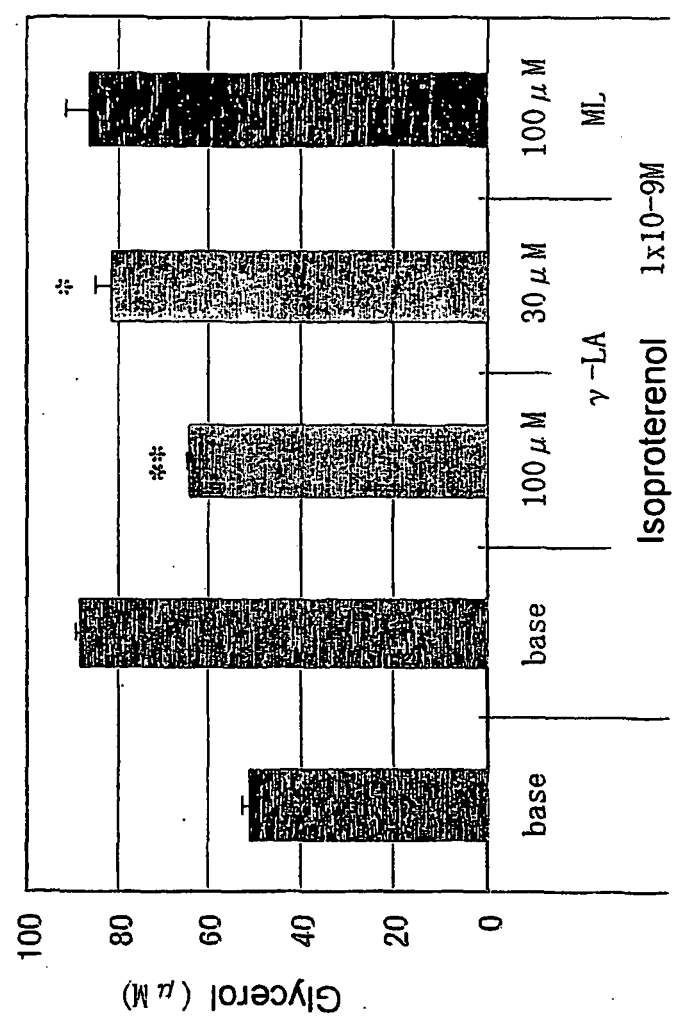
Glycerol (μM)
100
80
60
40
20
0
base
base
100 μM
30 μM
100 μM
**
*
γ-LA
ML
Isoproterenol
1x10-9M

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200000611 A
- WO 200267868 A
- WO 02067868 A
- US 6448005 B1
- WO 02057783 A
- JP 2003010001 A
- JP 2003104540 A
- JP 2003194497 A
- JP 2003329080 A
- JP 2004000248 W

### Non-patent literature cited in the description


- **M. Bodanszky ; M.A. Ondetti.** Peptide Synthesis. Interscience Publishers, 1966
- **Schroeder ; Luebke.** The Peptide. Academic Press, 1965
- Peptide Gosei-no-Kiso to Jikken. Maruzen Co, 1975
- **Haruaki Yajima ; Shunpei Sakakibara.** Seikagaku Jikken Koza. *Tanpakushitsu no Kagaku,* 1977, vol. IV, 205
- **Zoku Iyakuhin no Kaihatsu.** Peptide Synthesis. Hirokawa Shoten, vol. 14
- New PCR and its application. *Jikken Igaku,* 1997, vol. 15 (7
- Molecular Cloning. Cold Spring Harbor Lab. Press, 1989
- **J. Kawakami et al.** *Pharm. Tech. Japan,* 1992, vol. 8, 247
- *PHARM.TECH.JAPAN,* vol. 8, 395
- Antisense Research and Applications. CRC Press, 1993
- **J. Sambrook et al.** Molecular Cloning. Cold Spring Harbor Lab. Press, 1989
- *Proc. Natl. Acad. Sci. U.S.A.,* 1968, vol. 60, 160
- *Nucleic Acids Research,* 1981, vol. 9, 309
- *Journal of Molecular Biology,* vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **Vaughn, J. L. et al.** *In Vivo,* 1977, vol. 13, 213-217
- **Maeda et al.** *Nature,* 1985, vol. 315, 592
- *Proc. Natl. Acad. Sci. U.S.A.,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proc. Natl. Acad. Sci. U.S.A.,* 1978, vol. 75, 1929
- *Bio/Technology,* 1988, vol. 6, 47-55
- Shin Saibo Kogaku Jikken Protocol. Saibo Kogaku. Shujunsha, 1995, vol. 8, 263-267
- *Virology,* 1973, vol. 52, 456
- **Miller.** Journal of Experiments in Molecular Genetics. Cold Spring Harbor Laboratory, 1972, 431-433
- **Bostian, K. L. et al.** *Proc. Natl. Acad. Sci. U.S.A.,* 1980, vol. 77, 4505
- **Bitter, G. A. et al.** *Proc. Natl. Acad. Sci. U.S.A.,* 1984, vol. 81, 5330
- **Grace, T. C. C.** *Nature,* 1962, vol. 195, 788
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- **Koehler ; Milstein.** *Nature,* 1975, vol. 256, 495
- *Genomics,* vol. 5, 874-879
- *Proceedings of the National Academy of Sciences of the United States of America,* vol. 86, 2766-2770
- Radioimmunoassay. Kodansha, 1974
- Sequel to the Radioimmunoassay. Kodansha, 1979
- Enzyme immunoassay. Igakushoin, 1978
- Immunoenzyme assay. Igakushoin, 1982
- Immunoenzyme assay. Igakushoin, 1987
- Immunochemical Techniques (Part A). Methods in ENZYMOLOGY. vol. 70
- Immunochemical Techniques (Part B). METHODS IN ENZYMOLOGY. vol. 73
- Immunochemical Techniques (Part C). METHODS IN ENZYMOLOGY. vol. 74

- Immunochemical Techniques (Part D: Selected Immunoassays. METHODS IN ENZYMOLOGY. vol. 84
- Immunochemical Techniques (Part E: Monoclonal Antibodies and General Immunoassay Methods. METHODS IN ENZYMOLOGY. vol. 92
- Immunochemical Techniques (Part I: Hybridoma Technology and Monoclonal Antibodies. METHODS IN ENZYMOLOGY. Academic Press, vol. 121
- **Nambi, P. et al.** *J. Biol. Chem.,* 1992, vol. 267, 19555-19559
- *Cancer Research,* 1989, vol. 49, 5935-5939
- *Journal of Clinical Oncology,* 1994, vol. 12, 213-225
- *British Journal of Cancer,* 1993, vol. 68, 314-318
- **M. J. Evans ; M. H. Kaufman.** *Nature,* 1981, vol. 292, 154
- **G. R. Martin.** *Proc. Natl. Acad. Sci. U.S.A.,* 1981, vol. 78, 7634
- **T. C. Doetschman et al.** *Journal of Embryology Experimental Morphology,* 1985, vol. 87, 27